# EUROPEAN PATENT SPECIFICATION

(11) **EP 3 234 183 B1**
(45) Date of publication and mention of the grant of the patent: **12.02.2020**
(21) Application number: 15816759.3
(22) Date of filing: 18.12.2015
(51) Int. Cl.: C12Q 1/689

(54) **COMPOSITIONS AND METHODS FOR DETECTION OF DRUG RESISTANT MYCOBACTERIUM TUBERCULOSIS**
ZUSAMMENSETZUNGEN UND VERFAHREN ZUM NACHWEIS VON WIRKSTOFFRESISTENTEM MYCOBACTERIUM TUBERCULOSIS
COMPOSITIONS ET PROCÉDÉS DE DÉTECTION DE MYCOBACTERIUM TUBERCULOSIS RÉSISTANT AUX MÉDICAMENTS

(30) Priority: 18.12.2014 US 201414575879
(43) Date of publication of application: 25.10.2017
(73) Proprietor: Roche Diagnostics GmbH, 68305 Mannheim (DE); F. Hoffmann-La Roche AG, 4070 Basel (CH)
(72) Inventor: JOHNSON, Jenny A., Castro Valley, California 94546 (US); MEHTA, Rochak, Fremont, CA 94555 (US); YUEN, Andy, Dublin, California 94568 (US)
(74) Representative: Schwarz, Ralf
(86) International application number: PCT/EP2015/080484
(87) International publication number: WO 2016/097291

(56) References cited:
- WO-A2-2011/140237
- CN-A- 102 719 537
- CN-B- 102 559 916
- KR-A- 20090 121 611
- US-A1- 2010 261 163
- D. C. T. ONG ET AL: "Rapid Detection of Rifampicin- and Isoniazid-Resistant Mycobacterium tuberculosis by High-Resolution Melting Analysis", JOURNAL OF CLINICAL MICROBIOLOGY, vol. 48, no. 4, 17 February 2010 (2010-02-17), pages 1047-1054, XP055105684, ISSN: 0095-1137, DOI: 10.1128/JCM.02036-09
- NIKOLAYEVSKY V ET AL: "Detection of mutations associated with isoniazid and rifampin resistance in Mycobacterium tuberculosis isolates from Samara Region, Russian Federation", JOURNAL OF CLINICAL MICROBIOLOGY, AMERICAN SOCIETY FOR MICROBIOLOGY, US, vol. 42, no. 10, 1 October 2004 (2004-10-01), pages 4498-4502, XP002330872, ISSN: 0095-1137, DOI: 10.1128/JCM.42.10.4498-4502.2004
- TORRES M J ET AL: "Use of real-time PCR and fluorimetry for rapid detection of rifampin and isoniazid resistance-associated mutations in Mycobacterium tuberculosis", JOURNAL OF CLINICAL MICROBIOLOGY, AMERICAN SOCIETY FOR MICROBIOLOGY, US, vol. 38, no. 9, 1 September 2000 (2000-09-01), pages 3194-3199, XP002252652, ISSN: 0095-1137
- DARÍO GARCÍA DE VIEDMA ET AL: "New real-time PCR able to detect in a single tube multiple rifampin resistance mutations and high-level isoniazid resistance mutations in Mycobacterium tuberculosis", JOURNAL OF CLINICAL MICROBIOLOGY, AMERICAN SOCIETY FOR MICROBIOLOGY, US, vol. 40, no. 3, 1 March 2002 (2002-03-01), pages 988-995, XP002621308, ISSN: 0095-1137, DOI: 10.1128/JCM.40.3.988-995.2002
- Maria Gisele Gonçalves ET AL: "Fast test for assessing the susceptibility of Mycobacterium tuberculosis to isoniazid and rifampin by real-time PCR", Memórias do Instituto Oswaldo Cruz, 1 November 2012 (2012-11-01), pages 903-908, XP055262430, Brazil DOI: 10.1590/S0074-02762012000700011 Retrieved from the Internet: URL:http://www.ncbi.nlm.nih.gov/pmc/articl es/PMC4643165/pdf/nihms735634.pdf [retrieved on 2016-04-04]

## Description

### FIELD OF THE INVENTION

The present invention relates to the field of virus diagnostic, and more particularly, to PCR detection methods utilizing hydrolysis probes for detection of drug resistant *Mycobacterium tuberculosis.*

### BACKGROUND OF THE INVENTION

Tuberculosis (TB) is a bacterial disease caused by various strains of mycobacteria, such as *Mycobacterium tuberculosis* (MTB) most often found in the lungs. It is transmitted from person to person through the air when individuals with pulmonary or laryngeal tuberculosis, cough, sneeze, or spit, and propel MTB into the air. It is estimated that one-third of the world population is infected with MTB and 9 million people develop TB each year. TB continues to be a leading cause of human infectious disease and drug-resistant strains of MTB are on the rise, especially in developing countries.

Two common first-line drugs for the treatment of MTB include isoniazid (INH) and rifampicin (RIF), and patients can acquire drug resistant MTB from living in or visiting a place where drug resistance is prevalent. Patients can also develop drug resistant MTB when their antibiotic treatment regimen is interrupted. Culturing on solid or liquid media is still considered the gold standard for MTB and MTB drug resistance detection, but culturing can take up to eight weeks for results. Many commercial nucleic acid tests for MTB drug resistance have a very fast turn-around time, but cannot detect a population with a small percentage of mutant species in a mixed infection containing both wild type and mutant species. Thus there is a need in the art for a quick and reliable method to specifically detect MTB resistant to rifampicin (MTB-RIF) and/or MTB resistant to isoniazid (MTB-INH) in a sensitive manner.

### SUMMARY OF THE INVENTION

Embodiments described herein relate to methods for the rapid detection of the presence or absence of MTB-RIF and/or MTB-INH in a biological or non-biological sample, for example, multiplex detection of MTB-RIF and/or MTB-INH by real-time polymerase chain reaction in a single test tube. Embodiments include methods of detection of MTB-RIF and/or MTB-INH comprising performing at least one cycling step, which may include an amplifying step and a hybridizing step. Furthermore, embodiments include primers, probes, and kits that are designed for the detection of single MTB-RIF or MTB-INH, or MTB-RIF and MTB-INH co-infections in a single tube. The detection methods are designed to specifically identify single polymorphism (SNP) in target MTB genes for *rpo*B (beta subunit prokaryotic RNA polymerase), *inh*A (enoyl-acyl carrier protein reductase), and *kat*G (catalase-peroxidase) simultaneously, which allows detection and differentiation of MTB-RIF and/or MTB-INH infections in a singlet test. For example, there are 17 SNPs in the *rboB* gene which confer resistance to rifampicin in MTB which include *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P, and *rpo*B 526R; there are 3 SNPs in the *inh*A gene which confer resistance to isoniazid in MTB which include *inh*A*-*15T*, inh*A*-*8A*,* and *inh*A*-*8C*;* and there are 4 SNPs in the *kat*G gene which also confer resistance to isoniazid in MTB which include *kat*G 3151, *kat*G 315N, *kat*G 315T, and *kat*G 315T2.

In one aspect, a method of detecting MTB-RIF and/or MTB-INH in a sample is provided, including performing an amplifying step comprising contacting the sample with at least a set of *rpo*B primers, a set of *inh*A primers, and a set of *kat*G primers to produce one or more amplification products if any *rpo*B*, inh*A*,* and *kat*G target nucleic acid is present in the sample; performing a hybridizing step comprising contacting said one or more amplification products with a plurality of detectable *rpo*B probes, a plurality of detectable *inh*A probes, and a plurality of detectable *kat*G probes, including: at least 17 *rpo*B probes for detection of one or more of 17 single nucleotide polymorphisms SNPs which confer rifampicin resistance to MTB; at least 3 *inhA* probes for detection of one or more of 3 SNPs which confer isoniazid resistance to MTB; and at least 4 *katG* probes for detection of one or more of 4 SNPs which confer isoniazid resistance to MTB; and detecting the presence or absence of said one or more amplification products, wherein the presence of said one or more amplification products is indicative of the presence of MTB-RIF and/or MTB-INH in the sample and wherein the absence of said one or more amplification products is indicative of the absence of MTB-RIF and/or MTB-INH in the sample; wherein said plurality of *rpo*B probes comprise hydrolysis probes for detection of each of the 17 SNPs which confer rifampicin resistance to MTB, comprising *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P, and *rpo*B 526R; wherein said plurality of *inh*A probes comprise hydrolysis probes for detection of each of the 3 SNPs which confer isoniazid resistance to MTB, comprising *inhA-15T, inh*A-8A*,* and *inh*A-8C; and wherein said plurality of *katG* probes comprise hydrolysis probes for detection of each of the 4 SNPs which confer isoniazid resistance to MTB, comprising *katG* 3151, *katG* 315N, *katG* 315T, and *katG* 315T2. In certain embodiments the rpoB probes comprise or consist of a nucleic acid sequence selected from the group consisting of SEQ ID NOs: 1-289 and 437-438, or a complement thereof; the inhA probes comprise or consist of a nucleic acid sequence selected from the group consisting of SEQ ID NOs: 344-409 and 436, or a complement thereof; and the katG probes comprise or consist of a nucleic acid sequence selected from the group consisting of SEQ ID NOs: 290-343 and 434-435, or a complement thereof. In some embodiments the rpoB probes are selected from the group consisting of SEQ ID NOs: 1-289 and 437-438, or a complement thereof; the inhA probes are selected from the group consisting of SEQ ID NOs: 344-409 and 436, or a complement thereof; and the katG probes are selected from the group consisting of SEQ ID NOs: 290-343 and 434-435, or a complement thereof. In some embodiments, each of the plurality of detectable rpoB, inhA, and katG probes is labeled with a donor fluorescent moiety and a corresponding acceptor fluorescent moiety and the detecting step comprises detecting the presence or absence of fluorescence resonance energy transfer (FRET) between the donor fluorescent moiety and the acceptor fluorescent moiety of one or more of the rpoB, inhA, and katG probes, wherein the presence or absence of fluorescence emission is indicative of the presence or absence of MTB-RIF and/or MTB-INH in the sample. In some embodiments said amplification employs a polymerase enzyme having 5' to 3' nuclease activity. In some embodiments said donor and acceptor fluorescent moieties are within no more than 8 nucleotides of each other on said probe. In some embodiments said acceptor fluorescent moiety is a quencher.

In another aspect an oligonucleotide is provided comprising or consisting of a sequence of nucleotides selected from SEQ ID NOs: 1-409 and 434-438, or a complement thereof, which oligonucleotide has 100 or fewer nucleotides. In another aspect, the present disclosure provides an oligonucleotide that includes a nucleic acid having at least 70% sequence identity (e.g., at least 75%, 80%, 85%, 90% or 95%, etc.) to one of SEQ ID NOs: 1-409 and 434-438, or a complement thereof, which oligonucleotide has 100 or fewer nucleotides. Generally, these oligonucleotides may be primer nucleic acids, probe nucleic acids, or the like in these embodiments. In certain of these embodiments, the oligonucleotides have 40 or fewer nucleotides (e.g. 35 or fewer nucleotides, 30 or fewer nucleotides, etc.) In some embodiments, the oligonucleotides comprise at least one modified nucleotide, e.g. to alter nucleic acid hybridization stability relative to unmodified nucleotides. In some embodiments the oligonucleotides comprise one or more detectable label. Optionally, the oligonucleotides comprise at least one label and/or at least one quencher moiety. In some embodiments, the oligonucleotides include at least one conservatively modified variation. "Conservatively modified variations" or, simply, "conservative variations" of a particular nucleic acid sequence refers to those nucleic acids, which encode identical or essentially identical amino acid sequences, or, where the nucleic acid does not encode an amino acid sequence, to essentially identical sequences. One of skill will recognize that individual substitutions, deletions or additions which alter, add or delete a single amino acid or a small percentage of amino acids (typically less than 5%, more typically less than 4%, 2% or 1%) in an encoded sequence are "conservatively modified variations" where the alterations result in the deletion of an amino acid, addition of an amino acid, or substitution of an amino acid with a chemically similar amino acid. In one aspect, amplification can employ a polymerase enzyme having 5' to 3' nuclease activity. Thus, the first and second fluorescent moieties may be within no more than 8 nucleotides of each other along the length of the probe.

In another aspect, a set of oligonucleotides is provided comprising a plurality of detectable rpoB, inhA, and katG probes specific for detection of a rpoB, inhA, and katG amplification products, comprising 17 rpoB probes for detection of one or more of 17 SNPs which confer rifampicin resistance to MTB; 3 inhA probes for detection of one or more of 3 SNPs which confer isoniazid resistance to MTB; and 4 katG probes for detection of one or more of 4 SNPs which confer isoniazid resistance to MTB; wherein said plurality of *rpo*B probes comprise hydrolysis probes for detection of each of the 17 SNPs which confer rifampicin resistance to MTB, comprising *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P, and *rpo*B 526R; wherein said plurality of *inhA* probes comprise hydrolysis probes for detection of each of the 3 SNPs which confer isoniazid resistance to MTB, comprising *inh*A-15T*, inh*A-8A*,* and *ink*A-8C; and wherein said plurality of *kat*G probes comprise hydrolysis probes for detection of each of the 4 SNPs which confer isoniazid resistance to MTB, comprising *kat*G 315I, *kat*G 315N, *kat*G 315T, and *kat*G 315T2. Herein, the set of oligonucleotides is suitable for detecting a nucleic acid of *Mycobacterium tuberculosis* (MTB) resistant to rifampicin (MTB-RIF) and/or MTB resistant to isoniazid (MTB-INH) in a sample. In some embodiments the rpoB probes comprise a nucleic acid sequence selected from the group consisting of SEQ ID NOs: 1-289 and 437-438, or a complement thereof; the inhA probes comprise a nucleic acid sequence selected from the group consisting of SEQ ID NOs: 344-409 and 436, or a complement thereof; and the katG probes comprise a nucleic acid sequence selected from the group consisting of SEQ ID NOs: 290-343 and 434-435, or a complement thereof. In some embodiments, within the set the oligonucleotides have 100 or fewer nucleotides. In certain of these embodiments, the oligonucleotides have 40 or fewer nucleotides (e.g. 35 or fewer nucleotides, 30 or fewer nucleotides, etc.) In some embodiments, the oligonucleotides comprise at least one modified nucleotide, e.g. to alter nucleic acid hybridization stability relative to unmodified nucleotides. Optionally, the oligonucleotides comprise at least one label and/or at least one quencher moiety. In some embodiments, the oligonucleotides include at least one conservatively modified variation. In some embodiments, the oligonucleotides comprise first and second fluorescent moieties which may be within no more than 8 nucleotides of each other along the length of the probe.

In a further aspect, a kit for detecting one or more nucleic acids of MTB-RIF and/or MTB-INH is provided. The kit can include a plurality of sets of *rpoB, inh*A*,* and *katG* primers specific for amplification of a *rpoB, inh*A*,* and *katG* gene targets; and a plurality of detectable *rpo*B*, inh*A*,* and *katG* probes specific for detection of a *rpoB, inh*A*,* and *katG* amplification products. In some embodiments the plurality of detectable rpoB, inhA, and katG probes specific at least comprises 17 rpoB probes for detection of one or more of 17 SNPs which confer rifampicin resistance to MTB; 3 inhA probes for detection of one or more of 3 SNPs which confer isoniazid resistance to MTB; and 4 katG probes for detection of one or more of 4 SNPs which confer isoniazid resistance to MTB; wherein said plurality of rpoB probes comprise hydrolysis probes for detection of each of the 17 SNPs which confer rifampicin resistance to MTB, comprising rpoB 531L, rpoB 531W, rpoB 526L, rpoB 526Y, rpoB 526D, rpoB 526N, rpoB 513L, rpoB 513K, rpoB 513P, rpoB 522L, rpoB 522Q, rpoB 522W, rpoB 516V, rpoB 516Y, rpoB 533P, rpoB 511P, and rpoB 526R; wherein said plurality of inhA probes comprise hydrolysis probes for detection of each of the 3 SNPs which confer isoniazid resistance to MTB, comprising inhA-15T, inhA-8A, and inhA-8C; and wherein said plurality of katG probes comprise hydrolysis probes for detection of each of the 4 SNPs which confer isoniazid resistance to MTB, comprising katG 3151, katG 315N, katG 315T, and katG 315T2. In some embodiments the rpoB probes comprise or consist of a nucleic acid sequence selected from the group consisting of SEQ ID NOs: 1-289 and 437-438, or a complement thereof; the inhA probes comprise or consist of a nucleic acid selected from the group consisting of SEQ ID NOs: 344-409 and 436, or a complement thereof; and the katG probes comprise or consist of a nucleic acid selected from the group consisting of SEQ ID NOs: 290-343 and 434-435, or a complement thereof. In some embodiments the rpoB probes are selected from the group consisting of SEQ ID NOs: 1-289 and 437-438, or a complement thereof; the inhA probes are selected from the group consisting of SEQ ID NOs: 344-409 and 436, or a complement thereof; and the katG probes are selected from the group consisting of SEQ ID NOs: 290-343 and 434-435, or a complement thereof. In some embodiments, the kit can include probes already labeled with donor and corresponding acceptor fluorescent moieties, or can include fluorophoric moieties for labeling the probes. In some embodiments, each probe of said plurality of detectable rpoB, inhA, and katG probes comprises a donor fluorescent moiety and a corresponding acceptor fluorescent moiety. In some embodiments, the acceptor fluorescent moiety is a quencher. The kit can also include nucleoside triphosphates, nucleic acid polymerase, and buffers necessary for the function of the nucleic acid polymerase. The kit can also include a package insert and instructions for using the primers, probes, and fluorophoric moieties to detect the presence or absence of MTB-RIF and/or MTB-INH in a sample.

Unless otherwise defined, all technical and scientific terms used herein have the same meaning as commonly understood by one of ordinary skill in the art to which this invention belongs. Although methods and materials similar or equivalent to those described herein can be used in the practice or testing of the present subject matter, suitable methods and materials are described below. In addition, the materials, methods, and examples are illustrative only and not intended to be limiting.

The details of one or more embodiments are set forth in the accompanying drawings and the description below. Other features, objects, and advantages will be apparent from the drawings and detailed description, and from the claims.

### BRIEF DESCRIPTION OF THE FIGURES

FIGRUE 1A and 1B show wild type and mutant amplicon sequences for the *rpo*B gene target and indication each of the 17 SNPs which confer rifampicin resistance to MTB.

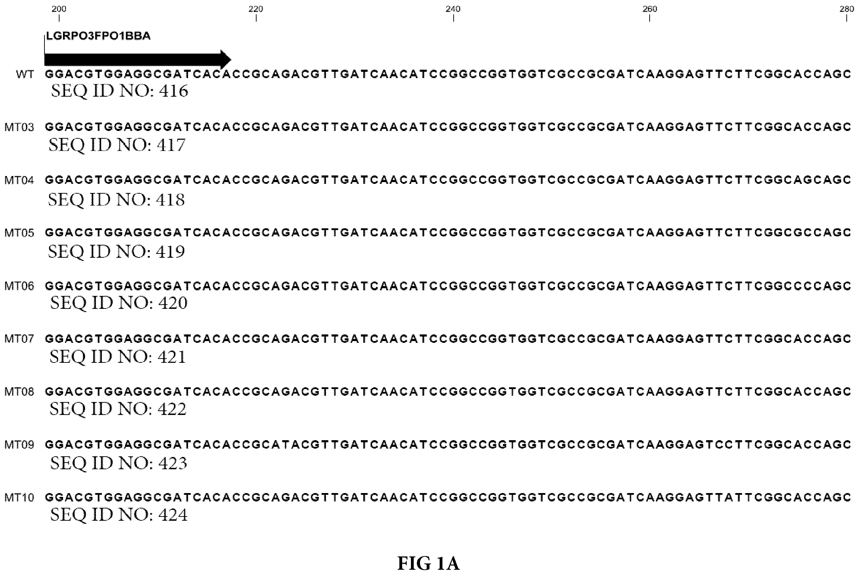

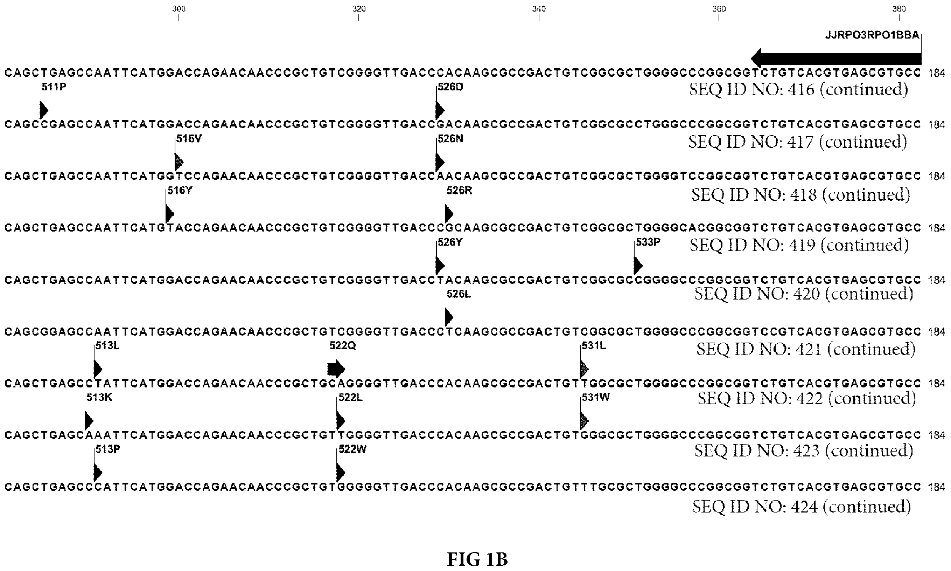

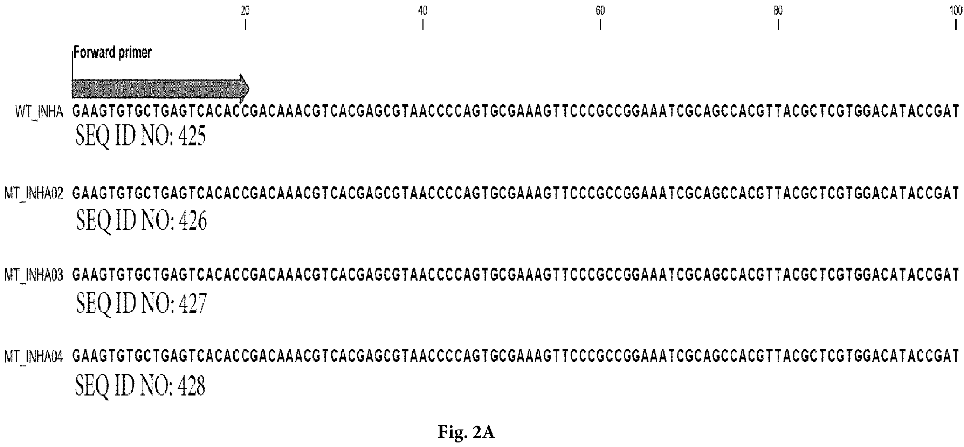

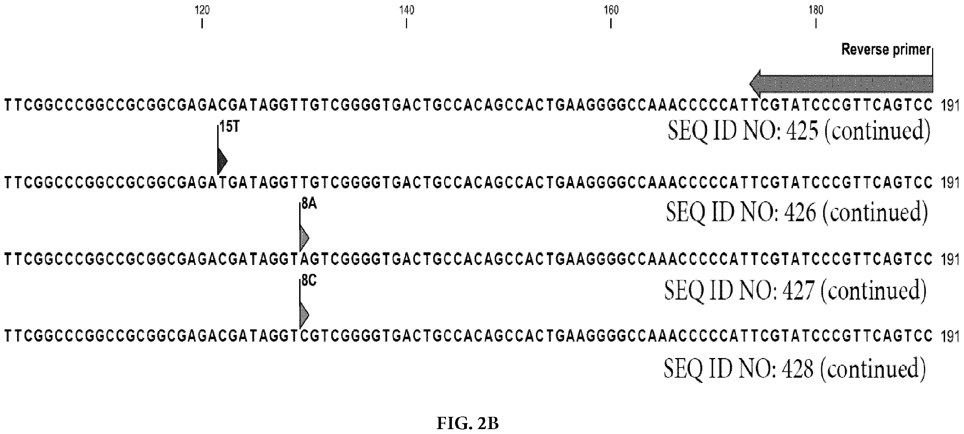

FIGURE 2A and 2B show wild type and mutant amplicon sequences for the *inh*A gene target and indication each of the 3 SNPs which confer isoniazid resistance to MTB.

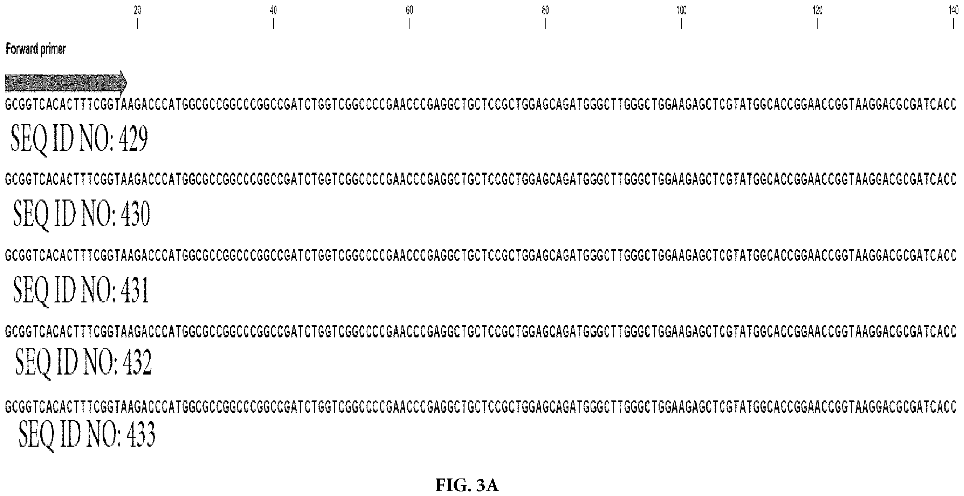

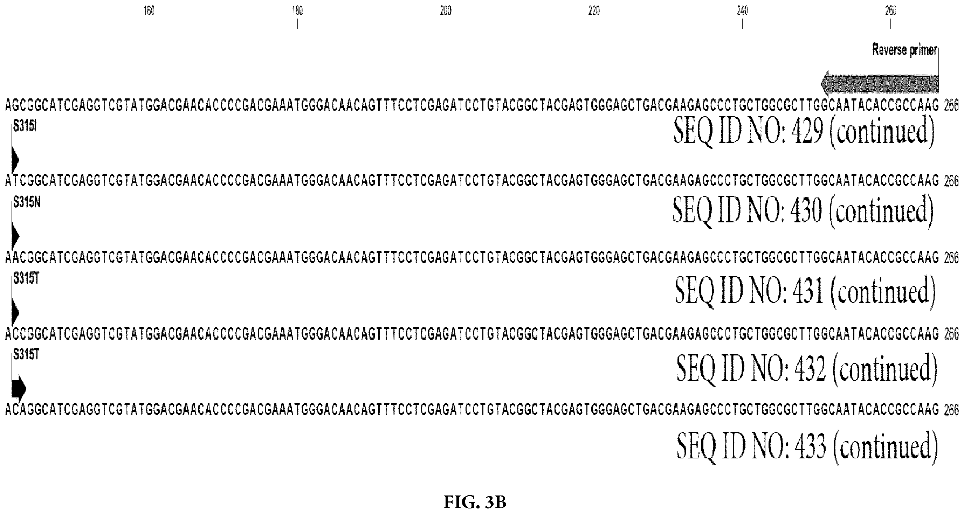

FIGURE 3A and 3B show wild type and mutant amplicon sequences for the *katG* gene target and indication each of the 4 SNPs which confer isoniazid resistance to MTB..

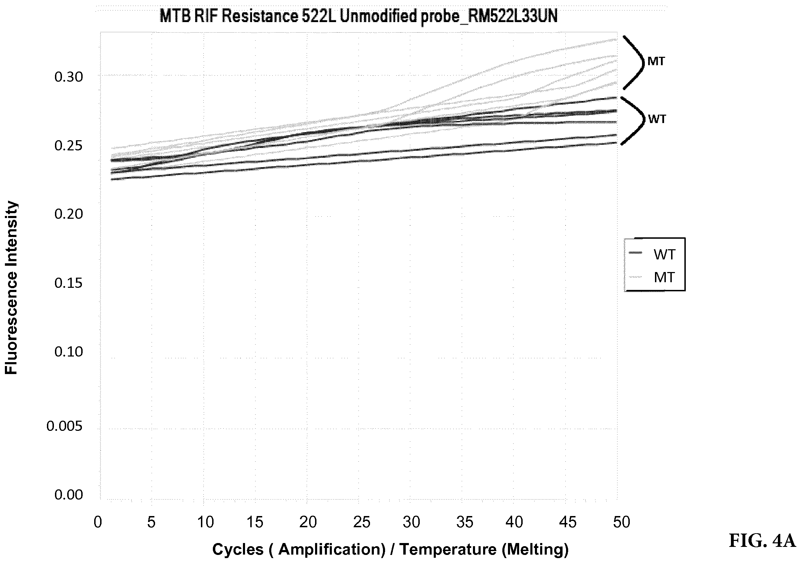

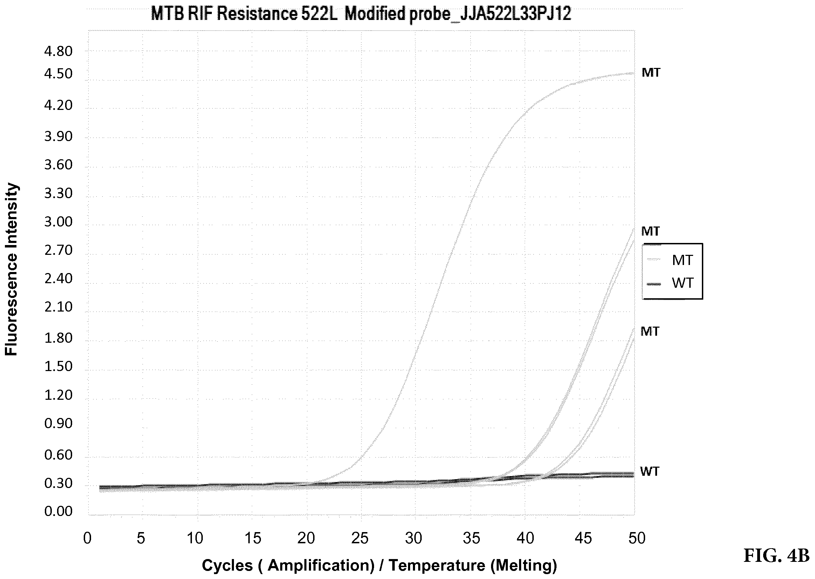

FIGURE 4 and 4B show growth curve assay for singleplex PCR SNP-specific probe detection using unmodified probe for 522L SNP compared to using modified probe for 522L SNP (MT target(∼ 1 e6, 1 e2, 1 e3 10c/PCR) compared with WT).

### DETAILED DESCRIPTION OF THE INVENTION

Diagnosis of MTB-RIF and/or MTB-INH infections by nucleic acid amplification provides a method for rapidly and accurately detecting MTB-RIF and/or MTB-INH infections. A real-time assay for detecting MTB-RIF and/or MTB-INH in a sample is described herein. Primers and probes for detecting the *rpo*B*, inh*A, and *kat*G target nucleic acids of MTB-RIF and/or MTB-INH are provided, as are articles of manufacture or kits containing such primers and probes. The increased sensitivity of real-time PCR for detection of MTB-RIF and/or MTB-INH compared to other methods, as well as the improved features of real-time PCR including sample containment and real-time detection of the amplified product, make feasible the implementation of this technology for routine diagnosis of MTB-RIF and/or MTB-INH infections in the clinical laboratory.

Identification of drug resistant MTB requires detection of numerous single nucleotide polymorphisms (SNPs) in the MTB genome located on several different genes. Using a novel variation of hydrolysis probe (also known as TaqMan probe) design, a multiplex of highly discriminating TaqMan probes were created, wherein each TaqMan probe can detect a single SNP without cross reactivity. The probes are designed to be very short and highly stabilized in order to bind and cleave with great specificity only to a perfectly matched drug resistant (mutant) sequence.

The present disclosure provides Taqman probes for detection of the various SNPs which confer resistance to MTB-RIF and MTB-INH. TaqMan compatible probes are not generally able to detect single base pair mismatches. Generally, TaqMan probes are designed to be much longer, and have a significantly higher melting temperature than the associated PCR primers in order to ensure adequate probe binding to the target sequence prior to the primers and attain maximum probe cleavage during PCR. Due to the high melting temperature and length of such probes, they are generally very tolerant of a single base mismatch under the probe region and thus do not discriminate between two targets that differ by only a single base. In the present disclosures, the hydrolysis TaqMan probes are able to successfully detect only drug resistant MTB and not cross react with drug sensitive MTB, which can only differ by a single base. The present disclosure provides numerous short, highly modified probes that can detect a single base pair mismatch under the probe region. The modified bases that can be substituted in the probe designs include propynyl-dC, t-butyl-benzyl dC, propynyl-dU, G-clamps, methyl-dC, N6-methl dA, and 7-deaza-dG. In designing the probes, the present inventors strategically placed several modified base pairs within the probe sequences in order to maximize the discrimination ability of the probe. It was discovered that some modifications work better than others, and that the discrimination ability is affected by the placement of the modifications in the probe.

The present disclosure provide methods and kits for multiplexed assays which can utilize a plurality of highly modified TaqMan probes wherein each probe can detect a single SNP known to confer Rrifampicin (RIF) and Isoniazid (INH) drug resistance in the MTB genome without significantly cross reacting with drug sensitive (wild type) MTB. The unique ability of the disclosed TaqMan probes to detect mutant SNPs without significant WT cross reactivity enables the assay to detect a minor presence of drug resistant MTB when mixed in a background of wild type (WT). The presence of mixed infection has been reported, and it has been suggested that the prevalence of mixed infection is underreported due to the inability of current commercial assays to detect drug resistant MTB in the presence of drug sensitive MTB. It is reported by the Center for Disease Control (CDC) that a patient that is infected with as little as 1% drug resistant MTB in a background of WT may fail their proposed treatment regimen.

The methods may include performing at least one cycling step that includes amplifying one or more portions of *rpoB, inh*A*,* and *katG* nucleic acid molecule gene targets from a sample using a plurality of pairs of primers, including *rpoB, inh*A*,* and *katG* specific primers as used herein refer to oligonucleotide primers that specifically anneal to nucleic acid sequences encoding *rpo*B*, inh*A*,* and *kat*G*,* respectively, and initiate synthesis therefrom under appropriate conditions. Each of the discussed *rpo*B*, inh*A*,* and *katG* primers anneals to a target within or adjacent to the respective *rpo*B*, inh*A*,* and *kat*G target nucleic acid molecule such that at least a portion of each amplification product contains nucleic acid sequence corresponding to respective target. The one or more of *rpo*B*, inh*A, and *kat*G amplification products are produced provided that one or more of *rpo*B, *inh*A, and *kat*G nucleic acid is present in the sample, thus the presence of the one or more of *rpo*B, *inh*A, and *kat*G amplification products is indicative of the presence of *rpo*B, *inh*A, and *kat*G in the sample. The amplification product should contain the nucleic acid sequences that are complementary to one or more detectable probes for detection of the SNPs in *rpo*B*, inh*A, and *katG* which confer rifampicin and/or isoniazid resistance to MTB. Each cycling step includes an amplification step, a hybridization step, and a detection step, in which the sample is contacted with the one or more detectable probes for *rpo*B*, inh*A, and *kat*G for detection of the presence or absence of MTB-RIF and/or MTB-INH in the sample.

As used herein, the term "amplifying" refers to the process of synthesizing nucleic acid molecules that are complementary to one or both strands of a template nucleic acid molecule (e.g., *rpo*B, *inh*A, and *kat*G nucleic acid molecules). Amplifying a nucleic acid molecule typically includes denaturing the template nucleic acid, annealing primers to the template nucleic acid at a temperature that is below the melting temperatures of the primers, and enzymatically elongating from the primers to generate an amplification product. Amplification typically requires the presence of deoxyribonucleoside triphosphates, a DNA polymerase enzyme (e.g., Platinum® Taq) and an appropriate buffer and/or co-factors for optimal activity of the polymerase enzyme (e.g., MgCl₂ and/or KCl).

The term "primer" is used herein as known to those skilled in the art and refers to oligomeric compounds, primarily to oligonucleotides but also to modified oligonucleotides that are able to "prime" DNA synthesis by a template-dependent DNA polymerase, i.e., the 3'-end of the, e.g., oligonucleotide provides a free 3'-OH group whereto further "nucleotides" may be attached by a template-dependent DNA polymerase establishing 3' to 5' phosphodiester linkage whereby deoxynucleoside triphosphates are used and whereby pyrophosphate is released. Therefore, there is - except possibly for the intended function - no fundamental difference between a "primer", an "oligonucleotide", or a "probe".

The term "hybridizing" refers to the annealing of one or more probes to an amplification product. Hybridization conditions typically include a temperature that is below the melting temperature of the probes but that avoids non-specific hybridization of the probes.

The term "5' to 3' nuclease activity" refers to an activity of a nucleic acid polymerase, typically associated with the nucleic acid strand synthesis, whereby nucleotides are removed from the 5' end of nucleic acid strand.

The term "thermostable polymerase" refers to a polymerase enzyme that is heat stable, i.e., the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber, T. thermophilus, T. aquaticus, T. lacteus*, *T. rubens*, *Bacillus stearothermophilus*, and *Methanothermus fervidus.* Nonetheless, polymerases that are not thermostable also can be employed in PCR assays provided the enzyme is replenished.

The term "complement thereof' refers to nucleic acid that is both the same length as, and exactly complementary to, a given nucleic acid.

The term "extension" or "elongation" when used with respect to nucleic acids refers to when additional nucleotides (or other analogous molecules) are incorporated into the nucleic acids. For example, a nucleic acid is optionally extended by a nucleotide incorporating biocatalyst, such as a polymerase that typically adds nucleotides at the 3' terminal end of a nucleic acid.

The terms "identical" or percent "identity" in the context of two or more nucleic acid sequences, refer to two or more sequences or subsequences that are the same or have a specified percentage of nucleotides that are the same, when compared and aligned for maximum correspondence, e.g., as measured using one of the sequence comparison algorithms available to persons of skill or by visual inspection. Exemplary algorithms that are suitable for determining percent sequence identity and sequence similarity are the BLAST programs, which are described in, e.g., Altschul et al. (1990) "Basic local alignment search tool" J. Mol. Biol. 215:403-410, Gish et al. (1993) "Identification of protein coding regions by database similarity search" Nature Genet. 3:266-272, Madden et al. (1996) "Applications of network BLAST server" Meth. Enzymol. 266:131-141, Altschul et al. (1997) "Gapped BLAST and PSI-BLAST: a new generation of protein database search programs" Nucleic Acids Res. 25:3389-3402, and Zhang et al. (1997) "PowerBLAST: A new network BLAST application for interactive or automated sequence analysis and annotation" Genome Res. 7:649-656.

A "modified nucleotide" in the context of an oligonucleotide refers to an alteration in which at least one nucleotide of the oligonucleotide sequence is replaced by a different nucleotide that provides a desired property to the oligonucleotide. Exemplary modified nucleotides that can be substituted in the oligonucleotides described herein include, e.g., a C5-methyl-dC, a C5-ethyl-dC, a C5-methyl-dU, a C5-ethyl-dU, a 2,6-diaminopurine, a C5-propynyl-dC, a C5-propynyl-dU, a C7-propynyl-dA, a C7-propynyl-dG, a C5-propargylamino-dC, a C5-propargylamino-dU, a C7-propargylamino-dA, a C7-propargylamino-dG, a 7-deaza-2-deoxyxanthosine, a pyrazolopyrimidine analog, a pseudo-dU, a nitro pyrrole, a nitro indole, 2'-0-methyl Ribo-U, 2'-0-methyl Ribo-C, an N4-ethyl-dC, an N6-methyl-dA, and the like. Many other modified nucleotides that can be substituted in the oligonucleotides are referred to herein or are otherwise known in the art. In certain embodiments, modified nucleotide substitutions modify melting temperatures (Tm) of the oligonucleotides relative to the melting temperatures of corresponding unmodified oligonucleotides. To further illustrate, certain modified nucleotide substitutions can reduce non-specific nucleic acid amplification (e.g., minimize primer dimer formation or the like), increase the yield of an intended target amplicon, and/or the like in some embodiments. Examples of these types of nucleic acid modifications are described in, e.g., U.S. Pat. No. 6,001,611.

### MTB-RIF and/or MTB-INH Nucleic Acids and Oligonucleotides

The present dislosure provides methods to detect MTB-RIF and/or MTB-INH by amplifying, for example, a portion of one or more of the *rpo*B*, inh*A*,* and *katG* nucleic acid sequences. Nucleic acid sequences for *rpo*B*, inh*A*,* and *kat*G are available, e.g., through GenBank. Specifically, primers and probes to amplify and detect *rpo*B, *inh*A, and *kat*G nucleic acid molecule targets are provided by the embodiments in the present disclosure.

More specifically, embodiments of the oligonucleotides each include a nucleic acid with a sequence selected from SEQ ID NOs: 1-409 and 434-438, a substantially identical variant thereof in which the variant has at least, e.g., 80%, 90%, or 95% sequence identity to one of SEQ ID NOs: 1-409 and 434-438, or a complement of SEQ ID NOs: 1-409 and 434-438, and the variant.

**TABLE I: Probe for rpoB, inhA, and katG nucleic acid molecule targets**

| **rpoB 531L** | | **TCG/TTG** | **Ser/Leu** |
|---|---|---|---|
| **Oligo Name** | **SEQ ID NO** | **Sequence** | **Modifications** |
| RMRPO3SP531L09 | 1 | FGTTGGQJGCTGGGGCP | F-Threo-FAM::P-Phosphate::Q-BHQ-2, J-G-clamp |
| RMRPO3SP531L18 | 2 | FACTGTTQGGLGLTGGGP | F-Threo-FAM::P-Phosphate::Q-BHQ-2 ,L= Propynyl dC |
| RMRPO3SP531L19 | 3 | FCTGTTQGGLGLUGGGP | F-Threo-FAM::P-Phosphate::Q-BHQ-2 ,L=Propynyl dC , U=propynyl dU |
| RMRPO3S531L18B | 4 | FACTGTTQGGLGLUGGGP | F-Threo-FAM::P-Phosphate::Q-BHQ-2 ,L= Propynyl dC , U=propynyl dU |
| RMRPO3S531L18C | 5 | FCTGTUQGGLGLUGGGP | F-Threo-FAM::P-Phosphate::Q-BHQ-2 ,L= Propynyl dC , U=propynyl dU |
| RMRPO3531L1B | 6 | FCTGTTQGGLGCTGGGGCP | F-Threo-FAM::P-Phosphate::Q-BHQ-2 ,L= Propynyl dC |
| RMRPO3SP531L20 | 7 | FALUGTTQGGLGLUGGP | F-Threo-FAM::P-Phosphate::Q-BHQ-2 ,L= Propynyl dC , U=propynyl dU |
| RMRPO3S531L20 | 8 | JCCGALTGTTGQGLGLUP | J-Threo-JA270::P-Phosphate::Q- BHQ-2,L= Propynyl dC , U=propynyl dU |
| RMRPO3SP531L22 | 9 | JCTGTTGGCGLUGQGGP | J-Threo-JA270::P-Phosphate::Q-BHQ-2,L= Propynyl dC , U=propynyl dU |
| RMRPO3SP531L24 | 10 | FLUGUUQGGLGLTGGGGLP | F-Threo-FAM::P-Phosphate::Q-BHQ-2 ,L= Propynyl dC , U=propynyl dU |
| RMRPO3SP531L25 | 11 | FLUGUUQGGLGLTGGGGP | F-Threo-FAM::P-Phosphate::Q-BHQ-2 ,L= Propynyl dC , U=propynyl dU |
| RMRPO3SP531L26 | 12 | FUGUUGQGLGLTGGGGLLLP | F-Threo-FAM::P-Phosphate::Q-BHQ-2 ,L= Propynyl dC , U=propynyl dU |
| RMRPO3S531L20B | 13 | FALUGUUQGGLGLUGGP | F-Threo-FAM::P-Phosphate::Q-BHQ-2 ,L= Propynyl dC , U=propynyl dU |
| RMRPO3S531L20C | 14 | EALUGUUQGGLGLUGGP | E-Threo-HEX::P-Phosphate::Q-BHQ-2 ,L= Propynyl dC , U=propynyl dU |
| RMRPO3S531L20D | 15 | FALUGUTQGGLGLUGGP | F-Threo-FAM::P-Phosphate::Q-BHQ-2 ,L= Propynyl dC , U=propynyl dU |
| RMRPO3S531L25B | 16 | ELUGUUQGGLGLUGGGP | E-Threo-HEX::P-Phosphate::Q-BHQ-2 ,L= Propynyl dC, U=propynyl dU |
| RMRPO3S531L25C | 17 | ELUGUUQGGLGLUGGLP | E-Threo-HEX::P-Phosphate::Q-BHQ-2 ,L= Propynyl dC, U=propynyl dU |
| RMRPO3S531L20F | 18 | EALUGUUQGGLGLUGLAGLP | E-Threo-HEX::P-Phosphate::Q-BHQ-2 ,L= Propynyl dC, U=propynyl dU |
| RMRPO3531L20HS | 19 | EALUGUUQJGGLGLUGGP | E-Threo-HEX::P-Phosphate::Q-BHQ-2 ,L= Propynyl dC , U=propynyl dU |
| RMRPO3531L25C2 | 20 | ELUGUUGQGLGLUGGLP | E-Threo-HEX::P-Phosphate::Q-BHQ-2 ,L= Propynyl dC , U=propynyl dU |
| RMRPO3531L20C2 | 21 | EALUGUUQGGLGLUGLP | E-Threo-HEX::P-Phosphate::Q-BHQ-2 ,L= Propynyl dC, U=propynyl dU |
| RMRPO3531L25C3 | 22 | EALUGUUQGGLGLUGGLP | E-Threo-HEX::P-Phosphate::Q-BHQ-2 ,L= Propynyl dC, U=propynyl dU |
| RMRPO3S531F1 | 23 | EALUGUUQLGLGLUGGP | E-Threo-HEX::P-Phosphate::Q-BHQ-2 ,L= Propynyl dC , U=propynyl dU |
| RMRPO3531L25B2 | 24 | ELUGUUQGGLGLTGGGP | E-Threo-HEX::P-Phosphate::Q-BHQ-2 ,L= Propynyl dC , U=propynyl dU |
| RMRPO3531L31 | 25 | EUGUUGQGLGLTGGGGP | E-Threo-HEX::P-Phosphate::Q-BHQ-2 ,L= Propynyl dC , U=propynyl dU |
| RMRPO3531L25B3 | 26 | ELUGUUGQGLGLTGGGP | E-Threo-HEX::P-Phosphate::Q-BHQ-2 ,L= Propynyl dC , U=propynyl dU |
| RMRPO3531L25B4 | 27 | ELUGUUGQGLGLTGGCP | E-Threo-HEX::P-Phosphate::Q-BHQ-2 ,L= Propynyl dC , U=propynyl dU |
| RMRPO3F531L | 28 | FCCJACAQGTCGGCGCTTGP | F-Threo-FAM::J-t-Butyl benzyl-dA::P-Phosphate::Q-BHQ-2 |
| RMRPO3F531L 02 | 29 | FCCJACAQGTCGGCGCTTGTGGGTCP | F-Threo-FAM::J-t-Butyl benzyl-dA::P-Phosphate::Q-BHQ-2 |
| RMRPO3F531L 04 | 30 | FCCAACAQGTLGGLGLTTGP | F-Threo-FAM::P-Phosphate::Q-BHQ-2 ,L= Propynyl dC |
| RMRPO3F531L05 | 31 | FCCAACQAGTLGGLGLTTGP | F-Threo-FAM::P-Phosphate::Q-BHQ-2 ,L= Propynyl dC |
| RMRPO3F531L06 | 32 | FCCAACAQGULGGLGLTUGP | F-Threo-FAM::P-Phosphate::Q-BHQ-2 ,L= Propynyl dC , U=propynyl dU |
| RMRPO3AP531L11 | 33 | FCCAACAQGTJGGCGCTTGP | F-Threo-FAM::P-Phosphate::Q-BHQ-2, J-G-clamp |
| RMRPO3AP531L12 | 34 | FCCAAJAQGTCGGCGCTTGP | F-Threo-FAM::P-Phosphate::Q-BHQ-2, J-G-clamp |
| RMRPO3AP531L13 | 35 | FCCAACAQGTCGGJGCTTGP | F-Threo-FAM::P-Phosphate::Q-BHQ-2, J-G-clamp |
| RMRPO3AP531L14 | 36 | FCCAACAQGTCGGCGJTTGP | F-Threo-FAM::P-Phosphate::Q-BHQ-2, J-G-clamp |
| RMRPO3A531L12B | 37 | FCCAALQAGUCGGCGCTTGP | F-Threo-FAM::P-Phosphate::Q-BHQ-2 ,L= Propynyl dC, U=propynyl dU |
| RMRPO3AP531L17 | 38 | FCCAALAQGTLGGLGLP | F-Threo-FAM::P-Phosphate::Q-BHQ-2 ,L= Propynyl dC |
| RMRPO3A531L17B | 39 | FCCAALQAGTLGGLGLP | F-Threo-FAM::P-Phosphate::Q-BHQ-2 ,L= Propynyl dC |
| RMRPO3A531L19 | 40 | FCCAALQAGTLGGLGCTP | F-Threo-FAM::P-Phosphate::Q-BHQ-2 ,L= Propynyl dC |
| RMRPO3A531L20 | 41 | ECCAALAQGTLGGLGCTP | E-Threo-HEX::P-Phosphate::Q-BHQ-2 ,L= Propynyl dC |
| RMRPO3A531L18D | 42 | JCCAALAGULGGCQGLTP | J-Threo-JA270::P-Phosphate::Q-BHQ-2 ,L= Propynyl dC , U=propynyl dU |
| RMRPO3A531L12C | 43 | ECCAALQAGUCGGCGCTTGP | E-Threo-HEX::P-Phosphate::Q-BHQ-2 ,L= Propynyl dC, U=propynyl dU |
| RMRPO3A531L12D | 44 | ECCAALQAGULGGCGCTTGP | E-Threo-HEX::P-Phosphate::Q-BHQ-2 ,L= Propynyl dC, U=propynyl dU |
| RMRPO3531L17B2 | 45 | ECCAALAQGULGGLGLP | E-Threo-HEX::P-Phosphate::Q-BHQ-2 ,L= Propynyl dC , U=propynyl dU |
| RMRPO3531L17B3 | 46 | ELLAALAQGULGGLGLP | E-Threo-HEX::P-Phosphate::Q-BHQ-2 ,L= Propynyl dC , U=propynyl dU |
| RMRPO3A531L21 | 47 | FLLAALQAGULGGLGLUP | F-Threo-FAM::P-Phosphate::Q-BHQ-2 ,L= Propynyl dC , U=propynyl dU |
| RMRPO3A531L22 | 48 | FLLJALQAGULGGLGLP | F-Threo-FAM::P-Phosphate::Q-BHQ-2 ,L= Propynyl dC , U=propynyl dU, J-G-clamp |
| RMRPO3531L17B4 | 49 | FLLAALQAGULGGLGLP | F-Threo-FAM::P-Phosphate::Q-BHQ-2 ,L= Propynyl dC , U=propynyl dU |
| RMRPO3531L17B5 | 50 | FLLJALQAGULGGLGLP | E-Threo-FAM::P-Phosphate::Q-BHQ-2 ,L= Propynyl dC , U=propynyl dU,t-Butyl benzyl-dA |
| RMRPO3A531L20B | 51 | ELLAALAQGULGGLGLP | E-Threo-HEX::P-Phosphate::Q-BHQ-2 ,L= Propynyl dC |
| RM5L17B3a | 52 | ELLAALAQGTLGGLGLP | E-Threo-HEX::P-Phosphate::Q-BHQ-2 ,L= Propynyl dC |

| **rpoB 531W** | | **TCG/TGG** | **Ser/Trp** |
|---|---|---|---|
| **Oligo Name** | **SEQ ID NO:** | **Sequence** | **Modifications** |
| RMRPO3F531W | 53 | FCCLACAQGTCGGCGCTTGP | F= Threo-FAM; Q= t-Butyl benzyl-dC; P= Phosphate; Q= BHQ-2 |
| RMRPO3F531W02 | 54 | FCCLACAQGTCGGCGCTTGTP | F= Threo-FAM; L= t-Butyl benzyl-dC; P= Phosphate; Q= BHQ-2 |
| RMRPO3F531W06 | 55 | FCCJACAQGTCGGCGCP | F-Threo-FAM::P-Phosphate::Q-BHQ-2, J-G-clamp |
| | | | |

| **rpoB 526L** | | **CAC/CTC** | **His/Leu** |
|---|---|---|---|
| **Oligo Name** | **SEQ ID NO:** | **Sequence** | **Modifications** |
| RMRPO3A07 | 56 | FLUUGAGQGGULAALLLLP | F-Threo-FAM::P-Phosphate::Q-BHQ-2 ,L= Propynyl dC:U=propynyl dU |
| RMRPO3A07B2 | 57 | FTTGAGGQGTLAALCCCGACGGGGP | F-Threo-FAM::P-Phosphate::Q-BHQ-2 ,L= Propynyl dC:U=propynyl dU |
| RMRPO3A08 | 58 | FALLLULQAAGLGLLGP | F-Threo-FAM::P-Phosphate::Q-BHQ-2 ,L= Propynyl dC:U=propynyl dU |
| RMRPO3A08B | 59 | FALLLULQAAGLGLLP | F-Threo-FAM::P-Phosphate::Q-BHQ-2 ,L= Propynyl dC:U=propynyl dU |
| RMRPO3A08C | 60 | FALLLULQAAGCGLLGP | F-Threo-FAM::P-Phosphate::Q-BHQ-2 ,L= Propynyl dC:U=propynyl dU |
| RMRPO3A09 | 61 | JLUUGAGGGULAAQLLLLP | J-JA270::P-Phosphate::Q-BHQ-2 ,L= Propynyl dC:U=propynyl dU |
| AYRPO3526LFM07 | 62 | FLUUGAGGGQULAALLLLGAP | F-Threo-FAM::P-Phosphate::Q-BHQ-2::L - pdC:: U-pdU |
| AYRPO3526LFM08 | 63 | FGLUUGAGQGGULAALLLLGAP | F-Threo-FAM::P-Phosphate::Q-BHQ-2::L - pdC:: U-pdU |
| AYRPO3526LFM09 | 64 | FGLUUGAGGQGULAALLLLGAP | F-Threo-FAM::P-Phosphate::Q-BHQ-2::L - pdC:: U-pdU |
| AYRPO3526LFM10 | 65 | FGLUUGAGGGQULAALLLLGAP | F-Threo-FAM::P-Phosphate::Q-BHQ-2::L - pdC:: U-pdU |
| AYRPO3526LFM11 | 66 | FGLUUGAGGQGJLAALLLLGAP | F-Threo-FAM::P-Phosphate::Q-BHQ-2::J-G-clamp::L-pdC:: U-pdU |
| AYRPO3526LFM12 | 67 | FGLUUGAGGGQUJAALLLLGAP | F-Threo-FAM::P-Phosphate::Q- BHQ-2::J-G-clamp::L -pdC:: U-pdU |
| AYRPO3526LJA01 | 68 | FGLUUGAGGQGULAALLLLGAP | F-th-JA270::P-Phosphate::Q-BHQ-2::L - pdC:: U-pdU |
| AYRPO3526LJA02 | 69 | FGLUUGAGGGQULAALLLLGAP | F-th-JA270::P-Phosphate::Q-BHQ-2::L - pdC:: U-pdU |
| AYRPO3526LJA03 | 70 | FGLUUGAGGQGJLAALLLLGAP | F-th-JA270::P-Phosphate::Q- BHQ-2::J-G-clamp::L -pdC:: U-pdU |
| AYRPO3526LJA04 | 71 | FGLUUGAGGGQUJAALLLLGAP | F-th-JA270::P-Phosphate::Q- BHQ-2::J-G-clamp::L -pdC:: U-pdU |
| AYRPO3526LJA05 | 72 | FGLUUGAGGGJQLAALLLLGAP | F-th-JA270::P-Phosphate::Q- BHQ-2::J-G-clamp::L -pdC:: U-pdU |
| AYRPO3526LJA06 | 73 | FGLUUGAGGGJLAAQLLLLGAP | F-th-JA270::P-Phosphate::Q- BHQ-2::J-G-clamp::L -pdC:: U-pdU |
| | | | |

| **rpoB 526Y** | | **CAC/TAC** | **His/Tyr** |
|---|---|---|---|
| **Oligo Name** | **SEQ ID NO:** | **Sequence** | **Modifications** |
| RMRPO3A06E | 74 | JUUGUAGGULAALQLLLGAP | J-JA270::P-Phosphate::Q-BHQ-2,L= Propynyl dC, U=propynyl dU |
| AYRPO3526YFM07 | 75 | FLUUGUJGGQULAALLLLGAP | F-Threo-FAM::P-Phosphate::Q- BHQ-2::J-T-propdA::L -pdC:: U-pdU |
| AYRPO3526YFM08 | 76 | FLUUGQUJGGULAALLLLGAP | F-Threo-FAM::P-Phosphate::Q- BHQ-2::J-T-propdA::L -pdC:: U-pdU |
| AYRPO3526YFM09 | 77 | FLUUGUJGQGULAALLLLGAP | F-Threo-FAM::P-Phosphate::Q- BHQ-2::J-T-propdA::L -pdC:: U-pdU |
| AYRPO3526YFM10 | 78 | FLUUGUAGQGULAALLLLGAP | F-Threo-FAM::P-Phosphate::Q-BHQ-2::L - pdC:: U-pdU |
| AYRPO3526YHX01 | 79 | FLUUGUJGQGULAALLLLGAP | F-Threo-HEX::P-Phosphate::Q-BHQ-2::J-T-propdA::L-pdC::U-pdU |
| AYRPO3526YHX02 | 80 | FLUUGUAGQGULAALLLLGAP | F-Threo-HEX::P-Phosphate::Q-BHQ-2::L - pdC:: U-pdU |
| AYRPO3526YHX03 | 81 | FUUGUJGQGULAALLLLGAP | F-Threo-HEX::P-Phosphate::Q- BHQ-2::J-T-propdA::L -pdC:: U-pdU |
| AYRPO3526YHX04 | 82 | FUUGUJGQGULAALLLLP | F-Threo-HEX::P-Phosphate::Q- BHQ-2::J-T-propdA::L -pdC:: U-pdU |
| AYRPO3526YHX05 | 83 | FUUGUJQGGULAALLLLP | F-Threo-HEX::P-Phosphate::Q- BHQ-2::J-T-propdA::L -pdC:: U-pdU |
| AYRPO3526YHX06 | 84 | FLUUp66GUAGQGJLAALLLLGAP | F-Threo-HEX::P-Phosphate::Q- BHQ-2::J-G-clamp::L -pdC:: U-pdU |
| AYRPO3526YHX07 | 85 | FLUUGUAGQGULAALLLLGALAP | F-Threo-HEX::P-Phosphate::Q-BHQ-2::L - pdC:: U-pdU |
| AYRPO3526YHX08 | 86 | FLGLUUGUAGQGULAALLLLGAP | F-Threo-HEX::P-Phosphate::Q-BHQ-2::L - pdC:: U-pdU |
| AYRPO3526YHX09 | 87 | FGLUUGUAGQGULAALLLLGALAP | F-Threo-HEX::P-Phosphate::Q-BHQ-2::L - pdC:: U-pdU |
| AYRPO3526YHX10 | 88 | FLGLUUGUAGQGULAALLLLGALAP | F-Threo-HEX::P-Phosphate::Q-BHQ-2::L - pdC:: U-pdU |
| | | | |

| **rpoB: 526D** | | **CAC/GAC** | **His/Asp** |
|---|---|---|---|
| **Name** | **SEQ ID NO:** | **Sequence** | **Modifications** |
| RMRPO3A03C | 89 | FLUUGULQGGUCAACLLLP | F-Threo-FAM::P-Phosphate::Q-BHQ-2 ,L= Propynyl dC:U=propynyl dU |
| RMRPO3A03D | 90 | JLUUGULGGULAAQLLCCP | J-JA270::P-Phosphate::Q-BHQ-2,L= Propynyl dC, U=propynyl dU |
| AYRPO3526DFM03 | 91 | FTGTJGQGTCAACCCCGAP | F-Threo-FAM::P-Phosphate::Q- BHQ-2::J-G-Clamp |
| A YRPO3526DFM04 | 92 | FTGTJGGQTCAACCCCGAP | F-Threo-FAM::P-Phosphate::Q- BHQ-2::J-G-Clamp |
| AYRPO3526DFM05 | 93 | FGTTGTQJGGTCAACCCCGAP | F-Threo-FAM::P-Phosphate::Q- BHQ-2::J-G-Clamp |
| AYRPO3526DFM06 | 94 | FGTTGTJGGQTCAACCCCGAP | F-Threo-FAM::P-Phosphate::Q- BHQ-2::J-G-Clamp |
| AYRPO3526DJA07 | 95 | FUUGUJGGULAQALLLLP | F-JA270::P-Phosphate::Q-BHQ-2::L -pdC:: U-pdU::J -G-Clamp |
| AYRPO3526DJA08 | 96 | FLUUGUJGGULAQALLLLP | F-JA270::P-Phosphate::Q-BHQ-2::L -pdC:: U-pdU::J -G-Clamp |
| AYRPO3526DJA09 | 97 | FUUGUJGGULAQALLLLGAP | F-JA270::P-Phosphate::Q-BHQ-2::L -pdC:: U-pdU::J -G-Clamp |
| AYRPO3526DJA10 | 98 | FLUUGUJGGULAQALLLLGAP | F-JA270::P-Phosphate::Q-BHQ-2::L -pdC:: U-pdU::J -G-Clamp |
| AYRPO3526DJA11 | 99 | FGLUUGUJGGULAQALLLLGAP | F-JA270::P-Phosphate::Q-BHQ-2::L -pdC:: U-pdU::J -G-Clamp |
| | | | |

| **rpoB 526N** | | **CAC/AAC** | **His/Asn** |
|---|---|---|---|
| **Oligo Name** | **SEQ ID NO:** | **Sequence** | **Modifications** |
| RMRPO3SP526R2 | 100 | FACCAAQLAAGLGLLGP | F-Threo-FAM::P-Phosphate::Q-BHQ-2 ,L= Propynyl dC |
| RMRPO3SP526R3 | 101 | FALLAAQLAAGLGLLGP | F-Threo-FAM::P-Phosphate::Q-BHQ-2 ,L= Propynyl dC |
| RMRPO3AP526R1 | 102 | FTTGTTQGGTLAALLCCGAP | F-Threo-FAM::P-Phosphate::Q-BHQ-2 ,L= Propynyl dC |
| RMRPO3AP526N1 | 103 | FUUGUUQGGULAALLLP | F-Threo-FAM::P-Phosphate::Q-BHQ-2 ,L= Propynyl dC |
| RMRPO3AP526N2 | 104 | FUUGUUQGGULAALLLGP | F-Threo-FAM::P-Phosphate::Q-BHQ-2 ,L= Propynyl dC |
| RMRPO3AP526N2B | 105 | FUUGUUQGGULJJLLLP | F-Threo-FAM::P-Phosphate::Q-BHQ-2 ,L= Propynyl dC:U=propynyl dU |
| RMRPO3SP526N4 | 106 | FALLAALQAAGLGLLGALUP | F-Threo-FAM::P-Phosphate::Q-BHQ-2 ,L= Propynyl dC:U=propynyl dU |
| RMRPO3SP526N4B | 107 | EALLAALQAAGLGLLGALUP | E-Threo-HEX::P-Phosphate::Q-BHQ-2 ,L= Propynyl dC:U=propynyl dU |
| RMRPO3SP526N4B2 | 108 | EALLAAQLAAGLGLLGALP | E-Threo-HEX::P-Phosphate::Q-BHQ-2 ,L= Propynyl dC:U=propynyl dU |
| RMRPO3SP526N4B2b | 109 | EALLAAQLAAGLGLLGAP | E-Threo-HEX::P-Phosphate::Q-BHQ-2 ,L= Propynyl dC:U=propynyl dU |
| RMRPO3SP526N4B3 | 110 | FLAALQAAGLGLLGALUGP | F-Threo-FAM::P-Phosphate::Q-BHQ-2 ,L= Propynyl dC:U=propynyl dU |
| RMRPO3SP526N4C | 111 | FALLAALQAAGLGLLGP | F-Threo-FAM::P-Phosphate::Q-BHQ-2 ,L= Propynyl dC |
| RMRPO3SP526N5 | 112 | FALLAAQLAAGLGLLGALUP | F-Threo-FAM::P-Phosphate::Q-BHQ-2 ,L= Propynyl dC:U=propynyl dU |
| RMRPO3AP526N6 | 113 | FUUGUUQGGULAALLLLGAP | F-Threo-FAM::P-Phosphate::Q-BHQ-2 ,L= Propynyl dC:U=propynyl dU |
| RMRPO3AP526N7 | 114 | FUUGUQUGGULAALLLLGAP | F-Threo-FAM::P-Phosphate::Q-BHQ-2 ,L= Propynyl dC:U=propynyl dU |
| RMRPO3AP526N8 | 115 | FUUGUUQGGULAALLLLP | F-Threo-FAM::P-Phosphate::Q-BHQ-2 ,L= Propynyl dC:U=propynyl dU |
| RMRPO3AP526N9 | 116 | FUUGUUQGGULOOLLLLP | F-Threo-FAM::P-Phosphate::Q-BHQ-2 ,L= Propynyl dC:U=propynyl dU ,O-t-butyl benzyl dA |
| RMRPO3AP526N1B | 117 | FUUGUUQGGULJJLLLP | F-Threo-FAM::P-Phosphate::Q-BHQ-2 ,L= Propynyl dC ,J-N6 methyl dA |
| RMRPO3A526N8B | 118 | FUUGUUGQGULAALLLLP | F-Threo-FAM::P-Phosphate::Q-BHQ-2 ,L= Propynyl dC:U=propynyl dU |
| RM_NEW526N11B3 | 438 | ETTGTTGQGTCAACCCCGACGJGGP | E-Threo-HEX::P-Phosphate::Q- BHQ-2::J-7_Dz_dG |
| | | | |

| **rpoB 533P** | | **CTG/CCG** | **Leu/Pro** |
|---|---|---|---|
| **Oligo Name** | **SEQ ID NO:** | **Sequence** | **Modifications** |
| RMRPO3H533P10B | 119 | ECGCJGGQGGCCCGGCP | E-Threo-HEX::P-Phosphate::Q-BHQ-2 ,J-G-clamp |
| RMRPO3H533P10D | 120 | FLGLLGGQGGLLLGGLGGP | F-Threo-FAM::P-Phosphate::Q-BHQ-2, J-G-clamp ,L= Propynyl dC |
| RMRPO3H533P10 | 121 | ECGCCGGQGGCCCGGCGGP | E-Threo-HEX::P-Phosphate::Q-BHQ-2 |
| | | | |

| **rpoB: 513L** | | **CAA/CTA** | **Gln/Leu** |
|---|---|---|---|
| **Name** | **SEQ ID NO:** | **Sequence** | **Modifications** |
| JJS513L44PJ12 | 122 | FLAGLUGAGLLUAQUULP | L=pdC, U=pdU, F=th-JA270, Q=BHQ2, p=phosphate |
| JJS513L68PJ12 | 123 | FLLUAUULAUGGAQLLAGP | L=pdC, U=pdU, F=th-JA270, Q=BHQ2, p=phosphate |
| JJS513L59PJ12 | 124 | FLLAGLUGAGLLUQAUULP | L=pdC, U=pdU, F=th-JA270, Q=BHQ2, p=phosphate |
| JJS513L101PJ12 | 125 | FAGLLUAUULAUGQGALLAGP | L=pdC, U=pdU, F=th-JA270, Q=BHQ2, p=phosphate |
| JJS513L84PJ12 | 126 | FGLLUAUULATGGQALLAGP | L=pdC, U=pdU, F=th-JA270, Q=BHQ2, p=phosphate |
| JJA513L60PJ12 | 127 | FUGAAUAGGLULAQGLUGP | L=pdC, U=pdU, F=th-JA270, Q=BHQ2, p=phosphate |
| JJA513L68PJ12 | 128 | FLUGGULLAUGAAQTAGGP | L=pdC, U=pdU, F=th-JA270, Q=BHQ2, p=phosphate |
| JJA513L103PJ12 | 129 | FUULUGGULLAUGQAAUAGGP | L=pdC, U=pdU, F=th-JA270, Q=BHQ2, p=phosphate |
| JJA513L61PJ12 | 130 | FAUGAAUAGGLULQAGLUP | L=pdC, U=pdU, F=th-JA270, Q=BHQ2, p=phosphate |
| JJA513L76PJ12 | 131 | FUGAAUAGGLULAQGLUGGP | L=pdC, U=pdU, F=th-JA270, Q=BHQ2, p=phosphate |
| JJA513L59PJ12 | 132 | FGAAUAGGLULAGQLUGGP | L=pdC, U=pdU, F=th-JA270, Q=BHQ2, p=phosphate |
| JJA513L95PJ12 | 133 | FLAUGAAUAGGLUQLAGLUGP | L=pdC, U=pdU, F=th-JA270, Q=BHQ2, p=phosphate |
| JJA513L924PJ2 | 134 | FGAAUAGGLULAGQUUGGLUP | L=pdC, U=pdU, F=th-JA270, Q=BHQ2, p=phosphate |
| JJA513L928PJ2 | 135 | FGAAUAGGUULAGQLUGGLUP | L=pdC, U=pdU, F=th-JA270, Q=BHQ2, p=phosphate |
| JJA513L927PJ2 | 136 | FGAAUAGGLUUAGQLUGGLUP | L=pdC, U=pdU, F=th-JA270, Q=BHQ2, p=phosphate |
| JJA513L9232PJ2 | 137 | FGAAUAGGLUGAGQLUGGLUP | L=pdC, U=pdU, F=th-JA270, Q=BHQ2, p=phosphate |
| JJA513L9243PJ2 | 138 | FGAAUAGGLULLGQLUGGLUP | L=pdC, U=pdU, F=th-JA270, Q=BHQ2, p=phosphate |
| JJA513L9217PJ2 | 139 | FGAAUAGGLULAGQLAGGLUP | L=pdC, U=pdU, F=th-JA270, Q=BHQ2, p=phosphate |
| JJA513L9218PJ2 | 140 | FGAAUAGGLULAGQAUGGLUP | L=pdC, U=pdU, F=th-JA270, Q=BHQ2, p=phosphate |
| JJA513L9222PJ2 | 141 | FGAAUAGGAULAGQLUGGLUP | L=pdC, U=pdU, F=th-JA270, Q=BHQ2, p=phosphate |
| JJA513L75PJ12 | 142 | FGAAUAGGLULAGQLUGGLP | L=pdC, U=pdU, F=th-JA270, Q=BHQ2, p=phosphate |
| JJA513L75PCA12 | 143 | FGAAUAGGLULAGQLUGGLP | L=pdC, U=pdU, F= CAL Fluor Red 635, Q=BHQ2, p=phosphate |
| JJA513L75PCB12 | 144 | FGAAUAGGLULAGQLUGGLP | L=pdC, U=pdU, F= CAL Fluor Red 635 dT, Q=BHQ2, p=phosphate |
| JJA513L77PJ12 | 145 | FAUGAAUAGGLULQAGLUGP | L=pdC, U=pdU, F=th-JA270, Q=BHQ2, p=phosphate |
| JJA513L93PJ12 | 146 | FUGAAUAGGLULAQGLUGGLP | L=pdC, U=pdU, F=th-JA270, Q=BHQ2, p=phosphate |
| JJS513L85PJ12 | 147 | FLLUAUULAUGGAQLLAGAP | L=pdC, U=pdU, F=th-JA270, Q=BHQ2, p=phosphate |
| | | | |

| **rpoB: 513K** | | **CAA/AAA** | **Gln/Lys** |
|---|---|---|---|
| **Name** | **SEQ ID NO:** | **Sequence** | **Modifications** |
| JJS513K84PJ12 | 148 | FAGLAAAUULAUGQGALLAP | L=pdC, U=pdU, F=th-JA270, Q=BHQ2, p=phosphate |
| JJS513K77PJ12 | 149 | FLLAGLUGAGLAAQAUULAP | L=pdC, U=pdU, F=th-JA270, Q=BHQ2, p=phosphate |
| JJS513K79PJ12 | 150 | FAGLUGAGLAAAUQULAUGP | L=pdC, U=pdU, F=th-JA270, Q=BHQ2, p=phosphate |
| JJS513K85PJ12 | 151 | FGLAAAUULAUGGQALLAGP | L=pdC, U=pdU, F=th-JA270, Q=BHQ2, p=phosphate |
| JJA513K43PJ12 | 152 | FAUUUGLULAGLUQGGLP | L=pdC, U=pdU, F=th-JA270, Q=BHQ2, p=phosphate |
| JJA513K77PJ12 | 153 | FUGAAUUUGLULAQGLUGGP | L=pdC, U=pdU, F=th-JA270, Q=BHQ2, p=phosphate |
| JJA513K60PJ12 | 154 | FGAAUUUGLULAGQLUGGP | L=pdC, U=pdU, F=th-JA270, Q=BHQ2, p=phosphate |
| JJA513K76PJ12 | 155 | FGAAUUUGLULAGQLUGGLP | L=pdC, U=pdU, F=th-JA270, Q=BHQ2, p=phosphate |
| JJA513K76PCA12 | 156 | FGAAUUUGLULAGQLUGGLP | L=pdC, U=pdU, F= CAL Fluor Red 635, Q=BHQ2, p=phosphate |
| JJA513K76PCB12 | 157 | FGAAUUUGLULAGQLUGGLP | L=pdC, U=pdU, F= CAL Fluor Red 635 dT, Q=BHQ2, p=phosphate |
| JJA513K44PJ12 | 158 | FAAUUUGLULAGLQUGGP | L=pdC, U=pdU, F=th-JA270, Q=BHQ2, p=phosphate |
| JJA513K80PJ12 | 159 | FLLAUGAAUUUGLQULAGLP | L=pdC, U=pdU, F=th-JA270, Q=BHQ2, p=phosphate |
| JJA513K59PJ12 | 160 | FAAUUUGLULAGLQUGGLP | L=pdC, U=pdU, F=th-JA270, Q=BHQ2, p=phosphate |
| JJA513K75PJ12 | 161 | FAAUUUGLULAGQLUGGLUP | L=pdC, U=pdU, F=th-JA270, Q=BHQ2, p=phosphate |
| | | | |

| **rpoB: 513P** | | **CAA/CCA** | **Gln/Pro** |
|---|---|---|---|
| **Name** | **SEQ ID NO:** | **Sequence** | **Modifications** |
| JJS513P66PJ12 | 162 | FAGLLLAUULAUGQGALLP | L=pdC, U=pdU, F=th-JA270, Q=BHQ2, p=phosphate |
| JJS513P83PJ12 | 163 | FAGLLLAUULAUGQGALLAP | L=pdC, U=pdU, F=th-JA270, Q=BHQ2, p=phosphate |
| JJS513P68PJ12 | 164 | FLLLAUULAUGGAQLLAGP | L=pdC, U=pdU, F=th-JA270, Q=BHQ2, p=phosphate |
| JS513P81PJ12 | 165 | FUGAGLLLAUULAQUGGALP | L=pdC, U=pdU, F=th-JA270, Q=BHQ2, p=phosphate |
| JJA513P60PJ12 | 166 | FUGAAUGGGLULAQGLUGP | L=pdC, U=pdU, F=th-JA270, Q=BHQ2, p=phosphate |
| JJA513P76PJ12 | 167 | FUGAAUGGGLTLAQGLUGGP | L=pdC, U=pdU, F=th-JA270, Q=BHQ2, p=phosphate |
| JJS513P66GJ12 | 168 | FAGLLEAUULAUGQGALLP | E=G-clamp, L=pdC, |
| | | | U=pdU, F=th-JA270, Q=BHQ2, p=phosphate |
| JJS513P83GJ12 | 169 | FAGLLEAUULAUGQGALLAP | E=G-clamp, L=pdC, U=pdU, F=th-JA270, Q=BHQ2, p=phosphate |
| JJS513P84PJ12 | 170 | FGLLLAUULAUGGQALLAGP | L=pdC, U=pdU, F=th-JA270, Q=BHQ2, p=phosphate |
| JJS513P82PJ12 | 171 | FGAGLLLAUULAUQGGALLP | L=pdC, U=pdU, F=th-JA270, Q=BHQ2, p=phosphate |
| JJS513P84GJ12 | 172 | FGLLEAUUCAUGGQALLAGP | E=G-clamp, L=pdC, U=pdU, F=th-JA270, Q=BHQ2, p=phosphate |
| JJS513P82GJ12 | 173 | FGAGLLEAUULAUQGGALLP | E=G-clamp, L=pdC, U=pdU, F=th-JA270, Q=BHQ2, p=phosphate |
| | | | |

| **rpoB: 522L** | | **TCG/TTG** | **Ser/Leu** |
|---|---|---|---|
| **Name** | **SEQ ID NO:** | **Sequence** | **Modifications** |
| JJA522L50PJ12 | 174 | FGGULAALLLLAAQLAGP | L=pdC, U=pdU, F=th-JA270, Q=BHQ2, p=phosphate |
| JJA522L18PJ12 | 175 | FALLLLAALAGLGQGP | L=pdC, U=pdU, F=th-JA270, Q=BHQ2, p=phosphate |
| JJA522L16PJ12 | 176 | FLLLAALAGLGGGQUP | L=pdC, U=pdU, F=th-JA270, Q=BHQ2, p=phosphate |
| JJA522L17PJ12 | 177 | FLLLLAALAGLGGQGP | L=pdC, U=pdU, F=th-JA270, Q=BHQ2, p=phosphate |
| JJA522L83PJ12 | 178 | FUGGGULAALLLLQAALAGP | L=pdC, U=pdU, F=th-JA270, Q=BHQ2, p=phosphate |
| JJA522L32PJ12 | 179 | FAALLLLAALAGLQGGP | L=pdC, U=pdU, F=th-JA270, Q=BHQ2, p=phosphate |
| JJA522L33PJ12 | 180 | FLAALLLLAALAGQLGP | L=pdC, U=pdU, F=th-JA270, Q=BHQ2, p=phosphate |
| JJA522L48PJ12 | 181 | FULAALLLLAALAQGLGP | L=pdC, U=pdU, F=th-JA270, Q=BHQ2, p=phosphate |
| JJA522L30PJ12 | 182 | FLLLLAALAGLGGQGUP | L=pdC, U=pdU, F=th-JA270, Q=BHQ2, p=phosphate |
| JJA522L31PJ12 | 183 | FALLLLAALAGLGQGGP | L=pdC, U=pdU, F=th-JA270, Q=BHQ2, p=phosphate |
| JJA522L63PJ12 | 184 | FULAALLLLAALAQGLGGP | L=pdC, U=pdU, F=th-JA270, Q=BHQ2, p=phosphate |
| JJA522L47PJ12 | 185 | FLAALLLLAALAGQLGGP | L=pdC, U=pdU, F=th-JA270, Q=BHQ2, p=phosphate |
| | | | |

| **rpoB: 522Q** | | **TCG/CAG** | **Ser/Gln** |
|---|---|---|---|
| **Name** | **SEQ ID NO:** | **Sequence** | **Modifications** |
| JJS522Q48J12 | 186 | FCCGCTGCAGGGGQTTGAP | F=th-JA270, Q=BHQ2, p=phosphate |
| JJS522Q32J12 | 187 | FCCCGCTGCAGGGQGTTP | F=th-JA270, Q=BHQ2, p=phosphate |
| JJS522Q49J12 | 188 | FCGCTGCAGGGGTQTGACP | F=th-JA270, Q=BHQ2, p=phosphate |
| JJA522Q31J12 | 189 | FACCCCTGCAGCGQGGTP | F=th-JA270, Q=BHQ2, p=phosphate |
| JJA522Q33J12 | 190 | FCAACCCCTGCAGQCGGP | F=th-JA270, Q=BHQ2, p=phosphate |
| JJA522Q43J12 | 191 | FCCCTGCAGCGGGQTTGTP | F=th-JA270, Q=BHQ2, p=phosphate |
| JJA522Q31PJ12 | 192 | FALLLLUGCAGLGQGGUP | L=pdC, U=pdU, F=th-JA270, Q=BHQ2, p=phosphate |
| JJS522Q49PJ12 | 193 | FLGLUGLAGGGGUQUGALP | L=pdC, U=pdU, F=th-JA270, Q=BHQ2, p=phosphate |
| JJA522Q18PJ12 | 194 | FALLLLUGLAGLGQGGP | L=pdC, U=pdU, F=th-JA270, Q=BHQ2, p=phosphate |
| JJS522Q34PJ12 | 195 | FLGLUGLAGGGGUQUGAP | L=pdC, U=pdU, F=th-JA270, Q=BHQ2, p=phosphate |
| JJA522Q20PJ12 | 196 | FLAALLLLUGLAGQLGP | L=pdC, U=pdU, F=th-JA270, Q=BHQ2, p=phosphate |
| JJA522Q33PJ12 | 197 | FLAALLLLUGLAGQLGGP | L=pdC, U=pdU, F=th-JA270, Q=BHQ2, p=phosphate |
| JJA522Q47PJ12 | 198 | FLAALLLLUGLAGQLGGGP | L=pdC, U=pdU, F=th-JA270, Q=BHQ2, p=phosphate |
| | | | |

| **rpoB: 522W** | | **TCG/TGG** | **Ser/Trp** |
|---|---|---|---|
| **Name** | **SEQ ID NO:** | **Sequence** | **Modifications** |
| JJA522W33PJ12 | 199 | FLAALLLLLALAGQLGP | L=pdC, U=pdU, F=th-JA270, Q=BHQ2, p=phosphate |
| JJA522W33GJ12 | 200 | FLAALLLLEALAGQLGP | E=G-clamp, L=pdC, U=pdU, F=th-JA270, Q=BHQ2, p=phosphate |
| JJA522W47J12 | 201 | FLAALLLLLALAGQLGGP | L=pdC, U=pdU, F=th-JA270, Q=BHQ2, p=phosphate |
| JJA522W47GJ12 | 202 | FLAALLLLEALAGQLGGP | E=G-clamp,L=pdC, U=pdU, F=th-JA270, Q=BHQ2, p=phosphate |
| JJA522W48PJ12 | 203 | FULAALLLLLALAQGLGP | L=pdC, U=pdU, F=th-JA270, Q=BHQ2, p=phosphate |
| JJA522W48GJ12 | 204 | FULAALLLLEALAQGLGP | E=G-clamp,L=pdC, U=pdU, F=th-JA270, Q=BHQ2, p=phosphate |
| | | | |

| **rpoB 516V** | | **GAC/GTC** | **Asp/Val** |
|---|---|---|---|
| **Name** | **SEQ ID NO:** | **Sequence** | **Modifications** |
| RMRPO3F516V | 205 | FCTGGJCQCATGAATTGGCTCP | F-Threo-FAM::J-t-Butyl benzyl-dA::P-Phosphate::Q-BHQ-2 |
| RMRPO3UNF516V | 206 | FCTGGACQCATGAATTGGCTCP | F-Threo-FAM::P-Phosphate::Q-BHQ-2 |
| RMRPO3SP516V | 207 | FTGGTCQCAGAACAACCCGCTP | F-Threo-FAM::P-Phosphate::Q-BHQ-2 |
| RMRPO3SP516Y | 208 | EATGTACQCAGAACAACCCGCTGP | E-Threo-HEX::P-Phosphate::Q-BHQ-2 |
| RMRPO3AP516Y | 209 | ECTGGTACQATGAATTGGCTCP | E-Threo-HEX::P-Phosphate::Q-BHQ-2 |
| RMRPO3A2P516Y | 210 | ECTGGTACQATGAATTGGCTCAGCP | E-Threo-HEX::P-Phosphate::Q-BHQ-2 |
| RMRPO3F516V02 | 211 | FCTGGJCQLATGAATTGGLTLP | F-Threo-FAM::P-Phosphate::Q-BHQ-2 ,L= Propynyl dC, J-t-Butyl benzyl-dA |
| RMRPO3SP516V03 | 212 | FTGGTCQCAGAACAACCCGCTGCGGP | F-Threo-FAM::P-Phosphate::Q-BHQ-2 |
| RMRPO3SP516V04 | 213 | FTGGTCQCAAAACAACCCGCTP | F-Threo-FAM::P-Phosphate::Q-BHQ-2 |
| RMRPO3SP516V05 | 214 | FTGGTEQCAGAACAACCCGCTP | F-Threo-FAM::P-Phosphate::Q-BHQ-2, EG-clamp |
| RMRPO3SP516V06 | 215 | FTGGTCQEAGAACAACCCGCTP | F-Threo-FAM::P-Phosphate::Q-BHQ-2, EG-clamp |
| RMRPO3SP516Y02 | 216 | EATGTACQCAGAACAACCCGGGGTP | E-Threo-HEX::P-Phosphate::Q-BHQ-2 |
| RMRPO3SP516V07 | 217 | FTGGTCQCAGAATAACCCGCTGCGGP | F-Threo-FAM::P-Phosphate::Q-BHQ-2 |
| RMRPO3SP516V08 | 218 | FTGGTCQCAAAACAACCCGCTGCGGP | F-Threo-FAM::P-Phosphate::Q-BHQ-2 |
| RMRPO3SP516V09 | 219 | FGGTCCQAGAACAACCCGCTGP | F-Threo-FAM::P-Phosphate::Q-BHQ-2 |
| RMRPO3SP516V10 | 220 | FATGGTCQCAGAACAACCCGCTP | F-Threo-FAM::P-Phosphate::Q-BHQ-2 |
| RMRPO3SP516V11 | 221 | ECATGGTCQCAGAACAACCCGP | E-Threo-HEX::P-Phosphate::Q-BHQ-2 |
| RMRPO3SP516V12 | 222 | FATGGTCQCAGAACAACCGGTTGP | F-Threo-FAM::P-Phosphate::Q-BHQ-2 |
| RMRPO3S516V11B | 223 | FCATGGTQCCAGAACAACCCGP | F-Threo-FAM::P-Phosphate::Q-BHQ-2 |
| RMRPO3S516V11C | 224 | FLAUGGUQLLAGAALAALLLGP | F-Threo-FAM::P-Phosphate::Q-BHQ-2, L= Propynyl dC, U=propynyl dU |
| RMRPO3S516V11D | 225 | FCATGGQTCCAGAACAACCCGP | F-Threo-FAM::P-Phosphate::Q-BHQ-2 |
| RMRPO3S516V11E | 226 | FATGGTQCCAGAACAACCCGP | F-Threo-FAM::P-Phosphate::Q-BHQ-2 |
| RMRPO3S516V11F | 227 | JCATGGTCCAGAAQCAACCCGP | J-JA270::P-Phosphate::Q-BHQ-2 |
| RMRPO3S516V11G | 228 | JLAUGGULLAGAAQLAALP | J-JA270::P-Phosphate::Q-BHQ-2 |
| RMRPO3516V11C2 | 229 | FLAUGGUQLLJGAJLAJLP | F-Threo-FAM::P-Phosphate::Q-BHQ-2, L= Propynyl dC, U=propynyl dU,J: N6-Benzyl dA |
| RM516V11G2 | 230 | FLLUGGUQLLAGAALAALP | F-Threo-FAM::P-Phosphate::Q-BHQ-2 |
| RMRPO3516V11G2 | 231 | JLAUGGULLAGAAQLAP | J-JA270::P-Phosphate::Q-BHQ-2,L= Propynyl dC, U=propynyl dU |
| RMRPO3516V11G3 | 232 | JLAUGGTLLAGAAQLAP | J-JA270::P-Phosphate::Q-BHQ-2,L= Propynyl dC, U=propynyl dU |
| RMRPO3S516V12B | 233 | FCATGGTCQCAGAACAACCGGTTGP | F-Threo-FAM::P-Phosphate::Q-BHQ-2 |
| RMRPO3516V11C4 | 234 | FLAUGGUQLLAGAALAALLP | F-Threo-FAM::P-Phosphate::Q-BHQ-2, L= Propynyl dC, U=propynyl dU |
| RMRPO3516V11E2 | 235 | FTGGTQCCAGAACAACCCGCTGP | F-Threo-FAM::P-Phosphate::Q-BHQ-2 |
| RMRPO3516V11E3 | 236 | FATGGTQCCAGAACAACAGTTGP | F-Threo-FAM::P-Phosphate::Q-BHQ-2 |
| RMRPO3516V11E5 | 237 | FTGGTQCCAGAACAACCCGCTP | F-Threo-FAM::P-Phosphate::Q-BHQ-2 |
| RMRPO3516V11E6 | 238 | FTGGTCCQAGAACAACCCGCTP | F-Threo-FAM::P-Phosphate::Q-BHQ-2 |
| | | | |

| **rpoB 516Y** | | **GAC/TAC** | **Asp/Tyr** |
|---|---|---|---|
| **Oligo Name** | **SEQ ID NO:** | **Sequence** | **Modifications** |
| RMRPO3SP516Y03 | 239 | EATGTAJCQAGAACAACCCGCP | E-Threo-HEX::P-Phosphate::Q-BHQ-2 ,J-G-clamp |
| RMRPO3S516YB2 | 240 | FATGTQACCAGAACAACCCGCTGP | F-Threo-FAM::P-Phosphate::Q-BHQ-2 |
| RMRPO3SP516Y | 241 | EATGTACQCAGAACAACCCGCTGP | E-Threo-HEX::P-Phosphate::Q-BHQ-2 |
| RMRPO3AP516Y | 242 | ECTGGTACQATGAATTGGCTCP | E-Threo-HEX::P-Phosphate::Q-BHQ-2 |
| RMRPO3A2P516Y | 243 | ECTGGTACQATGAATTGGCTCAGCP | E-Threo-HEX::P-Phosphate::Q-BHQ-2 |
| RMRPO3SP516Y02 | 244 | EATGTACQCAGAACAACCCGGGGTP | E-Threo-HEX::P-Phosphate::Q-BHQ-2 |
| RMRPO3SP516Y4B | 245 | EATGTACQCAGAACAACCCGCTGTP | E-Threo-HEX::P-Phosphate::Q-BHQ-2 |
| RMRPO3S516YB | 246 | FATGTACQCAGAACAACCCGCTGP | F-Threo-FAM::P-Phosphate::Q-BHQ-2 |
| RMRPO3S516YC | 247 | FATGTAQCCAGAACAACCCGCTGP | F-Threo-FAM::P-Phosphate::Q-BHQ-2 |
| | | | |

| **rpoB 533P** | | **CTG/CCG** | **Leu/Pro** |
|---|---|---|---|
| **Oligo Name** | **SEQ ID NO:** | **Sequence** | **Modifications** |
| RMRPO3H533P10B | 248 | ECGCJGGQGGCCCGGCP | E-Threo-HEX::P-Phosphate::Q-BHQ-2 ,J-G-clamp |
| RMRPO3H533P10D | 249 | FLGLLGGQGGLLLGGLGGP | F-Threo-FAM::P-Phosphate::Q-BHQ-2, J-G-clamp |
| RMRPO3H533 | 250 | ECCGGCGQCCGACAGTCGGCGP | E-Threo-HEX::P-Phosphate::Q-BHQ-2 |
| RMRPO3H533P02 | 251 | ECCGGCGQCCGACAGTCGGP | E-Threo-HEX::P-Phosphate::Q-BHQ-2 |
| RMRPO3H533P03 | 252 | ECCGGCGQCCTACAGTCGGCGP | E-Threo-HEX::P-Phosphate::Q-BHQ-2 |
| RMRPO3H533P04 | 253 | ECCGGCGQCCAACAGTCGGCGP | E-Threo-HEX::P-Phosphate::Q-BHQ-2 |
| RMRPO3H533P05 | 254 | ECCGGCGQCCCACAGTCGGCGP | E-Threo-HEX::P-Phosphate::Q-BHQ-2 |
| RMRPO3H533P06 | 255 | ECCGGCQACCGACAGTCGGP | E-Threo-HEX::P-Phosphate::Q-BHQ-2 |
| RMRPO3H533P07 | 256 | ECCGGCGQTCGACAGTCGGP | E-Threo-HEX::P-Phosphate::Q-BHQ-2 |
| RMRPO3H533P08 | 257 | ECCGGCQGCCGACAGTCGGCP | E-Threo-HEX::P-Phosphate::Q-BHQ-2 |
| RMRPO3H533P09 | 258 | ECCGGCQACCGACAGTCGGCP | E-Threo-HEX::P-Phosphate::Q-BHQ-2 |
| RMRPO3H533P10 | 259 | ECGCCGGQGGCCCGGCGGP | E-Threo-HEX::P-Phosphate::Q-BHQ-2 |
| RMRPO3H533P11 | 260 | ECGCCGQGGGCCCGGCGP | E-Threo-HEX::P-Phosphate::Q-BHQ-2 |
| RMRPO3H533P12 | 261 | ECGCCGGQGGCCCGGCP | E-Threo-HEX::P-Phosphate::Q-BHQ-2 |
| RMRPO3H533P8B | 262 | ECCGGCQGCCGACAGTCGGP | E-Threo-HEX::P-Phosphate::Q-BHQ-2 |
| RMRPO3H533P8C | 263 | ECCGGCQGCCGACAGTCGP | E-Threo-HEX::P-Phosphate::Q-BHQ-2 |
| RMRPO3H533P13 | 264 | FCGCCGQGGGCCGGCCP | F-Threo-FAM::P-Phosphate::Q-BHQ-2 |
| RMRPO3H533P10C | 265 | FCGCCGGQGGCCCGGCGGP | F-Threo-FAM:P-Phosphate::Q-BHQ-2 |
| RMRPO3H533P10E | 266 | FCGCCGQGGGCCCGGCGGP | F-Threo-FAM:P-Phosphate::Q-BHQ-2 |
| RMRPO3533P10C2 | 267 | FCGCCGGQGGCCCGGCP | F-Threo-FAM:P-Phosphate::Q-BHQ-2 |
| RMRPO3533P12B | 268 | FCGCCGQGGGCCCGGCP | F-Threo-FAM::P-Phosphate::Q-BHQ-2 |
| RMRPO3533P12C | 269 | FCGCCQGGGGCCCGGCP | F-Threo-FAM::P-Phosphate::Q-BHQ-2 |
| RMRPO3533P12B2 | 270 | FCGCCGQGGGCCCGGCGCP | F-Threo-FAM::P-Phosphate::Q-BHQ-2 |
| RMRPO3533P12C2 | 271 | FCGCCQGGGGCCCGGCGGP | F-Threo-FAM::P-Phosphate::Q-BHQ-2 |
| RMRPO3533P12C3 | 272 | FCGCCQGAGGCCCGGCGGP | F-Threo-FAM::P-Phosphate::Q-BHQ-2 |
| RMRPO3533P12C4 | 273 | FAGCCQGGGGCCCGGCGGP | F-Threo-FAM::P-Phosphate::Q-BHQ-2 |
| RMRPO3533P13 | 274 | FCCGGGQGCCCGGCGGP | F-Threo-FAM::P-Phosphate::Q-BHQ-2 |
| RMRPO3533P14 | 275 | FCCGGGGQCCCGGCGGP | F-Threo-FAM::P-Phosphate::Q-BHQ-2 |
| RMRPO3H533P02B | 276 | ECCGGCGQCCGACAGTCP | E-Threo-HEX::P-Phosphate::Q-BHQ-2 |
| | | | |

| **rpoB 511P** | | **CTG/CCG** | **Leu/Pro** |
|---|---|---|---|
| **Oligo Name** | **SEQ ID NO:** | **Sequence** | **Modifications** |
| AYRPO3511PFM01 | 277 | FCAGCQCGAGCCAATTCATGP | F-Threo-FAM::P-Phosphate::Q-BHQ-2 |
| AYRPO3511PFM02 | 278 | FCAGCCQGAGCCAATTCATGP | F-Threo-FAM::P-Phosphate::Q-BHQ-2 |
| AYRPO3511PFM03 | 279 | FCAGCQJGAGCCAATTCATGP | F-Threo-FAM::P-Phosphate::Q- BHQ-2::J-G-Clamp |
| AYRPO3511PFa0 | 280 | FCAGCJGQAGCCAATTCATGP | F-Threo-FAM::P-Phosphate::Q- BHQ-2::J-G-Clamp |
| AYRPO3511PFM05 | 281 | FCAGCCGQAGCCAATTCATGP | F-Threo-FAM::P-Phosphate::Q-BHQ-2 |
| | | | |

| **rpoB 526R** | | **CAC/CGC** | **His/Arg** |
|---|---|---|---|
| **Oligo Name** | **SEQ ID NO:** | **Sequence** | **Modifications** |
| AYRPO3526RFM01 | 282 | FCTTGCGQGGTCAACCCCGAP | F-Threo-FAM::P-Phosphate::Q-BHQ-2 |
| AYRPO3526RFM02 | 283 | FTGCGGGQTCAACCCCGAP | F-Threo-FAM::P-Phosphate::Q-BHQ-2 |
| AYRPO3526RFM03 | 284 | FTGCGGGTCQAACCCCGAP | F-Threo-FAM::P-Phosphate::Q-BHQ-2 |
| AYRPO3526RFM04 | 285 | FCTTGCGGGQTCAACCCCGAP | F-Threo-FAM::P-Phosphate::Q-BHQ-2 |
| AYRPO3526RFM05 | 286 | FCTTGJGQGGTCAACCCCGAP | F-Threo-FAM::P-Phosphate::Q- BHQ-2::J-G-Clamp |
| AYRPO3526RFM06 | 287 | FTGJGGGQTCAACCCCGAP | F-Threo-FAM::P-Phosphate::Q- BHQ-2::J-G-Clamp |
| AYRPO3526RFM07 | 288 | FTGJGGGTCQAACCCCGAP | F-Threo-FAM::P-Phosphate::Q- BHQ-2::J-G-Clamp |
| AYRPO3526RFM08 | 289 | FCTTGJGGGQTCAACCCCGAP | F-Threo-FAM::P-Phosphate::Q- BHQ-2::J-G-Clamp |
| AYRPO3526RFM11 | 437 | ECTTGJQGGGTCAACCCCGAP | E-Threo-HEX::P-Phosphate::Q- BHQ-2::J-G-Clamp |

| **katG 315I** | | **AGC/ATC** | |
|---|---|---|---|
| **Oligo Name** | **SEQ ID NO:** | **Sequence** | **Modifications** |
| AYKAT315ICM01 | 290 | FGATCACCATCGGCATCGAQ | F-Threo-Coum343::Q-BHQ-2 |
| AYKAT315ICM02 | 291 | FCACCATQCGGCATCGAGGTCP | F-Threo-Coum343::P-Phosphate::Q-BHQ-2 |
| AYKAT315ICM03 | 292 | FCATCGGQCATCGAGGTCGTAP | F-Threo-Coum343::P-Phosphate::Q-BHQ-2 |
| AYKAT315ICM04 | 293 | FCACCATQCGGCATCGAGGTCGTAP | F-Threo-Coum343::P-Phosphate::Q-BHQ-2 |
| AYKAT315ICM05 | 294 | FATCGGCQATCGAGGTCGTAP | F-Threo-Coum343::P-Phosphate::Q-BHQ-2 |
| AYKAT315ICM06 | 295 | FAUCGGCQAUCGAGGUCGUAP | F-Threo-Coum343::P-Phosphate::Q-BHQ-2:: U-5-Propynyl dU |
| AYKAT315ICM07 | 296 | FAULGGLQAULGAGGULGUAP | F-Threo-Coum343::P-Phosphate::Q-BHQ-2:: U-5-Propynyl dU:: L-5-Propynyl dC |
| AYKAT315ICM03a | 297 | FCATCGQGCATCGAGGTCGTAP | F-Threo-Coum343::P-Phosphate::Q-BHQ-2 |
| AYKAT315ICM03b | 298 | FCATCGGCAQTCGAGGTCGTAP | F-Threo-Coum343::P-Phosphate::Q-BHQ-2 |
| AYKAT315ICM03c | 299 | FCATCGGCATCQGAGGTCGTAP | F-Threo-Coum343::P-Phosphate::Q-BHQ-2 |
| AYKAT315ICM03d | 300 | FCATCGGCQATCGAGGTCGTAP | F-Threo-Coum343::P-Phosphate::Q-BHQ-2 |
| AYKAT315ICY03a1 | 301 | FCATCGQGCATCGAGGTCGTAP | F-CY5.5::P-Phosphate::Q-BHQ-2 |
| AYKAT315ICY03a2 | 302 | FCATCGGCAQTCGAGGTCGTAP | F-CY5.5::P-Phosphate::Q-BHQ-2 |
| AYKAT315ICY03a3 | 303 | FCATCGGCATCQGAGGTCGTAP | F-CY5.5::P-Phosphate::Q-BHQ-2 |
| AYKAT315ICY03a4 | 304 | FCATCGGCATCGAQGGTCGTAP | F-CY5.5::P-Phosphate::Q-BHQ-2 |

| **katG 315N** | | **AGC/AAC** | |
|---|---|---|---|
| **Oligo Name** | **SEQ ID NO:** | **Sequence** | **Modifications** |
| AYKAT315NCM01 | 305 | FCAACGQGCATCGAGGTCGTAP | F-Threo-Coum343::P-Phosphate::Q-BHQ-2 |
| AYKAT315NCM02 | 306 | FCAACGGCAQTCGAGGTCGTAP | F-Threo-Coum343::P-Phosphate::Q-BHQ-2 |
| AYKAT315NCM03 | 307 | FCAACGGCATCQGAGGTCGTAP | F-Threo-Coum343::P-Phosphate::Q-BHQ-2 |
| AYKAT315NCM04 | 308 | FCAACGGQCATCGAGGTCGTAP | F-Threo-Coum343::P-Phosphate::Q-BHQ-2 |
| AYKAT315NCM05 | 309 | FCAACGGCQATCGAGGTCGTAP | F-Threo-Coum343::P-Phosphate::Q-BHQ-2 |
| AYKAT315NCM04a | 310 | FCAACGGQCATCGAGGTCGTAP | F-Threo-Coum343::P-Phosphate::Q-BHQ-2 |
| AYKAT315NCM05a | 311 | FCAACGGCQATCGAGGTCGTAP | F-Threo-Coum343::P-Phosphate::Q-BHQ-2 |
| AYKAT315NCM07 | 312 | FCAALGQGCATLGAGGTLGTAP | F-Threo-Coum343::P-Phosphate::Q-BHQ-2::L-5_Me_dC |
| AYKAT315NCM08 | 313 | FAACGGCQATCGAGGTCGTAP | F-Threo-Coum343::P-Phosphate::Q-BHQ-2 |
| AYKAT315NCM09 | 314 | FCACCAAQCGGCATCGAGGTCP | F-Threo-Coum343::P-Phosphate::Q-BHQ-2 |
| AYKAT315NCM10 | 315 | FCACCAAQCGGCATCGAGGTCGTAP | F-Threo-Coum343::P-Phosphate::Q-BHQ-2 |
| AYKAT315NCM11 | 316 | FLAALGQGLATLGAGGTLGTAP | F-Threo-Coum343::P-Phosphate::Q-BHQ-2 |
| AYKAT315NCM12 | 317 | FAALGGLQATLGAGGTLGTAP | F-Threo-Coum343::P-Phosphate::Q-BHQ-2::L-5_Me_dC |
| AYKAT315NCM13 | 318 | FLALLAAQLGGLATLGAGGTLP | F-Threo-Coum343::P-Phosphate::Q-BHQ-2::L-5_Me_dC |
| AYKAT315NCM14 | 319 | FLALLAAQLGGLATLGAGGTLGTAP | F-Threo-Coum343::P-Phosphate::Q-BHQ-2::L-5_Me_dC |
| AYKAT315NCM15 | 320 | FLAALGQGLATLGAGGTLGTAP | F-Threo-Coum343::P-Phosphate::Q-BHQ-2:: L-5-Propynyl dC |
| AYKAT315NCM16 | 321 | FAALGGLQATLGAGGTLGTAP | F-Threo-Coum343::P-Phosphate::Q-BHQ-2:: L-5-Propynyl dC |
| AYKAT315NCM17 | 322 | FLALLAAQLGGLATLGAGGTLP | F-Threo-Coum343::P-Phosphate::Q-BHQ-2:: L-5-Propynyl dC |
| AYKAT315NCM18 | 323 | FLALLAAQLGGLATLGAGGTLGTAP | F-Threo-Coum343::P-Phosphate::Q-BHQ-2:: L-5-Propynyl dC |
| AYKAT315NCM19 | 324 | FCAACQGGCATCGAGGTCGTAP | F-Threo-Coum343::P-Phosphate::Q-BHQ-2 |
| AYKAT315NCM20 | 325 | FCCAACGQGCATCGAGGTCGTAP | F-Threo-Coum343::P-Phosphate::Q-BHQ-2 |
| AYKAT315NCM21 | 326 | FCCAACQGGCATCGAGGTCGTAP | F-Threo-Coum343::P-Phosphate::Q-BHQ-2 |
| AYKAT315NCM22 | 327 | FACCAACQGGCATCGAGGTCGTAP | F-Threo-Coum343::P-Phosphate::Q-BHQ-2 |
| AYKAT315NCM23 | 328 | FACCAAQCGGCATCGAGGTCGTAP | F-Threo-Coum343::P-Phosphate::Q-BHQ-2 |
| RM315N2B | 434 | FCAAJQGCAUCGAGGUCGUAP | F-Threo-Coum343::P-Phosphate::Q- BHQ-2::J-G-Clamp::U-5-Propynyl dU |
| | | | |

| **katG 315T** | | **AGC/ACC** | |
|---|---|---|---|
| **Oligo Name** | **SEQ ID NO:** | **Sequence** | **Modifications** |
| AYKAT315TCM01 | 329 | FCACCACQCGGCATCGAGGTCP | F-Threo-Coum343::P-Phosphate::Q-BHQ-2 |
| AYKAT315TCM02 | 330 | FCACCAJQCGGCATCGAGGTCP | F-Threo-Coum343::P-Phosphate::Q- BHQ-2::J-G-Clamp |
| AYKAT315TCM03 | 331 | FCAJCGGQCATCGAGGTCGTAP | F-Threo-Coum343::P-Phosphate::Q- BHQ-2::J-G-Clamp |
| AYKAT315TCM04 | 332 | FCAJCGGQCATCGAGGTCP | F-Threo-Coum343::P-Phosphate::Q- BHQ-2::J-G-Clamp |
| AYKAT315TCM05 | 333 | FCACCAJQCGGCATCGAP | F-Threo-Coum343::P-Phosphate::Q- BHQ-2::J-G-Clamp |
| AYKAT315TCM05a | 334 | FCACCAQJCGGCATCGAP | F-Threo-Coum343::P-Phosphate::Q- BHQ-2::J-G-Clamp |
| AYKAT315TCM05b | 335 | FCACCAJCGQGCATCGAP | F-Threo-Coum343::P-Phosphate::Q- BHQ-2::J-G-Clamp |
| AYKAT315TCM05c | 336 | FCACCAJCGGCQATCGAP | F-Threo-Coum343::P-Phosphate::Q- BHQ-2::J-G-Clamp |
| AYKAT315TCY05b1 | 337 | FCACCAJCGQGCATCGAP | F-CY5.5::P-Phosphate::Q-BHQ-2::J-G-Clamp |
| AYKAT315TCY05b2 | 338 | FCACCAJCGGCQATCGAP | F-CY5.5::P-Phosphate::Q-BHQ-2::J-G-Clamp |
| AYKAT315TCY05b3 | 339 | FCACCAJCGGCATQCGAP | F-CY5.5::P-Phosphate::Q-BHQ-2::J-G-Clamp |
| AYKAT315TCY05b4 | 340 | FCACCAJCGGCATCGAQ | F-CY5.5::Q-BHQ-2::J-G-Clamp |
| | | | |

| **katG 315T2** | | **AGC/ACA** | |
|---|---|---|---|
| **Oligo Name** | **SEQ ID NO:** | **Sequence** | **Modifications** |
| AYKAT315T2CM01 | 341 | FCACCAQJAGGCATCGAP | F-Threo-Coum343::P-Phosphate::Q- BHQ-2::J-G-Clamp |
| AYKAT315T2CM02 | 342 | FCACCAJAGQGCATCGAP | F-Threo-Coum343::P-Phosphate::Q- BHQ-2::J-G-Clamp |
| AYKAT315T2CM03 | 343 | FCACCAJAGGCQATCGAP | F-Threo-Coum343::P-Phosphate::Q- BHQ-2::J-G-Clamp |
| RM315T2 | 435 | FACCACAQGGCATCGAGGTCGTAP | F-Threo-Coum343::P-Phosphate::Q-BHQ-2 |
| | | | |

| **inhA Probe Designs** | | | |
|---|---|---|---|
| **inhA -15T** | | **C → T** | |
| **Oligo Name** | **SEQ ID NO:** | **Sequence** | **Modifications** |
| AYINHA15TCM01 | 344 | FGCGAGAQTGATAGGTTGTCGGP | F-Threo-Coum343::P-Phosphate::Q-BHQ-2 |
| AYINHA15TCM02 | 345 | FGAGATGQATAGGTTGTCGGGGTGAP | F-Threo-Coum343::P-Phosphate::Q-BHQ-2 |
| AYINHA15TCM03 | 346 | FGLGAGAQUGAUAGGUUGULGGP | F-Threo-Coum343::P-Phosphate::Q-BHQ-2::L-5-Propynyl dC::U-5-Propynyl dU |
| AYINHA15TCM04 | 347 | FAGATGAQTAGGTTGTCGGGGTGAP | F-Threo-Coum343::P-Phosphate::Q-BHQ-2 |
| AYINHA15TCM05 | 348 | FAGAUGAQUAGGUUGULGGGGUGAP | F-Threo-Coum343::P-Phosphate::Q-BHQ-2::L-5-Propynyl dC::U-5-Propynyl dU |
| AYINHA15TCM06 | 349 | FGATGATQAGGTTGTCGGGGTGAP | F-Threo-Coum343::P-Phosphate::Q-BHQ-2 |
| AYINHA15TCM04a | 350 | FAGATGATAQGGTTGTCGGGGTGAP | F-Threo-Coum343::P-Phosphate::Q-BHQ-2 |
| AYINHA15TCM04b | 351 | FAGATGATAGGQTTGTCGGGGTGAP | F-Threo-Coum343::P-Phosphate::Q-BHQ-2 |
| AYINHA15TCM04c | 352 | FAGATQGATAGGTTGTCGGGGTGAP | F-Threo-Coum343::P-Phosphate::Q-BHQ-2 |
| AYINHA15TCM04d | 353 | FAGATQGATAGGTTGTCGGGGTGAP | F-Threo-Coum343::P-Phosphate::Q-BHQ-2 |
| AYINHA15TCM07 | 354 | FAGAUGAQUAGGUUGUCGGGGUGAP | F-Threo-Coum343::P-Phosphate::Q-BHQ-2::U-5-Propynyl dU |
| AYINHA15TCM08 | 355 | FGAUGAQUAGGUUGUCGGGGUGAP | F-Threo-Coum343::P-Phosphate::Q-BHQ-2::U-5-Propynyl dU |
| AYINHA15TCM09 | 356 | FAUGAQUAGGUUGUCGGGGUGAP | F-Threo-Coum343::P-Phosphate::Q-BHQ-2::U-5-Propynyl dU |
| AYINHA15TCM09a | 357 | FAUGAUQAGGUUGUCGGGGUGAP | F-Threo-Coum343::P-Phosphate::Q-BHQ-2::U-5-Propynyl dU |
| AYINHA15TCM09b | 358 | FATGAUQAGGUUGUCGGGGUGAP | F-Threo-Coum343::P-Phosphate::Q-BHQ-2::U-5-Propynyl dU |
| AYINHA15TCM06a | 359 | FGAUGAUQAGGUUGUCGGGGUGAP | F-Threo-Coum343::P-Phosphate::Q-BHQ-2::U-5-Propynyl dU |
| AYINHA15TCM06b | 360 | FGATGAQUAGGUUGUCGGGGUGAP | F-Threo-Coum343::P-Phosphate::Q-BHQ-2::U-5-Propynyl dU |
| JFINHA15TCM06A_1 | 361 | FGAUGAUQAGGUUGUCGJGGUGAP | F-Threo-Coum343::P-Phosphate::Q-BHQ-2::U-5-Propynyl dU::J-7_Dz_dG |
| JFINHA15TCM06A_2 | 362 | FGAUGAUQAGGUUGUCGGJGUGAP | F-Threo-Coum343::P-Phosphate::Q-BHQ-2::U-5-Propynyl dU::J-7_Dz_dG |
| JFINHA15TCM06B_1 | 363 | FGATGAQUAGGUUGUCGJGGUGAP | F-Threo-Coum343::P-Phosphate::Q-BHQ-2::U-5-Propynyl dU::J-7_Dz_dG |
| JFINHA15TCM06B_2 | 364 | FGATGAQUAGGUUGUCGGJGUGAP | F-Threo-Coum343::P-Phosphate::Q-BHQ-2::U-5-Propynyl dU::J-7_Dz_dG |
| JFYINHA15TCM09A_1 | 365 | FAUGAUQAGGUUGUCGJGGUGAP | F-Threo-Coum343::P-Phosphate::Q-BHQ-2::U-5-Propynyl dU::J-7_Dz_dG |
| JFINHA15TCM09A_1 | 366 | FAUGAUQAGGUUGUCGGJGUGAP | F-Threo-Coum343::P-Phosphate::Q-BHQ-2::U-5-Propynyl dU::J-7_Dz_dG |
| JFINHA15TCM09B_1 | 367 | FATGAUQAGGUUGUCGJGGUGAP | F-Threo-Coum343::P-Phosphate::Q-BHQ-2::U-5-Propynyl dU::J-7_Dz_dG |
| JFINHA15TCM09B_2 | 368 | FATGAUQAGGUUGUCGGJGUGAP | F-Threo-Coum343::P-Phosphate::Q-BHQ-2::U-5-Propynyl dU::J-7_Dz_dG |
| AYINHAR15TCM01 | 369 | FCTATCAQTCTCGCCGCGGCP | F-Threo-Coum343::P-Phosphate::Q-BHQ-2 |
| AYINHAR15TCM02 | 370 | FCTATCAQTCTCGCCGCGGCCP | F-Threo-Coum343::P-Phosphate::Q-BHQ-2 |
| AYINHAR15TCM03 | 371 | FCTATCAQTCTCGCCGCGGCCGP | F-Threo-Coum343::P-Phosphate::Q-BHQ-2 |
| AYINHAR15TCM04 | 372 | FTATCATQCTCGCCGCGGCCP | F-Threo-Coum343::P-Phosphate::Q-BHQ-2 |
| | | | |

| **inhA -8A** | | **T -> A** | |
|---|---|---|---|
| **Oligo Name** | **SEQ ID NO:** | **Sequence** | **Modifications** |
| AYINHA8ACM01 | 373 | FTAGGATQGTCGGGGTGACTGCCAP | F-Threo-Coum343::P-Phosphate::Q-BHQ-2 |
| AYINHA8ACM02 | 374 | FTAGGATQGTCGGGGTGACTGCP | F-Threo-Coum343::P-Phosphate::Q-BHQ-2 |
| AYINHA8ACM03 | 375 | FGATAGGQATGTCGGGGTGACTGCP | F-Threo-Coum343::P-Phosphate::Q-BHQ-2 |
| AYINHA8ACM04 | 376 | FUAGGAUQGULGGGGUGALUP | F-Threo-Coum343::P-Phosphate::Q-BHQ-2::U-5-Propynyl dU |
| AYINHA8ACM05 | 377 | FTAGGAQTGTCGGGGTGACTGCCAP | F-Threo-Coum343::P-Phosphate::Q-BHQ-2 |
| AYINHA8ACM06 | 378 | FTAGGATGTQCGGGGTGACTGCCAP | F-Threo-Coum343::P-Phosphate::Q-BHQ-2 |
| AYINHA8ACM07 | 379 | FTAGGATGTCGQGGGTGACTGCCAP | F-Threo-Coum343::P-Phosphate::Q-BHQ-2 |
| AYINHA8ACM08 | 380 | FTAGGQATGTCGGGGTGACTGCCAP | F-Threo-Coum343::P-Phosphate::Q-BHQ-2 |
| AYINHA8ACM08a | 381 | FUAGGQAUGUCGGGGUGACUGCCAP | F-Threo-Coum343::P-Phosphate::Q-BHQ-2::U-5-Propynyl dU |
| AYINHA8ACM08b | 382 | FUAGGQAUGULGGGGUGALUGLLAP | F-Threo-Coum343::P-Phosphate::Q-BHQ-2::U-5-Propynyl dU |
| AYINHAR8ACM01 | 383 | FCGACATQCCTATCGTCTCGCCGCP | F-Threo-Coum343::P-Phosphate::Q-BHQ-2 |
| AYINHAR8ACM02 | 384 | FGACATCQCTATCGTCTCGCCGCP | F-Threo-Coum343::P-Phosphate::Q-BHQ-2 |
| AYINHAR8ACM03 | 385 | FACATCCQTATCGTCTCGCCGCP | F-Threo-Coum343::P-Phosphate::Q-BHQ-2 |
| AYINHAR8ACM04 | 386 | FCATCCTQATCGTCTCGCCGCP | F-Threo-Coum343::P-Phosphate::Q-BHQ-2 |
| AYINHAR8ACM05 | 387 | FCGACATQCCTATCGTCTCGCCP | F-Threo-Coum343::P-Phosphate::Q-BHQ-2 |
| AYINHAR8ACM02a | 388 | FGACATCQCTATCGTCTCGCCGCP | F-Threo-Coum343::P-Phosphate::Q-BHQ-2 |
| AYINHAR8ACM02b | 389 | FGACATCCQTATCGTCTCGCCGCP | F-Threo-Coum343::P-Phosphate::Q-BHQ-2 |
| AYINHAR8ACM02c | 390 | FGACATCCTAQTCGTCTCGCCGCP | F-Threo-Coum343::P-Phosphate::Q-BHQ-2 |
| AYINHAR8ACM02d | 391 | FGACATQCCTATCGTCTCGCCGCP | F-Threo-Coum343::P-Phosphate::Q-BHQ-2 |
| AYINHAR8ACM02e | 392 | FGACATCQCUAUCGUCUCGCCGCP | F-Threo-Coum343::P-Phosphate::Q-BHQ-2::U-5-Propynyl dU |
| AYINHAR8ACM02f | 393 | FGACATCCQUAUCGUCUCGCCGCP | F-Threo-Coum343::P-Phosphate::Q-BHQ-2::U-5-Propynyl dU |
| AYINHAR8ACM02g | 394 | FGACATCCUAQUCGUCUCGCCGCP | F-Threo-Coum343::P-Phosphate::Q-BHQ-2::U-5-Propynyl dU |
| AYINHAR8ACM02h | 395 | FGACATQCCUAUCGUCUCGCCGCP | F-Threo-Coum343::P-Phosphate::Q-BHQ-2::U-5-Propynyl dU |
| JFINHA8ACM08A_1 | 396 | FUAGGQAUGUCGJGGUGACUGCCAP | F-Threo-Coum343::P-Phosphate::Q-BHQ-2::U-5-Propynyl dU::J-7_Dz_dG |
| JFINHA8ACM08A_2 | 397 | FUAGGQAUGUCGGJGUGACUGCCAP | F-Threo-Coum343::P-Phosphate::Q-BHQ-2::U-5-Propynyl dU::J-7_Dz_dG |
| JFINHA8ACM08B_1 | 398 | FUAGGQAUGULGJGGUGALUGLLAP | F-Threo-Coum343::P-Phosphate::Q-BHQ-2::U-5-Propynyl dU::J-7_Dz_dG::L-5_Me_dC |
| JFINHA8ACM08B_2 | 399 | FUAGGQAUGULGGJGUGALUGLLAP | F-Threo-Coum343::P-Phosphate::Q-BHQ-2::U-5-Propynyl dU::J-7_Dz_dG::L-5_Me_dC |
| | | | |

| **inhA -8C** | | **T -> C** | |
|---|---|---|---|
| **Oligo Name** | **SEQ ID NO:** | **Sequence** | **Modifications** |
| AYINHA8ACM01 | 400 | FTAGGCTQGTCGGGGTGACTGCP | F-Threo-Coum343::P-Phosphate::Q-BHQ-2 |
| AYINHA8ACM02 | 401 | FTAGGJTQGTCGGGGTGACTGCP | F-Threo-Coum343::P-Phosphate::Q-BHQ-2::J-G-Clamp |
| AYINHA8ACM03 | 402 | FGATAGGQJTGTCGGGGTGACTGCP | F-Threo-Coum343::P-Phosphate::Q-BHQ-2::J-G-Clamp |
| AYINHA8ACM04 | 403 | FATAGGJQTGTCGGGGTGACTGCP | F-Threo-Coum343::P-Phosphate::Q-BHQ-2::J-G-Clamp |
| AYINHA8ACM05 | 404 | FAGGJTGQTCGGGGTGACTGCP | F-Threo-Coum343::P-Phosphate::Q-BHQ-2::J-G-Clamp |
| AYINHAR8CCM01 | 405 | FCGACAGQCCTATCGTCTCGCCGCP | F-Threo-Coum343::P-Phosphate::Q- BHQ-2 |
| AYINHAR8CCM02 | 406 | FGACAGCQCTATCGTCTCGCCGCP | F-Threo-Coum343::P-Phosphate::Q-BHQ-2 |
| AYINHAR8CCM03 | 407 | FACAGCCQTATCGTCTCGCCGCP | F-Threo-Coum343::P-Phosphate::Q-BHQ-2 |
| AYINHAR8CCM04 | 408 | FCAGCCTQATCGTCTCGCCGCP | F-Threo-Coum343::P-Phosphate::Q-BHQ-2 |
| AYINHAR8CCM05 | 409 | FCGACAGQCCTATCGTCTCGCCP | F-Threo-Coum343::P-Phosphate::Q-BHQ-2 |
| RMINHA8C4BB | 436 | FACAGCQCUAUCGUCUCGCP | F-Threo-Coum343::P-Phosphate::Q-BHQ-2:: U-5-Propynyl dU |

In one embodiment, the above described a plurality of sets of *rpo*B, *inh*A, and *kat*G primers and probes are used in order to provide for detection of MTB-RIF and/or MTB-INH in a biological sample suspected of containing MTB-RIF and/or MTB-INH. The sets of primers and probes may comprise or consist of the primers and probes specific for the *rpo*B, *inh*A, and *kat*G nucleic acid sequences, comprising or consisting of the nucleic acid sequences of SEQ ID NOs: 1-409 and 434-438. In another embodiment, the primers and probes for the *rpo*B*, inh*A, and *kat*G targets comprise or consist of a functionally active variant of any of the primers of SEQ ID NOs: 1-409 and 434-438.

A functionally active variant of any of the probes of SEQ ID NOs: 1-409 and 434-438 may be identified by using the probes in the disclosed method. A functionally active variant of a probe of any of the SEQ ID NOs: 1-409 and 434-438pertains to a primer which provides a similar or higher specificity and sensitivity in the method or kit described herein as compared to the respective sequence of SEQ ID NOs: 1-409 and 434-438.

The variant may, e.g., vary from the sequence of SEQ ID NOs: 1-409 and 434-438by one or more nucleotide additions, deletions or substitutions such as one or more nucleotide additions, deletions or substitutions at the 5' end and/or the 3' end of the respective sequence of SEQ ID NOs: 1-409 and 434-438. As detailed above, a primer (and/or probe) may be chemically modified, i.e., a primer and/or probe may comprise a modified nucleotide or a non-nucleotide compound. A probe (or a primer) is then a modified oligonucleotide. "Modified nucleotides" (or "nucleotide analogs") differ from a natural "nucleotide" by some modification but still consist of a base or base-like compound, a pentofuranosyl sugar or a pentofuranosyl sugar-like compound, a phosphate portion or phosphate-like portion, or combinations thereof. For example, a "label" may be attached to the base portion of a "nucleotide" whereby a "modified nucleotide" is obtained. A natural base in a "nucleotide" may also be replaced by, e.g., a 7-desazapurine whereby a "modified nucleotide" is obtained as well. The terms "modified nucleotide" or "nucleotide analog" are used interchangeably in the present application. A "modified nucleoside" (or "nucleoside analog") differs from a natural nucleoside by some modification in the manner as outlined above for a "modified nucleotide" (or a "nucleotide analog").

Oligonucleotides including modified oligonucleotides and oligonucleotide analogs that amplify a nucleic acid molecule encoding the *rpoB, inh*A, and *katG* nucleic acid sequences, e.g., nucleic acids encoding alternative portions of *rpoB, inh*A, and *katG* can be designed using, for example, a computer program such as OLIGO (Molecular Biology Insights Inc., Cascade, Colo.). Important features when designing oligonucleotides to be used as amplification primers include, but are not limited to, an appropriate size amplification product to facilitate detection (e.g., by electrophoresis), similar melting temperatures for the members of a pair of primers, and the length of each primer (i.e., the primers need to be long enough to anneal with sequence-specificity and to initiate synthesis but not so long that fidelity is reduced during oligonucleotide synthesis). Typically, oligonucleotide primers are 8 to 50, particularly 10 to 40 or 12 to 40 nucleotides in length (e.g., 8, 10, 12, 14, 16, 18, 20, 22, 24, 26, 28, 30, 32, 34, 36, 38, 40, 42, 44, 46, 48, or 50 nucleotides in length).

In addition to a set of primers, the disclosed methods may use one or more probes in order to detect the presence or absence of MTB-RIF and/or MTB-INH. The term "probe" refers to synthetically or biologically produced nucleic acids (DNA or RNA), which by design or selection, contain specific nucleotide sequences that allow them to hybridize under defined predetermined stringencies specifically (i.e., preferentially) to "target nucleic acids", in the present case to a MTB-RIF and/or MTB-INH (target) nucleic acid. A "probe" can be referred to as a "detection probe" meaning that it detects the target nucleic acid.

In some embodiments, the described *rpoB, inh*A, and *katG* probes can be labeled with at least one fluorescent label. In one embodiment, the *rpo*B, *inh*A, and *kat*G probes can be labeled with a donor fluorescent moiety, e.g., a fluorescent dye, and a corresponding acceptor fluorescent moiety, e.g., a quencher.

In one embodiment, the probes comprise or consist of a fluorescent moiety and the nucleic acid sequences comprise or consist of SEQ ID NOs: 1-409 and 434-438.

Designing oligonucleotides to be used as hybridization probes can be performed in a manner similar to the design of primers. Embodiments may use a single probe or a pair of probes for detection of the amplification product. Depending on the embodiment, the probe(s) use may comprise at least one label and/or at least one quencher moiety. As with the primers, the probes usually have similar melting temperatures, and the length of each probe must be sufficient for sequence-specific hybridization to occur but not so long that fidelity is reduced during synthesis. Oligonucleotide probes generally have 40 or fewer nucleotides and are particularly between 12 to 40, 15 to 40 and 15 to 30 (e.g., 16, 18, 20, 21, 22, 23, 24, or 25) nucleotides in length.

### Polymerase Chain Reaction (PCR)

U.S. Pat. Nos. 4,683,202, 4,683,195, 4,800,159, and 4,965,188 disclose conventional PCR techniques. PCR typically employs two oligonucleotide primers that bind to a selected nucleic acid template (e.g., DNA or RNA). Primers useful in some embodiments include oligonucleotides capable of acting as points of initiation of nucleic acid synthesis within the described *rpoB, inh*A, and *katG* nucleic acid sequences. A primer can be purified from a restriction digest by conventional methods, or it can be produced synthetically. The primer is preferably single-stranded for maximum efficiency in amplification, but the primer can be double-stranded. Double-stranded primers are first denatured, i.e., treated to separate the strands. One method of denaturing double stranded nucleic acids is by heating.

If the template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (e.g., greater than 50%, 60%, 70%, 80%, 90% or 95% denatured). The heating conditions necessary for denaturing template nucleic acid will depend, e.g., on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min (e.g., 1 min to 2 min 30 sec, or 1.5 min).

If the double-stranded template nucleic acid is denatured by heat, the reaction mixture is allowed to cool to a temperature that promotes annealing of each primer to its target sequence on the described *rpo*B, *inh*A, and *kat*G nucleic acid molecules. The temperature for annealing is usually from about 35°C to about 65°C (e.g., about 40°C to about 60°C; about 45°C to about 50°C). Annealing times can be from about 10 sec to about 1 min (e.g., about 20 sec to about 50 sec; about 30 sec to about 40 sec). The reaction mixture is then adjusted to a temperature at which the activity of the polymerase is promoted or optimized, i.e., a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template (e.g., the temperature for extension generally ranges from about 40°C to about 80°C (e.g., about 50°C to about 70°C; about 60°C). Extension times can be from about 10 sec to about 5 min (e.g., about 30 sec to about 4 min; about 1 min to about 3 min; about 1 min 30 sec to about 2 min).

PCR assays can employ MTB-RIF and/or MTB-INH nucleic acid such as RNA or DNA (cDNA). The template nucleic acid need not be purified; it may be a minor fraction of a complex mixture, such as MTB-RIF and/or MTB-INH nucleic acid contained in human cells. MTB-RIF and/or MTB-INH nucleic acid molecules may be extracted from a biological sample by routine techniques such as those described in Diagnostic Molecular Microbiology: Principles and Applications (Persing et al. (eds), 1993, American Society for Microbiology, Washington D.C.). Nucleic acids can be obtained from any number of sources, such as plasmids, or natural sources including bacteria, yeast, viruses, organelles, or higher organisms such as plants or animals.

The oligonucleotide primers are combined with PCR reagents under reaction conditions that induce primer extension. For example, chain extension reactions generally include 50 mM KCl, 10 mM Tris-HCl (pH 8.3), 15 mM MgCl₂, 0.001% (w/v) gelatin, 0.5-1.0 µg denatured template DNA, 50 pmoles of each oligonucleotide primer, 2.5 U of Taq polymerase, and 10% DMSO). The reactions usually contain 150 to 320 µM each of dATP, dCTP, dTTP, dGTP, or one or more analogs thereof.

The newly synthesized strands form a double-stranded molecule that can be used in the succeeding steps of the reaction. The steps of strand separation, annealing, and elongation can be repeated as often as needed to produce the desired quantity of amplification products corresponding to the target MTB-RIF and/or MTB-INH nucleic acid molecules. The limiting factors in the reaction are the amounts of primers, thermostable enzyme, and nucleoside triphosphates present in the reaction. The cycling steps (i.e., denaturation, annealing, and extension) are preferably repeated at least once. For use in detection, the number of cycling steps will depend, e.g., on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times.

### Fluorescence Resonance Energy Transfer (FRET)

FRET technology (see, for example, U.S. Pat. Nos. 4,996,143, 5,565,322, 5,849,489, and 6,162,603) is based on a concept that when a donor fluorescent moiety and a corresponding acceptor fluorescent moiety are positioned within a certain distance of each other, energy transfer takes place between the two fluorescent moieties that can be visualized or otherwise detected and/or quantitated. The donor typically transfers the energy to the acceptor when the donor is excited by light radiation with a suitable wavelength. The acceptor typically re-emits the transferred energy in the form of light radiation with a different wavelength.

In one example, a oligonucleotide probe can contain a donor fluorescent moiety and a corresponding quencher, which may or not be fluorescent, and which dissipates the transferred energy in a form other than light. When the probe is intact, energy transfer typically occurs between the two fluorescent moieties such that fluorescent emission from the donor fluorescent moiety is quenched. During an extension step of a polymerase chain reaction, a probe bound to an amplification product is cleaved by the 5' to 3' nuclease activity of, e.g., a Taq Polymerase such that the fluorescent emission of the donor fluorescent moiety is no longer quenched. Exemplary probes for this purpose are described in, e.g., U.S. Pat. Nos. 5,210,015, 5,994,056, and 6,171,785. Commonly used donor-acceptor pairs include the FAM-TAMRA pair. Commonly used quenchers are DABCYL and TAMRA. Commonly used dark quenchers include BlackHole Quenchers™ (BHQ), (Biosearch Technologies, Inc., Novato, Cal.), Iowa Black™, (Integrated DNA Tech., Inc., Coralville, Iowa), BlackBerry™ Quencher 650 (BBQ-650), (Berry & Assoc., Dexter, Mich.).

In another example, two oligonucleotide probes, each containing a fluorescent moiety, can hybridize to an amplification product at particular positions determined by the complementarity of the oligonucleotide probes to the MTB-RIF and/or MTB-INH target nucleic acid sequence. Upon hybridization of the oligonucleotide probes to the amplification product nucleic acid at the appropriate positions, a FRET signal is generated. Hybridization temperatures can range from about 35° C. to about 65° C. for about 10 sec to about 1 min.

Fluorescent analysis can be carried out using, for example, a photon counting epifluorescent microscope system (containing the appropriate dichroic mirror and filters for monitoring fluorescent emission at the particular range), a photon counting photomultiplier system, or a fluorometer. Excitation to initiate energy transfer can be carried out with an argon ion laser, a high intensity mercury (Hg) arc lamp, a fiber optic light source, or other high intensity light source appropriately filtered for excitation in the desired range.

As used herein with respect to donor and corresponding acceptor fluorescent moieties "corresponding" refers to an acceptor fluorescent moiety having an emission spectrum that overlaps the excitation spectrum of the donor fluorescent moiety. The wavelength maximum of the emission spectrum of the acceptor fluorescent moiety should be at least 100 nm greater than the wavelength maximum of the excitation spectrum of the donor fluorescent moiety. Accordingly, efficient non-radiative energy transfer can be produced there between.

Fluorescent donor and corresponding acceptor moieties are generally chosen for (a) high efficiency Forster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, Helium-Cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (>600 nm).

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothio-cyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC Red 640, LC Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate, or other chelates of Lanthanide ions (e.g., Europium, or Terbium). Donor and acceptor fluorescent moieties can be obtained, for example, from Molecular Probes (Junction City, Oreg.) or Sigma Chemical Co. (St. Louis, Mo.).

The donor and acceptor fluorescent moieties can be attached to the appropriate probe oligonucleotide via a linker arm. The length of each linker arm is important, as the linker arms will affect the distance between the donor and acceptor fluorescent moieties. The length of a linker arm is the distance in Angstroms (Å) from the nucleotide base to the fluorescent moiety. In general, a linker arm is from about 10 Å to about 25 Å. The linker arm may be of the kind described in WO 84/03285. WO 84/03285 also discloses methods for attaching linker arms to a particular nucleotide base, and also for attaching fluorescent moieties to a linker arm.

An acceptor fluorescent moiety, such as an LC Red 640, can be combined with an oligonucleotide which contains an amino linker (e.g., C6-amino phosphoramidites available from ABI (Foster City, Calif.) or Glen Research (Sterling, VA)) to produce, for example, LC Red 640-labeled oligonucleotide. Frequently used linkers to couple a donor fluorescent moiety such as fluorescein to an oligonucleotide include thiourea linkers (FITC-derived, for example, fluorescein-CPG's from Glen Research or ChemGene (Ashland, Mass.)), amide-linkers (fluorescein-NHS-ester-derived, such as CX-fluorescein-CPG from BioGenex (San Ramon, Calif.)), or 3'-amino-CPGs that require coupling of a fluorescein-NHS-ester after oligonucleotide synthesis.

### Detection of MTB-RIF and/or MTB-INH

The present disclosure provides methods for detecting the presence or absence of MTB-RIF and/or MTB-INH in a biological or non-biological sample. Methods provided herein avoid problems of sample contamination, false negatives, and false positives. The methods include performing at least one cycling step that includes amplifying a portion of *rpoB, inh*A, and *katG* target nucleic acid molecules from a sample using a plurality of pairs of *rpoB, inh*A, and *katG* primers, and a FRET detecting step. Multiple cycling steps are performed, preferably in a thermocycler. Methods described herein can be performed using the *rpoB, inh*A, and *katG* primers and probes to detect the presence of *rpoB, inh*A, and *katG* targets, and the detection of the described SNPs in the *rpoB, inh*A, and *katG* targets indicates the presence of MTB-RIF and/or MTB-INH in the sample.

As described herein, amplification products can be detected using labeled hybridization probes that take advantage of FRET technology. One FRET format utilizes TaqMan® technology to detect the presence or absence of an amplification product, and hence, the presence or absence of the target nucleic acid. TaqMan® technology utilizes one single-stranded hybridization probe labeled with, e.g., one fluorescent dye and one quencher, which may or may not be fluorescent. When a first fluorescent moiety is excited with light of a suitable wavelength, the absorbed energy is transferred to a second fluorescent moiety according to the principles of FRET. The second fluorescent moiety is generally a quencher molecule. During the annealing step of the PCR reaction, the labeled hybridization probe binds to the target DNA (i.e., the amplification product) and is degraded by the 5' to 3' nuclease activity of, e.g., the Taq Polymerase during the subsequent elongation phase. As a result, the fluorescent moiety and the quencher moiety become spatially separated from one another. As a consequence, upon excitation of the first fluorescent moiety in the absence of the quencher, the fluorescence emission from the first fluorescent moiety can be detected. By way of example, an ABI PRISM® 7700 Sequence Detection System (Applied Biosystems) uses TaqMan® technology, and is suitable for performing the methods described herein for detecting the presence or absence of the target nucleic acid in the sample.

Generally, the presence of FRET indicates the presence of MTB-RIF and/or MTB-INH in the sample, and the absence of FRET indicates the absence of MTB-RIF and/or MTB-INH in the sample. Inadequate specimen collection, transportation delays, inappropriate transportation conditions, or use of certain collection swabs (calcium alginate or aluminum shaft) are all conditions that can affect the success and/or accuracy of a test result, however. Using the methods disclosed herein, detection of FRET within, e.g., 45 cycling steps is indicative of an MTB-RIF and/or MTB-INH infection.

Representative biological samples that can be used include, but are not limited to dermal swabs, nasal swabs, wound swabs, blood cultures, skin, and soft tissue infections. Collection and storage methods of biological samples are known to those of skill in the art. Biological samples can be processed (e.g., by nucleic acid extraction methods and/or kits known in the art) to release MTB-RIF and/or MTB-INH nucleic acid or in some cases, the biological sample can be contacted directly with the PCR reaction components and the appropriate oligonucleotides.

Within each thermocycler run, control samples can be cycled as well. Positive control samples can amplify target nucleic acid control template (other than described amplification products of target genes) using, for example, control primers and control probes. Positive control samples can also amplify, for example, a plasmid construct containing the target nucleic acid molecules. Such a plasmid control can be amplified internally (e.g., within the sample) or in a separate sample run side-by-side with the patients' samples using the same primers and probe as used for detection of the intended target. Such controls are indicators of the success or failure of the amplification, hybridization, and/or FRET reaction. Each thermocycler run can also include a negative control that, for example, lacks target template DNA. Negative control can measure contamination. This ensures that the system and reagents would not give rise to a false positive signal. Therefore, control reactions can readily determine, for example, the ability of primers to anneal with sequence-specificity and to initiate elongation, as well as the ability of probes to hybridize with sequence-specificity and for FRET to occur.

In an embodiment, the methods include steps to avoid contamination. For example, an enzymatic method utilizing uracil-DNA glycosylase is described in U.S. Pat. Nos. 5,035,996, 5,683,896 and 5,945,313 to reduce or eliminate contamination between one thermocycler run and the next.

Conventional PCR methods in conjunction with FRET technology can be used. In one embodiment, a LightCycler® instrument is used. The following patent applications describe real-time PCR as used in the LightCycler® technology: WO 97/46707, WO 97/46714, and WO 97/46712.

The LightCycler® can be operated using a PC workstation and can utilize a Windows NT operating system. Signals from the samples are obtained as the machine positions the capillaries sequentially over the optical unit. The software can display the fluorescence signals in real-time immediately after each measurement. Fluorescent acquisition time is 10-100 milliseconds (msec). After each cycling step, a quantitative display of fluorescence vs. cycle number can be continually updated for all samples. The data generated can be stored for further analysis.

It is understood that the embodiments described herein are not limited by the configuration of one or more commercially available instruments.

### Articles of Manufacture/Kits

Embodiments of the present disclosure further provide for articles of manufacture or kits to detect MTB-RIF and/or MTB-INH. An article of manufacture can include primers and probes used to detect *rpo*B, *inh*A, and *kat*G, together with suitable packaging materials. Representative primers and probes for detection of MTB-RIF and/or MTB-INH are capable of hybridizing to *rpoB, inh*A, and *katG* target nucleic acid molecules. In addition, the kits may also include suitably packaged reagents and materials needed for DNA immobilization, hybridization, and detection, such solid supports, buffers, enzymes, and DNA standards. Methods of designing primers and probes are disclosed herein, and representative examples of primers and probes that amplify and hybridize to *rpo*B, *inh*A, and *kat*G target nucleic acid molecules are provided.

Articles of manufacture can also include one or more fluorescent moieties for labeling the probes or, alternatively, the probes supplied with the kit can be labeled. For example, an article of manufacture may include a donor and/or an acceptor fluorescent moiety for labeling the *rpo*B, *inh*A, and *kat*G probes. Examples of suitable FRET donor fluorescent moieties and corresponding acceptor fluorescent moieties are provided above.

Articles of manufacture can also contain a package insert or package label having instructions thereon for using the *rpoB, inh*A, and *katG* primers and probes to detect MTB-RIF and/or MTB-INH in a sample. Articles of manufacture may additionally include reagents for carrying out the methods disclosed herein (e.g., buffers, polymerase enzymes, co-factors, or agents to prevent contamination). Such reagents may be specific for one of the commercially available instruments described herein.

Embodiments of the present disclosure will be further described in the following examples, which do not limit the scope of the invention described in the claims.

### EXAMPLE

### Detection of specific SNPs using exemplary probes for rpoB, inhA, and katG

Exemplary probes were examined for their ability to detect 17 SNPs in the *rboB* gene which confer resistance to rifampicin in MTB including *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P, and *rpo*B 526R, 3 SNPs in the *inh*A gene which confer resistance to isoniazid in MTB including *inh*A-15T, *inh*A-8A, and *inh*A-8C and 4 SNPs in the *kat*G gene which also confer resistance to isoniazid in MTB including *kat*G 3151, *kat*G 315N, *kat*G 315T, and *kat*G 315T2.

Various plasmids comprising respective nucleic acids each representing one, two or three SNPs were used. Herein, in each well the Mastermix (MMX) comprising buffer, ZO5 DNA polymerase, nucleotide triphosphates, primer pairs capable of amplifying the *rpoB, inhA,* and *katG* target regions and the exemplary probes shown in Table II was spiked with one type of plasmidic DNA each comprising one, two or three particular SNPs as displayed in Table II. A plasmid comprising a 16S sequence was used as positive control using a 16S specific primer pair and probe. Subsequently, multi-well plates were loaded on the cobas® 6800 system (Roche Molecular Systems) and subjected to an amplification and detection reaction. Results are indicated in Table II.

**TABLE II: Results for Exemplary Probes for rpoB, inhA, and katG**

| **Gene** | **SNP** | **Probe** | **SEQ ID NO:** | **Plasmid** | **Mutant Plasmid** | **Positive signal** |
|---|---|---|---|---|---|---|
| **KatG** | 3151 | AYKAT315ICM03a | 297 | AYKATG02 | 315I | Yes |
| | 315N | RM315N2B | 434 | AYKATG03 | 315N | Yes |
| | 315T | AYKAT315TCM05b | 335 | AYKATG04 | 315T | Yes |
| | 315T2 | RM315T2 | 435 | AYKATG05 | 315T2 | Yes |
| **InhA** | 15T | JFINHA15TCM09B_1 | 363 | AYINHA02 | 15T | Yes |
| | 8A | AYINHAR8ACM02 | 374 | AYINHA03 | 8A | Yes |
| | 8C | RMINHA8C4BB | 436 | AYINHA04 | 8C | Yes |
| | 516V | RMRPO3516V11E6 | 238 | JLHRIF103 | 516V | Yes |
| | 531W | RMRPO3F531W06 | 55 | JLHRIF109 | 531W | Yes |
| | 516Y | RMRPO3S516YB2 | 240 | JLHRIF110 | 516Y | Yes |
| | 511P | AYRPO3511PFM04 | 280 | JLHRIF108 | 511P | Yes |
| | 526L | RMRPO3A08 (526L) | 58 | JLHRIF107 | 526L | Yes |
| | 531L | RM5L17B3A | 52 | JLHRIF101 | 531L | Yes |
| | 533P | RMRP03H533P10 | 121 | JLHRIF106 | 533P | Yes |
| | 526R | AYRPO3526RFM11 | 437 | JLHRIF105 | 526R | Yes |
| **RpoB** | 526N | RM_NEW526N11B3 | 438 | JLHRIF111 | 526N | Yes |
| | 526N | RMRPO3SP526N4B2 | 108 | JLHRIF111 | 526N | Yes |
| | 526Y | AYRPO3526YHX06 | 84 | JLHRIF102 | 526Y | Yes |
| | 522L | JJA522L33PJ12 | 180 | JJRPOB09 | 531W 513K 522L | Yes |
| | 513L | JJA513L75PJ12 | 142 | JLHRIF113 | 513L | Yes |
| | 526D | AYRPO3526DJA11 | 99 | JLHRIF104 | 526D | Yes |
| | 513K | JJA513K76PJ12 | 155 | JJRPOB09 | 531W 513K 522L | Yes |
| | 513P | JJS513P66PJ12 | 162 | JJRPOB010 | 522W 513P | Yes |
| | 522Q | JJA522Q20PJ12 | 196 | JJRPOB08 | 531L 522Q 513L | Yes |
| | 522W | JJA522W33PJ12 | 199 | JJRPOB010 | 522W 513P | Yes |
| **16S** | n/a | RMMTB16 | | pkY5 | n/a | Yes |

While the foregoing disclosure has been described in some detail for purposes of clarity and understanding, it will be clear to one skilled in the art from a reading of this disclosure that various changes in form and detail can be made without departing from the true scope of the disclosure. For example, all the techniques and apparatus described above can be used in various combinations.

## Claims

1. A method of detecting *Mycobacterium tuberculosis* (MTB) resistant to rifampicin (MTB-RIF) and/or MTB resistant to isoniazid (MTB-INH) in a sample, the method comprising a multiplex real-time polymerase chain reaction assay comprising:
- performing an amplifying step comprising contacting the sample with at least a set of *rpo*B primers, a set of *inh*A primers, and a set of *kat*G primers to produce one or more amplification products if any *rpo*B, *inh*A, and *katG* target nucleic acid is present in the sample;
- performing a hybridizing step comprising contacting said one or more amplification products with a plurality of detectable *rpoB* probes, a plurality of detectable *inh*A probes, and a plurality of detectable *kat*G probes, at least comprising:
- 17 *rpo*B probes for detection of one or more of 17 single nucleotide polymorphisms SNPs which confer rifampicin resistance to MTB;
- 3 *inh*A probes for detection of one or more of 3 SNPs which confer isoniazid resistance to MTB; and
- 4 *katG* probes for detection of one or more of 4 SNPs which confer isoniazid resistance to MTB; and
- detecting the presence or absence of said one or more amplification products,
wherein the presence of said one or more amplification products is indicative of the presence of MTB-RIF and/or MTB-INH in the sample and wherein the absence of said one or more amplification products is indicative of the absence of MTB-RIF and/or MTB-INH in the sample;
wherein said plurality of *rpo*B probes comprise hydrolysis probes for detection of each of the 17 SNPs which confer rifampicin resistance to MTB, comprising *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P, and *rpo*B 526R;
wherein said plurality of *inhA* probes comprise hydrolysis probes for detection of each of the 3 SNPs which confer isoniazid resistance to MTB, comprising *inh*A-15T, *inh*A-8A, and *inh*A-8C;
wherein said plurality of *kat*G probes comprise hydrolysis probes for detection of each of the 4 SNPs which confer isoniazid resistance to MTB, comprising *kat*G 3151, *kat*G 315N, *kat*G 315T, and *kat*G 315T2; and
wherein:
- the *rpo*B probes comprise a nucleic acid sequence selected from the group consisting of SEQ ID NOs: 1-289 and 437-438, or a complement thereof;
- the *inh*A probes comprise a nucleic acid sequence selected from the group consisting of SEQ ID NOs: 344-409 and 436, or a complement thereof; and
- the *katG* probes comprise a nucleic acid sequence selected from the group consisting of SEQ ID NOs: 290-343 and 434-435, or a complement thereof.

2. The method of claim 1, wherein each of the plurality of detectable *rpo*B, *inh*A, and *katG* probes is labeled with a donor fluorescent moiety and a corresponding acceptor fluorescent moiety; and
wherein the detecting step comprises detecting the presence or absence of fluorescence resonance energy transfer (FRET) between the donor fluorescent moiety and the acceptor fluorescent moiety of one or more of the *rpo*B, *inh*A, and *kat*G probes,
wherein the presence or absence of fluorescence emission is indicative of the presence or absence of MTB-RIF and/or MTB-INH in the sample.

3. The method of any one of claims 1 to 2, wherein said amplification employs a polymerase enzyme having 5' to 3' nuclease activity.

4. The method of any one of claims 2 and 3, wherein said donor and acceptor fluorescent moieties are within no more than 8 nucleotides of each other on said probe.

5. The method of any one of claims 2 to 4, wherein said acceptor fluorescent moiety is a quencher.

6. A kit for detecting a nucleic acid of *Mycobacterium tuberculosis* (MTB) resistant to rifampicin (MTB-RIF) and/or MTB resistant to isoniazid (MTB-INH), comprising:
- a plurality of sets of *rpo*B, *inh*A, and *kat*G primers specific for amplification of *rpo*B, *inh*A, and *kat*G gene targets; and
- a plurality of detectable *rpo*B, *inh*A, and *kat*G probes specific for detection of *rpo*B, *inh*A, and *kat*G amplification products, comprising:
- 17 *rpo*B probes for detection of one or more of 17 SNPs which confer rifampicin resistance to MTB;
- 3 *inh*A probes for detection of one or more of 3 SNPs which confer isoniazid resistance to MTB; and
- 4 *katG* probes for detection of one or more of 4 SNPs which confer isoniazid resistance to MTB;
wherein said plurality of *rpo*B probes comprise hydrolysis probes for detection of each of the 17 SNPs which confer rifampicin resistance to MTB, comprising *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P, and *rpo*B 526R;
wherein said plurality of *inh*A probes comprise hydrolysis probes for detection of each of the 3 SNPs which confer isoniazid resistance to MTB, comprising *inh*A-15T, *inh*A-8A, and *inh*A-8C;
wherein said plurality of *kat*G probes comprise hydrolysis probes for detection of each of the 4 SNPs which confer isoniazid resistance to MTB, comprising *kat*G 3151, *kat*G 315N, *kat*G 315T, and *kat*G 315T2; and
wherein:
- the *rpo*B probes comprise a nucleic acid sequence selected from the group consisting of SEQ ID NOs: 1-289 and 437-438, or a complement thereof;
- the *inh*A probes comprise a nucleic acid sequence selected from the group consisting of SEQ ID NOs: 344-409 and 436, or a complement thereof; and
- the *katG* probes comprise a nucleic acid sequence selected from the group consisting of SEQ ID NOs: 290-343 and 434-435, or a complement thereof.

7. The kit of claim 6, wherein each probe of said plurality of detectable *rpo*B, *inh*A, and *katG* probes comprises a donor fluorescent moiety and a corresponding acceptor fluorescent moiety.

8. The kit of claim 7, wherein the acceptor fluorescent moiety is a quencher.

9. The kit of any one of claims 6 to 8, further comprising nucleoside triphosphates, nucleic acid polymerase, and buffers necessary for the function of the nucleic acid polymerase.

10. An oligonucleotide probe comprising a nucleic acid sequence selected from the group consisting of SEQ ID NOs: 1-409 and 434-438, or a complement thereof and further comprising one or more detectable labels.

11. The oligonucleotide of claim 10, wherein the oligonucleotide comprises at least one modified nucleotide.

12. The oligonucleotide of any one of claims 10 and 11, wherein the oligonucleotide comprises at least one conservatively modified variation.

13. The oligonucleotide of any one of claims 10 to 12, wherein the oligonucleotide has 40 or fewer nucleotides.

14. The oligonucleotide of any one of claims 10 to 13, wherein the oligonucleotide comprises at least one labeling moiety and/or at least one quencher moiety.

## Patentansprüche

1. Verfahren zum Nachweisen von *Mycobacterium tuberculosis* (MTB), das gegen Rifampicin (MTB-RIF) resistent ist, und/oder MTB, das gegen Isoniazid (MTB-INH) resistent ist, in einer Probe, wobei das Verfahren einen Multiplex-Echtzeit-Polymerasekettenreaktions-Assay umfasst, umfassend:
- Durchführen eines Amplifizierungsschrittes, umfassend das Inkontaktbringen der Probe mit mindestens einem Satz von *rpo*B-Primern, einem Satz von *inh*A-Primern und einem Satz von *kat*G-Primem zur Erzeugung eines oder mehrerer Amplifikationsprodukte, wenn eine *rpo*B-, *inh*A- und *kat*G-Zielnukleinsäure in der Probe vorliegt;
- Durchführen eines Hybridisierungsschrittes, umfassend das Inkontaktbringen des einen oder der mehreren Amplifikationsprodukte mit einer Vielzahl von nachweisbaren *rpoB-*Sonden, einer Vielzahl von nachweisbaren *inhA*-Sonden und einer Vielzahl von nachweisbaren *kat*G-Sonden, die mindestens Folgendes umfassen:
- 17 *rpo*B-Sonden zum Nachweis von einem oder mehreren von 17 Einzelnukleotid-Polymorphismen, SNPs, die MTB Rifampicin-Resistenz verleihen;
- 3 *inh*A-Sonden zum Nachweis von einem oder mehreren von 3 SNPs, die MTB Isoniazid-Resistenz verleihen; und
- 4 *kat*G-Sonden zum Nachweis von einem oder mehreren von 4 SNPs, die MTB Isoniazid-Resistenz verleihen; und
- Nachweisen der Gegenwart oder Abwesenheit des einen oder der mehreren Amplifikationsprodukte, wobei die Gegenwart des einen oder der mehreren Amplifikationsprodukte die Gegenwart von MTB-RIF und/oder MTB-INH in der Probe angibt, und wobei die Abwesenheit des einen oder der mehreren Amplifikationsprodukte die Abwesenheit von MTB-RIF und/oder MTB-INH in der Probe angibt;
wobei die Vielzahl von *rpo*B-Sonden Hydrolysesonden zum Nachweis von jedem der 17 SNPs, die MTB Rifampicin-Resistenz verleihen, umfasst, die *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P und *rpo*B 526R umfassen;
wobei die Vielzahl von *inh*A-Sonden Hydrolysesonden zum Nachweis von jedem der 3 SNPs, die MTB Isoniazid-Resistenz verleihen, umfasst, die *inh*A-15T, *inh*A-8A und *inh*A-8C umfassen;
wobei die Vielzahl von *kat*G-Sonden Hydrolysesonden zum Nachweis von jedem der 4 SNPs, die MTB Isoniazid-Resistenz verleihen, umfasst, die *kat*G 315I, *kat*G 315N, *kat*G 315T und *kat*G 315T2 umfassen; und
wobei:
- die *rpo*B-Sonden eine Nukleinsäuresequenz, die ausgewählt ist aus der Gruppe, bestehend aus SEQ ID NO:1-289 und 437-438, oder ein Komplement davon umfassen;
- die *inh*A-Sonden eine Nukleinsäuresequenz, die ausgewählt ist aus der Gruppe, bestehend aus SEQ ID NO:344-409 und 436, oder ein Komplement davon umfassen; und
- die *kat*G-Sonden eine Nukleinsäuresequenz, die ausgewählt ist aus der Gruppe, bestehend aus SEQ ID NO:290-343 und 434-435, oder ein Komplement davon umfassen.

2. Verfahren nach Anspruch 1, wobei jede der Vielzahl von nachweisbaren *rpo*B-, *inh*A- und *kat*G-Sonden mit einer Donor-Fluoreszenzeinheit und einer entsprechenden Akzeptor-Fluoreszenzeinheit markiert ist; und
wobei der Nachweisschritt das Nachweisen der Gegenwart oder Abwesenheit von Fluoreszenz-Resonanzenergietransfer (FRET) zwischen der Donor-Fluoreszenzeinheit und der Akzeptor-Fluoreszenzeinheit von einer oder mehreren der *rpo*B-, *inh*A- und *kat*G-Sonden umfasst,
wobei die Gegenwart oder Abwesenheit von Fluoreszenzemission die Gegenwart oder Abwesenheit von MTB-RIF und/oder MTB-INH in der Probe angibt.

3. Verfahren nach einem der Ansprüche 1 bis 2, wobei die Amplifikation ein Polymeraseenzym mit 5'- bis 3'-Nukleaseaktivität nutzt.

4. Verfahren nach einem der Ansprüche 2 und 3, wobei sich die Donor- und Akzeptor-Fluoreszenzeinheiten innerhalb von nicht mehr als 8 Nukleotiden voneinander auf der Sonde befinden.

5. Verfahren nach einem der Ansprüche 2 bis 4, wobei die Akzeptor-Fluoreszenzeinheit ein Quencher ist.

6. Kit zum Nachweisen einer Nukleinsäure von *Mycobacterium tuberculosis* (MTB), das gegen Rifampicin (MTB-RIF) resistent ist, und/oder MTB, das gegen Isoniazid (MTB-INH) resistent ist, umfassend:
- eine Vielzahl von Sätzen von *rpo*B-, *inh*A- und *kat*G-Primern, die für die Amplifikation von *rpo*B-, *inh*A- und *kat*G-Genzielen spezifisch sind; und
- eine Vielzahl von nachweisbaren *rpo*B-, *inh*A- und *kat*G-Sonden, die für den Nachweis von *rpo*B-, *inh*A- und *kat*G-Amplifikationsprodukten spezifisch sind, umfassend:
- 17 *rpo*B-Sonden zum Nachweis von einem oder mehreren von 17 SNPs, die MTB Rifampicin-Resistenz verleihen;
- 3 *inh*A-Sonden zum Nachweis von einem oder mehreren von 3 SNPs, die MTB Isoniazid-Resistenz verleihen; und
- 4 *kat*G-Sonden zum Nachweis von einem oder mehreren von 4 SNPs, die MTB Isoniazid-Resistenz verleihen; und
wobei die Vielzahl von *rpo*B-Sonden Hydrolysesonden zum Nachweis von jedem der 17 SNPs, die MTB Rifampicin-Resistenz verleihen, umfasst, die *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P und *rpo*B 526R umfassen;
wobei die Vielzahl von *inh*A-Sonden Hydrolysesonden zum Nachweis von jedem der 3 SNPs, die MTB Isoniazid-Resistenz verleihen, umfasst, die *inh*A-15T, *inh*A-8A und *inh*A-8C umfassen;
wobei die Vielzahl von *kat*G-Sonden Hydrolysesonden zum Nachweis von jedem der 4 SNPs, die MTB Isoniazid-Resistenz verleihen, umfasst, die *katG* 315I, *katG* 315N, *katG* 315T und *katG* 315T2 umfassen; und
wobei:
- die *rpo*B-Sonden eine Nukleinsäuresequenz, die ausgewählt ist aus der Gruppe, bestehend aus SEQ ID NO:1-289 und 437-438, oder ein Komplement davon umfassen;
- die *inh*A-Sonden eine Nukleinsäuresequenz, die ausgewählt ist aus der Gruppe, bestehend aus SEQ ID NO:344-409 und 436, oder ein Komplement davon umfassen; und
- die katG-Sonden eine Nukleinsäuresequenz, die ausgewählt ist aus der Gruppe, bestehend aus SEQ ID NO:290-343 und 434-435, oder ein Komplement davon umfassen.

7. Kit nach Anspruch 6, wobei jede Sonde von der Vielzahl von nachweisbaren *rpo*B-, *inh*A- und *kat*G-Sonden eine Donor-Fluoreszenzeinheit und eine entsprechende Akzeptor-Fluoreszenzeinheit umfasst.

8. Kit nach Anspruch 7, wobei die Akzeptor-Fluoreszenzeinheit ein Quencher ist.

9. Kit nach einem der Ansprüche 6 bis 8, ferner umfassend Nukleosidtriphosphate, Nukleinsäurepolymerase und Puffer, die für die Funktion der Nukleinsäurepolymerase notwendig sind.

10. Oligonukleotidsonde, die eine Nukleinsäuresequenz, die ausgewählt ist aus der Gruppe, bestehend aus SEQ ID NO:1-409 und 434-438, oder ein Komplement davon umfasst und ferner eine oder mehrere nachweisbare Markierungen umfasst.

11. Oligonukleotid nach Anspruch 10, wobei das Oligonukleotid mindestens ein modifiziertes Nukleotid umfasst.

12. Oligonukleotid nach einem der Ansprüche 10 und 11, wobei das Oligonukleotid mindestens eine konservierend modifizierte Variation umfasst.

13. Oligonukleotid nach einem der Ansprüche 10 bis 12, wobei das Oligonukleotid 40 oder weniger Nukleotide aufweist.

14. Oligonukleotid nach einem der Ansprüche 10 bis 13, wobei das Oligonukleotid mindestens eine Markierungseinheit und/oder mindestens eine Quenchereinheit umfasst.

## Revendications

1. Procédé de détection de *Mycobacterium tuberculosis* (MTB) résistant à la rifampicine (MTB-RIF) et/ou de MTB résistant à l'isoniazide (MTB-INH) dans un échantillon, le procédé comprenant un dosage par réaction en chaîne par polymérase multiplex en temps réel comprenant :
- la mise en œuvre d'une étape d'amplification comprenant la mise en contact de l'échantillon avec au moins un ensemble d'amorces *rpo*B, un ensemble d'amorces *inh*A et un ensemble d'amorces *kat*G pour produire un ou plusieurs produits d'amplification si un quelconque acide nucléique cible *rpo*B, *inh*A, et *kat*G est présent dans l'échantillon ;
- la mise en œuvre d'une étape d'hybridation comprenant la mise en contact dudit ou desdits produits d'amplification avec une pluralité de sondes *rpo*B détectables, une pluralité de sondes *inh*A détectables, et une pluralité de sondes *kat*G détectables, comprenant au moins :
- 17 sondes *rpo*B pour la détection d'un ou plusieurs de 17 polymorphismes mononucléotidiques SNP qui confèrent à MTB une résistance à la rifampicine ;
- 3 sondes *inh*A pour la détection d'un ou plusieurs de 3 SNP qui confèrent à MTB une résistance à l'isoniazide ; et
- 4 sondes *kat*G pour la détection d'un ou plusieurs de 4 SNP qui confèrent à MTB une résistance à l'isoniazide ; et
- la détection de la présence ou de l'absence dudit ou desdits produits d'amplification, dans laquelle la présence dudit ou desdits produits d'amplification indique la présence de MTB-RIF et/ou de MTB-INH dans l'échantillon et dans laquelle l'absence dudit ou desdits produits d'amplification indique l'absence de MTB-RIF et/ou de MTB-INH dans l'échantillon ;
dans lequel ladite pluralité de sondes *rpo*B comprend des sondes d'hydrolyse pour la détection de chacun des 17 SNP qui confèrent à MTB une résistance à la rifampicine, comprenant *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P et *rpo*B 526R ;
dans lequel ladite pluralité de sondes *inh*A comprend des sondes d'hydrolyse pour la détection de chacun des 3 SNP qui confèrent à MTB une résistance à l'isoniazide, comprenant *inh*A-15T, *inh*A-8A et *inh*A-8C *;*
dans lequel ladite pluralité de sondes *kat*G comprend des sondes d'hydrolyse pour la détection de chacun des 4 SNP qui confèrent à MTB une résistance à l'isoniazide, comprenant *kat*G 315I, *kat*G 315N, *kat*G 315T et *kat*G 315T2 ; et
dans lequel :
- les sondes *rpo*B comprennent une séquence d'acide nucléique choisie dans le groupe constitué par les SEQ ID NO : 1 à 289 et 437 à 438, ou un complément de celle-ci ;
- les sondes *inh*A comprennent une séquence d'acide nucléique choisie dans le groupe constitué par les SEQ ID NO : 344 à 409 et 436, ou un complément de celle-ci ; et
- les sondes *katG* comprennent une séquence d'acide nucléique choisie dans le groupe constitué par les SEQ ID NO : 290 à 343 et 434 à 435, ou un complément de celle-ci.

2. Procédé selon la revendication 1, dans lequel chacune de la pluralité de sondes *rpo*B, *inh*A et *kat*G détectables est marquée avec une fraction fluorescente donneuse et une fraction fluorescente acceptrice correspondante ; et
dans lequel l'étape de détection comprend la détection de la présence ou de l'absence d'un transfert d'énergie de fluorescence par résonance (FRET) entre la fraction fluorescente donneuse et la fraction fluorescente acceptrice d'une ou plusieurs des sondes *rpo*B, *inh*A et *kat*G, dans lequel la présence ou l'absence d'émission de fluorescence indique la présence ou l'absence de MTB-RIF et/ou de MTB-INH dans l'échantillon.

3. Procédé selon l'une quelconque des revendications 1 à 2, dans lequel ladite amplification utilise une enzyme polymérase ayant une activité nucléase 5' vers 3'.

4. Procédé selon l'une quelconque des revendications 2 et 3, dans lequel lesdites fractions fluorescentes donneuse et acceptrice sont à pas plus de 8 nucléotides l'une de l'autre sur ladite sonde.

5. Procédé selon l'une quelconque des revendications 2 à 4, dans lequel ladite fraction fluorescente acceptrice est un extincteur.

6. Kit de détection d'un acide nucléique de *Mycobacterium tuberculosis* (MTB) résistant à la rifampicine (MTB-RIF) et/ou de MTB résistant à l'isoniazide (MTB-INH) comprenant :
- une pluralité d'ensembles d'amorces *rpo*B, *inh*A et *kat*G spécifiques pour l'amplification de cibles des gènes *rpo*B, *inh*A et katG ; et
- une pluralité de sondes *rpo*B, *inh*A et *kat*G détectables spécifiques pour la détection de produits d'amplification de *rpo*B, *inh*A et *kat*G, comprenant :
- 17 sondes *rpo*B pour la détection d'un ou plusieurs de 17 SNP qui confèrent à MTB une résistance à la rifampicine ;
- 3 sondes *inh*A pour la détection d'un ou plusieurs de 3 SNP qui confèrent à MTB une résistance à l'isoniazide ; et
- 4 sondes *katG* pour la détection d'un ou plusieurs de 4 SNP qui confèrent à MTB une résistance à l'isoniazide ;
dans lequel ladite pluralité de sondes *rpo*B comprend des sondes d'hydrolyse pour la détection de chacun des 17 SNP qui confèrent à MTB une résistance à la rifampicine, comprenant *rpo*B 531L, *rpo*B 531W, *rpo*B 526L, *rpo*B 526Y, *rpo*B 526D, *rpo*B 526N, *rpo*B 513L, *rpo*B 513K, *rpo*B 513P, *rpo*B 522L, *rpo*B 522Q, *rpo*B 522W, *rpo*B 516V, *rpo*B 516Y, *rpo*B 533P, *rpo*B 511P et *rpo*B 526R ;
dans lequel ladite pluralité de sondes *inh*A comprend des sondes d'hydrolyse pour la détection de chacun des 3 SNP qui confèrent à MTB une résistance à l'isoniazide, comprenant *inh*A-15T, *inh*A-8A et *inh*A-8C *;*
dans lequel ladite pluralité de sondes *kat*G comprend des sondes d'hydrolyse pour la détection de chacun des 4 SNP qui confèrent à MTB une résistance à l'isoniazide, comprenant *kat*G 315I, *kat*G 315N, *kat*G 315T et *kat*G 315T2 ; et
dans lequel :
- les sondes *rpo*B comprennent une séquence d'acide nucléique choisie dans le groupe constitué par les SEQ ID NO : 1 à 289 et 437 à 438, ou un complément de celle-ci ;
- les sondes *inh*A comprennent une séquence d'acide nucléique choisie dans le groupe constitué par les SEQ ID NO : 344 à 409 et 436, ou un complément de celle-ci ; et
- les sondes *kat*G comprennent une séquence d'acide nucléique choisie dans le groupe constitué par les SEQ ID NO : 290 à 343 et 434 à 435, ou un complément de celle-ci.

7. Kit selon la revendication 6, dans lequel chaque sonde de ladite pluralité de sondes *rpo*B, *inh*A et *kat*G détectables comprend une fraction fluorescente donneuse et une fraction fluorescente acceptrice correspondante.

8. Kit selon la revendication 7, dans lequel la fraction fluorescente acceptrice est un extincteur.

9. Kit selon l'une quelconque des revendications 6 à 8, comprenant en outre des nucléosides triphosphates, une acide nucléique polymérase et des tampons nécessaires pour la fonction de l'acide nucléique polymérase.

10. Sonde oligonucléotidique comprenant une séquence d'acide nucléique choisie dans le groupe constitué par les SEQ ID NO : 1 à 409 et 434 à 438, ou un complément de celle-ci et comprenant en outre un ou plusieurs marqueurs détectables.

11. Oligonucléotide selon la revendication 10, dans lequel l'oligonucléotide comprend au moins un nucléotide modifié.

12. Oligonucléotide selon l'une quelconque des revendications 10 et 11, dans lequel l'oligonucléotide comprend au moins une variation modifiée de manière conservatrice.

13. Oligonucléotide selon l'une quelconque des revendications 10 à 12, dans lequel l'oligonucléotide a 40 nucléotides ou moins.

14. Oligonucléotide selon l'une quelconque des revendications 10 à 13, dans lequel l'oligonucléotide comprend au moins une fraction de marquage et/ou au moins une fraction extinctrice.


### SEQUENCE LISTING

<110> Roche Diagnostics GmbH
   F. Hoffmann-La Roche AG
   Roche Molecular Systems, Inc.
<120> COMPOSITIONS AND METHODS FOR DETECTION OF DRUG RESISTANT MYCOBACTERIUM TUBERCULOSIS
<130> P32319-WO-HS
<150> US 14/575879
   <151> 2014-12-18
<160> 438
<170> PatentIn version 3.5
<210> 1
   <211> 14
   <212> DNA
   <213> Artificial Sequence
<220>
   <223> Description of Artificial Sequence: Synthetic oligonucleotide
<220>
   <223> RMRPO3SP531L09
<400> 1
   gttggcgctg gggc 14
<210> 2
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP531L18
<400> 2
   actgttggcg ctggg 15
<210> 3
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP531L19
<400> 3
   ctgttggcgc uggg 14
<210> 4
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L18B
<400> 4
   actgttggcg cuggg 15
<210> 5
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L18C
<400> 5
   ctgtuggcgc uggg 14
<210> 6
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRP03531L1B
<400> 6
   ctgttggcgc tggggc 16
<210> 7
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP531L20
<400> 7
   acugttggcg cugg 14
<210> 8
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L20
<400> 8
   ccgactgttg gcgcu 15
<210> 9
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP531L22
<400> 9
   ctgttggcgc uggg 14
<210> 10
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP531L24
<400> 10
   cuguuggcgc tggggc 16
<210> 11
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP531L25
<400> 11
   cuguuggcgc tgggg 15
<210> 12
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP531L26
<400> 12
   uguuggcgct ggggccc 17
<210> 13
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L20B
<400> 13
   acuguuggcg cugg 14
<210> 14
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L20C
<400> 14
   acuguuggcg cugg 14
<210> 15
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L20D
<400> 15
   acugutggcg cugg 14
<210> 16
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L25B
<400> 16
   cuguuggcgc uggg 14
<210> 17
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L25C
<400> 17
   cuguuggcgc uggc 14
<210> 18
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531L20F
<400> 18
   acuguuggcg cugcagc 17
<210> 19
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L20HS
<400> 19
   acuguucggc gcugg 15
<210> 20
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L25C2
<400> 20
   cuguuggcgc uggc 14
<210> 21
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L20C2
<400> 21
   acuguuggcg cugc 14
<210> 22
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L25C3
<400> 22
   acuguuggcg cuggc 15
<210> 23
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S531F1
<400> 23
   acuguucgcg cugg 14
<210> 24
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L25B2
<400> 24
   cuguuggcgc tggg 14
<210> 25
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L31
<400> 25
   uguuggcgct gggg 14
<210> 26
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L25B3
<400> 26
   cuguuggcgc tggg 14
<210> 27
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L25B4
<400> 27
   cuguuggcgc tggc 14
<210> 28
   <211> 17
   <212> DNA
   <213> Artificial Sequence
<220>
   <223> Description of Artificial Sequence: Synthetic oligonucleotide
<220>
   <223> RMRPO3F531L
<400> 28
   ccaacagtcg gcgcttg 17
<210> 29
   <211> 23
   <212> DNA
   <213> Artificial Sequence
<220>
   <223> Description of Artificial Sequence: Synthetic oligonucleotide
<220>
   <223> RMRPO3F531L 02
<400> 29
   ccaacagtcg gcgcttgtgg gtc 23
<210> 30
   <211> 17
   <212> DNA
   <213> Artificial Sequence
<220>
   <223> Description of Artificial Sequence: Synthetic oligonucleotide
<220>
   <223> RMRPO3F531L 04
<400> 30
   ccaacagtcg gcgcttg 17
<210> 31
   <211> 17
   <212> DNA
   <213> Artificial Sequence
<220>
   <223> Description of Artificial Sequence: Synthetic oligonucleotide
<220>
   <223> RMRPO3F531L05
<400> 31
   ccaacagtcg gcgcttg 17
<210> 32
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3F531L06
<400> 32
   ccaacagucg gcgctug 17
<210> 33
   <211> 17
   <212> DNA
   <213> Artificial Sequence
<220>
   <223> Description of Artificial Sequence: Synthetic oligonucleotide
<220>
   <223> RMRPO3AP531L11
<400> 33
   ccaacagtcg gcgcttg 17
<210> 34
   <211> 17
   <212> DNA
   <213> Artificial Sequence
<220>
   <223> Description of Artificial Sequence: Synthetic oligonucleotide
<220>
   <223> RMRPO3AP531L12
<400> 34
   ccaacagtcg gcgcttg 17
<210> 35
   <211> 17
   <212> DNA
   <213> Artificial Sequence
<220>
   <223> Description of Artificial Sequence: Synthetic oligonucleotide
<220>
   <223> RMRPO3AP531L13
<400> 35
   ccaacagtcg gcgcttg 17
<210> 36
   <211> 17
   <212> DNA
   <213> Artificial Sequence
<220>
   <223> Description of Artificial Sequence: Synthetic oligonucleotide
<220>
   <223> RMRPO3AP531L14
<400> 36
   ccaacagtcg gcgcttg 17
<210> 37
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A531L12B
<400> 37
   ccaacagucg gcgcttg 17
<210> 38
   <211> 14
   <212> DNA
   <213> Artificial Sequence
<220>
   <223> Description of Artificial Sequence: Synthetic oligonucleotide
<220>
   <223> RMRPO3AP531L17
<400> 38
   ccaacagtcg gcgc 14
<210> 39
   <211> 14
   <212> DNA
   <213> Artificial Sequence
<220>
   <223> Description of Artificial Sequence: Synthetic oligonucleotide
<220>
   <223> RMRPO3A531L17B
<400> 39
   ccaacagtcg gcgc 14
<210> 40
   <211> 15
   <212> DNA
   <213> Artificial Sequence
<220>
   <223> Description of Artificial Sequence: Synthetic oligonucleotide
<220>
   <223> RMRPO3A531L19
<400> 40
   ccaacagtcg gcgct 15
<210> 41
   <211> 15
   <212> DNA
   <213> Artificial Sequence
<220>
   <223> Description of Artificial Sequence: Synthetic oligonucleotide
<220>
   <223> RMRPO3A531L20
<400> 41
   ccaacagtcg gcgct 15
<210> 42
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A531L18D
<400> 42
   ccaacagucg gcgct 15
<210> 43
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A531L12C
<400> 43
   ccaacagucg gcgcttg 17
<210> 44
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A531L12D
<400> 44
   ccaacagucg gcgcttg 17
<210> 45
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L17B2
<400> 45
   ccaacagucg gcgc 14
<210> 46
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L17B3
<400> 46
   ccaacagucg gcgc 14
<210> 47
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A531L21
<400> 47
   ccaacagucg gcgcu 15
<210> 48
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A531L22
<400> 48
   cccacagucg gcgc 14
<210> 49
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L17B4
<400> 49
   ccaacagucg gcgc 14
<210> 50
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3531L17B5
<400> 50
   ccaacagucg gcgc 14
<210> 51
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A531L20B
<400> 51
   ccaacagucg gcgc 14
<210> 52
   <211> 14
   <212> DNA
   <213> Artificial Sequence
<220>
   <223> Description of Artificial Sequence: Synthetic oligonucleotide
<220>
   <223> RM5L17B3a
<400> 52
   ccaacagtcg gcgc 14
<210> 53
   <211> 17
   <212> DNA
   <213> Artificial Sequence
<220>
   <223> Description of Artificial Sequence: Synthetic oligonucleotide
<220>
   <223> RMRPO3F531W
<400> 53
   cccacagtcg gcgcttg 17
<210> 54
   <211> 18
   <212> DNA
   <213> Artificial Sequence
<220>
   <223> Description of Artificial Sequence: Synthetic oligonucleotide
<220>
   <223> RMRPO3F531W02
<400> 54
   cccacagtcg gcgcttgt 18
<210> 55
   <211> 14
   <212> DNA
   <213> Artificial Sequence
<220>
   <223> Description of Artificial Sequence: Synthetic oligonucleotide
<220>
   <223> RMRPO3F531W06
<400> 55
   cccacagtcg gcgc 14
<210> 56
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A07
<400> 56
   cuugaggguc aacccc 16
<210> 57
   <211> 22
   <212> DNA
   <213> Artificial Sequence
<220>
   <223> Description of Artificial Sequence: Synthetic oligonucleotide
<220>
   <223> RMRPO3A07B2
<400> 57
   ttgagggtca accccgacgg gg 22
<210> 58
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A08
<400> 58
   acccucaagc gccg 14
<210> 59
   <211> 13
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A08B
<400> 59
   acccucaagc gcc 13
<210> 60
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A08C
<400> 60
   acccucaagc gccg 14
<210> 61
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A09
<400> 61
   cuugaggguc aacccc 16
<210> 62
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LFM07
<400> 62
   cuugaggguc aaccccga 18
<210> 63
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LFM08
<400> 63
   gcuugagggu caaccccga 19
<210> 64
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LFM09
<400> 64
   gcuugagggu caaccccga 19
<210> 65
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LFM10
<400> 65
   gcuugagggu caaccccga 19
<210> 66
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LFM11
<400> 66
   gcuugagggc caaccccga 19
<210> 67
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LFM12
<400> 67
   gcuugagggu caaccccga 19
<210> 68
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LJA01
<400> 68
   gcuugagggu caaccccga 19
<210> 69
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LJA02
<400> 69
   gcuugagggu caaccccga 19
<210> 70
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LJA03
<400> 70
   gcuugagggc caaccccga 19
<210> 71
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LJA04
<400> 71
   gcuugagggu caaccccga 19
<210> 72
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LJA05
<400> 72
   gcuugagggc caaccccga 19
<210> 73
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526LJA06
<400> 73
   gcuugagggc caaccccga 19
<210> 74
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A06E
<400> 74
   uuguagguca accccga 17
<210> 75
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YFM07
<400> 75
   cuuguagguc aaccccga 18
<210> 76
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YFM08
<400> 76
   cuuguagguc aaccccga 18
<210> 77
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YFM09
<400> 77
   cuuguagguc aaccccga 18
<210> 78
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YFM10
<400> 78
   cuuguagguc aaccccga 18
<210> 79
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX01
<400> 79
   cuuguagguc aaccccga 18
<210> 80
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX02
<400> 80
   cuuguagguc aaccccga 18
<210> 81
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX03
<400> 81
   uuguagguca accccga 17
<210> 82
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX04
<400> 82
   uuguagguca acccc 15
<210> 83
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX05
<400> 83
   uuguagguca acccc 15
<210> 84
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX06
<400> 84
   cuuguaggcc aaccccga 18
<210> 85
   <211> 20
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX07
<400> 85
   cuuguagguc aaccccgaca 20
<210> 86
   <211> 20
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX08
<400> 86
   cgcuuguagg ucaaccccga 20
<210> 87
   <211> 21
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX09
<400> 87
   gcuuguaggu caaccccgac a 21
<210> 88
   <211> 22
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526YHX10
<400> 88
   cgcuuguagg ucaaccccga ca 22
<210> 89
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A03C
<400> 89
   cuugucgguc aacccc 16
<210> 90
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A03D
<400> 90
   cuugucgguc aacccc 16
<210> 91
   <211> 16
   <212> DNA
   <213> Artificial Sequence
<220>
   <223> Description of Artificial Sequence: Synthetic oligonucleotide
<220>
   <223> AYRPO3526DFM03
<400> 91
   tgtcggtcaa ccccga 16
<210> 92
   <211> 16
   <212> DNA
   <213> Artificial Sequence
<220>
   <223> Description of Artificial Sequence: Synthetic oligonucleotide
<220>
   <223> AYRPO3526DFM04
<400> 92
   tgtcggtcaa ccccga 16
<210> 93
   <211> 18
   <212> DNA
   <213> Artificial Sequence
<220>
   <223> Description of Artificial Sequence: Synthetic oligonucleotide
<220>
   <223> AYRPO3526DFM05
<400> 93
   gttgtcggtc aaccccga 18
<210> 94
   <211> 18
   <212> DNA
   <213> Artificial Sequence
<220>
   <223> Description of Artificial Sequence: Synthetic oligonucleotide
<220>
   <223> AYRPO3526DFM06
<400> 94
   gttgtcggtc aaccccga 18
<210> 95
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526DJA07
<400> 95
   uugucgguca acccc 15
<210> 96
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526DJA08
<400> 96
   cuugucgguc aacccc 16
<210> 97
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526DJA09
<400> 97
   uugucgguca accccga 17
<210> 98
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526DJA10
<400> 98
   cuugucgguc aaccccga 18
<210> 99
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> AYRPO3526DJA11
<400> 99
   gcuugucggu caaccccga 19
<210> 100
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526R2
<400> 100
   accaacaagc gccg 14
<210> 101
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526R3
<400> 101
   accaacaagc gccg 14
<210> 102
   <211> 17
   <212> DNA
   <213> Artificial Sequence
<220>
   <223> Description of Artificial Sequence: Synthetic oligonucleotide
<220>
   <223> RMRPO3AP526R1
<400> 102
   ttgttggtca accccga 17
<210> 103
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3AP526N1
<400> 103
   uuguugguca accc 14
<210> 104
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3AP526N2
<400> 104
   uuguugguca acccg 15
<210> 105
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3AP526N2B
<400> 105
   uuguugguca accc 14
<210> 106
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526N4
<400> 106
   accaacaagc gccgacu 17
<210> 107
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526N4B
<400> 107
   accaacaagc gccgacu 17
<210> 108
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526N4B2
<400> 108
   accaacaagc gccgac 16
<210> 109
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526N4B2b
<400> 109
   accaacaagc gccga 15
<210> 110
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526N4B3
<400> 110
   caacaagcgc cgacug 16
<210> 111
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526N4C
<400> 111
   accaacaagc gccg 14
<210> 112
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3SP526N5
<400> 112
   accaacaagc gccgacu 17
<210> 113
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3AP526N6
<400> 113
   uuguugguca accccga 17
<210> 114
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3AP526N7
<400> 114
   uuguugguca accccga 17
<210> 115
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3AP526N8
<400> 115
   uuguugguca acccc 15
<210> 116
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3AP526N9
<400> 116
   uuguugguca acccc 15
<210> 117
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3AP526N1B
<400> 117
   uuguugguca accc 14
<210> 118
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3A526N8B
<400> 118
   uuguugguca acccc 15
<210> 119
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3H533P10B
<400> 119
   cgccggggcc cggc 14
<210> 120
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3H533P10D
<400> 120
   cgccggggcc cggcgg 16
<210> 121
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3H533P10
<400> 121
   cgccggggcc cggcgg 16
<210> 122
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513L44PJ12
<400> 122
   cagcugagcc uauuc 15
<210> 123
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513L68PJ12
<400> 123
   ccuauucaug gaccag 16
<210> 124
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513L59PJ12
<400> 124
   ccagcugagc cuauuc 16
<210> 125
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513L101PJ12
<400> 125
   agccuauuca uggaccag 18
<210> 126
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513L84PJ12
<400> 126
   gccuauucat ggaccag 17
<210> 127
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L60PJ12
<400> 127
   ugaauaggcu cagcug 16
<210> 128
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L68PJ12
<400> 128
   cugguccaug aatagg 16
<210> 129
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L103PJ12
<400> 129
   uucuggucca ugaauagg 18
<210> 130
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L61PJ12
<400> 130
   augaauaggc ucagcu 16
<210> 131
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L76PJ12
<400> 131
   ugaauaggcu cagcugg 17
<210> 132
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L59PJ12
<400> 132
   gaauaggcuc agcugg 16
<210> 133
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L95PJ12
<400> 133
   caugaauagg cucagcug 18
<210> 134
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L924PJ2
<400> 134
   gaauaggcuc aguuggcu 18
<210> 135
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L928PJ2
<400> 135
   gaauagguuc agcuggcu 18
<210> 136
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L927PJ2
<400> 136
   gaauaggcuu agcuggcu 18
<210> 137
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L9232PJ2
<400> 137
   gaauaggcug agcuggcu 18
<210> 138
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L9243PJ2
<400> 138
   gaauaggcuc cgcuggcu 18
<210> 139
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L9217PJ2
<400> 139
   gaauaggcuc agcaggcu 18
<210> 140
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L9218PJ2
<400> 140
   gaauaggcuc agauggcu 18
<210> 141
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L9222PJ2
<400> 141
   gaauaggauc agcuggcu 18
<210> 142
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L75PJ12
<400> 142
   gaauaggcuc agcuggc 17
<210> 143
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L75PCA12
<400> 143
   gaauaggcuc agcuggc 17
<210> 144
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L75PCB12
<400> 144
   tgaauaggcu cagcuggc 18
<210> 145
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L77PJ12
<400> 145
   augaauaggc ucagcug 17
<210> 146
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513L93PJ12
<400> 146
   ugaauaggcu cagcuggc 18
<210> 147
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513L85PJ12
<400> 147
   ccuauucaug gaccaga 17
<210> 148
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513K84PJ12
<400> 148
   agcaaauuca uggacca 17
<210> 149
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513K77PJ12
<400> 149
   ccagcugagc aaauuca 17
<210> 150
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513K79PJ12
<400> 150
   agcugagcaa auucaug 17
<210> 151
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513K85PJ12
<400> 151
   gcaaauucau ggaccag 17
<210> 152
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K43PJ12
<400> 152
   auuugcucag cuggc 15
<210> 153
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K77PJ12
<400> 153
   ugaauuugcu cagcugg 17
<210> 154
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K60PJ12
<400> 154
   gaauuugcuc agcugg 16
<210> 155
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K76PJ12
<400> 155
   gaauuugcuc agcuggc 17
<210> 156
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K76PCA12
<400> 156
   gaauuugcuc agcuggc 17
<210> 157
   <211> 18
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K76PCB12
<400> 157
   tgaauuugcu cagcuggc 18
<210> 158
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K44PJ12
<400> 158
   aauuugcuca gcugg 15
<210> 159
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K80PJ12
<400> 159
   ccaugaauuu gcucagc 17
<210> 160
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K59PJ12
<400> 160
   aauuugcuca gcuggc 16
<210> 161
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513K75PJ12
<400> 161
   aauuugcuca gcuggcu 17
<210> 162
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P66PJ12
<400> 162
   agcccauuca uggacc 16
<210> 163
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P83PJ12
<400> 163
   agcccauuca uggacca 17
<210> 164
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P68PJ12
<400> 164
   cccauucaug gaccag 16
<210> 165
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JS513P81PJ12
<400> 165
   ugagcccauu cauggac 17
<210> 166
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513P60PJ12
<400> 166
   ugaaugggcu cagcug 16
<210> 167
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA513P76PJ12
<400> 167
   ugaaugggct cagcugg 17
<210> 168
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P66GJ12
<400> 168
   agcccauuca uggacc 16
<210> 169
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P83GJ12
<400> 169
   agcccauuca uggacca 17
<210> 170
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P84PJ12
<400> 170
   gcccauucau ggaccag 17
<210> 171
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P82PJ12
<400> 171
   gagcccauuc auggacc 17
<210> 172
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P84GJ12
<400> 172
   gcccauucau ggaccag 17
<210> 173
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS513P82GJ12
<400> 173
   gagcccauuc auggacc 17
<210> 174
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522L50PJ12
<400> 174
   ggucaacccc aacag 15
<210> 175
   <211> 13
   <212> DNA
   <213> Artificial Sequence
<220>
   <223> Description of Artificial Sequence: Synthetic oligonucleotide
<220>
   <223> JJA522L18PJ12
<400> 175
   accccaacag cgg 13
<210> 176
   <211> 13
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522L16PJ12
<400> 176
   cccaacagcg ggu 13
<210> 177
   <211> 13
   <212> DNA
   <213> Artificial Sequence
<220>
   <223> Description of Artificial Sequence: Synthetic oligonucleotide
<220>
   <223> JJA522L17PJ12
<400> 177
   ccccaacagc ggg 13
<210> 178
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522L83PJ12
<400> 178
   ugggucaacc ccaacag 17
<210> 179
   <211> 14
   <212> DNA
   <213> Artificial Sequence
<220>
   <223> Description of Artificial Sequence: Synthetic oligonucleotide
<220>
   <223> JJA522L32PJ12
<400> 179
   aaccccaaca gcgg 14
<210> 180
   <211> 14
   <212> DNA
   <213> Artificial Sequence
<220>
   <223> Description of Artificial Sequence: Synthetic oligonucleotide
<220>
   <223> JJA522L33PJ12
<400> 180
   caaccccaac agcg 14
<210> 181
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522L48PJ12
<400> 181
   ucaaccccaa cagcg 15
<210> 182
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522L30PJ12
<400> 182
   ccccaacagc gggu 14
<210> 183
   <211> 14
   <212> DNA
   <213> Artificial Sequence
<220>
   <223> Description of Artificial Sequence: Synthetic oligonucleotide
<220>
   <223> JJA522L31PJ12
<400> 183
   accccaacag cggg 14
<210> 184
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522L63PJ12
<400> 184
   ucaaccccaa cagcgg 16
<210> 185
   <211> 15
   <212> DNA
   <213> Artificial Sequence
<220>
   <223> Description of Artificial Sequence: Synthetic oligonucleotide
<220>
   <223> JJA522L47PJ12
<400> 185
   caaccccaac agcgg 15
<210> 186
   <211> 16
   <212> DNA
   <213> Artificial Sequence
<220>
   <223> Description of Artificial Sequence: Synthetic oligonucleotide
<220>
   <223> JJS522Q48J12
<400> 186
   ccgctgcagg ggttga 16
<210> 187
   <211> 15
   <212> DNA
   <213> Artificial Sequence
<220>
   <223> Description of Artificial Sequence: Synthetic oligonucleotide
<220>
   <223> JJS522Q32J12
<400> 187
   cccgctgcag gggtt 15
<210> 188
   <211> 16
   <212> DNA
   <213> Artificial Sequence
<220>
   <223> Description of Artificial Sequence: Synthetic oligonucleotide
<220>
   <223> JJS522Q49J12
<400> 188
   cgctgcaggg gttgac 16
<210> 189
   <211> 15
   <212> DNA
   <213> Artificial Sequence
<220>
   <223> Description of Artificial Sequence: Synthetic oligonucleotide
<220>
   <223> JJA522Q31J12
<400> 189
   acccctgcag cgggt 15
<210> 190
   <211> 15
   <212> DNA
   <213> Artificial Sequence
<220>
   <223> Description of Artificial Sequence: Synthetic oligonucleotide
<220>
   <223> JJA522Q33J12
<400> 190
   caacccctgc agcgg 15
<210> 191
   <211> 16
   <212> DNA
   <213> Artificial Sequence
<220>
   <223> Description of Artificial Sequence: Synthetic oligonucleotide
<220>
   <223> JJA522Q43J12
<400> 191
   ccctgcagcg ggttgt 16
<210> 192
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522Q31PJ12
<400> 192
   accccugcag cgggu 15
<210> 193
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS522Q49PJ12
<400> 193
   cgcugcaggg guugac 16
<210> 194
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522Q18PJ12
<400> 194
   accccugcag cggg 14
<210> 195
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJS522Q34PJ12
<400> 195
   cgcugcaggg guuga 15
<210> 196
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522Q20PJ12
<400> 196
   caaccccugc agcg 14
<210> 197
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522Q33PJ12
<400> 197
   caaccccugc agcgg 15
<210> 198
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522Q47PJ12
<400> 198
   caaccccugc agcggg 16
<210> 199
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522W33PJ12
<400> 199
   caacccccac agcg 14
<210> 200
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522W33GJ12
<400> 200
   caacccccac agcg 14
<210> 201
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522W47J12
<400> 201
   caacccccac agcgg 15
<210> 202
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522W47GJ12
<400> 202
   caacccccac agcgg 15
<210> 203
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522W48PJ12
<400> 203
   ucaaccccca cagcg 15
<210> 204
   <211> 15
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> JJA522W48GJ12
<400> 204
   ucaaccccca cagcg 15
<210> 205
   <211> 19
   <212> DNA
   <213> Artificial Sequence
<220>
   <223> Description of Artificial Sequence: Synthetic oligonucleotide
<220>
   <223> RMRPO3F516V
<400> 205
   ctggaccatg aattggctc 19
<210> 206
   <211> 19
   <212> DNA
   <213> Artificial Sequence
<220>
   <223> Description of Artificial Sequence: Synthetic oligonucleotide
<220>
   <223> RMRPO3UNF516V
<400> 206
   ctggaccatg aattggctc 19
<210> 207
   <211> 19
   <212> DNA
   <213> Artificial Sequence
<220>
   <223> Description of Artificial Sequence: Synthetic oligonucleotide
<220>
   <223> RMRPO3SP516V
<400> 207
   tggtccagaa caacccgct 19
<210> 208
   <211> 21
   <212> DNA
   <213> Artificial Sequence
<220>
   <223> Description of Artificial Sequence: Synthetic oligonucleotide
<220>
   <223> RMRPO3SP516Y
<400> 208
   atgtaccaga acaacccgct g 21
<210> 209
   <211> 19
   <212> DNA
   <213> Artificial Sequence
<220>
   <223> Description of Artificial Sequence: Synthetic oligonucleotide
<220>
   <223> RMRPO3AP516Y
<400> 209
   ctggtacatg aattggctc 19
<210> 210
   <211> 22
   <212> DNA
   <213> Artificial Sequence
<220>
   <223> Description of Artificial Sequence: Synthetic oligonucleotide
<220>
   <223> RMRPO3A2P516Y
<400> 210
   ctggtacatg aattggctca gc 22
<210> 211
   <211> 19
   <212> DNA
   <213> Artificial Sequence
<220>
   <223> Description of Artificial Sequence: Synthetic oligonucleotide
<220>
   <223> RMRPO3F516V02
<400> 211
   ctggaccatg aattggctc 19
<210> 212
   <211> 23
   <212> DNA
   <213> Artificial Sequence
<220>
   <223> Description of Artificial Sequence: Synthetic oligonucleotide
<220>
   <223> RMRPO3SP516V03
<400> 212
   tggtccagaa caacccgctg cgg 23
<210> 213
   <211> 19
   <212> DNA
   <213> Artificial Sequence
<220>
   <223> Description of Artificial Sequence: Synthetic oligonucleotide
<220>
   <223> RMRPO3SP516V04
<400> 213
   tggtccaaaa caacccgct 19
<210> 214
   <211> 19
   <212> DNA
   <213> Artificial Sequence
<220>
   <223> Description of Artificial Sequence: Synthetic oligonucleotide
<220>
   <223> RMRPO3SP516V05
<400> 214
   tggtccagaa caacccgct 19
<210> 215
   <211> 19
   <212> DNA
   <213> Artificial Sequence
<220>
   <223> Description of Artificial Sequence: Synthetic oligonucleotide
<220>
   <223> RMRPO3SP516V06
<400> 215
   tggtccagaa caacccgct 19
<210> 216
   <211> 22
   <212> DNA
   <213> Artificial Sequence
<220>
   <223> Description of Artificial Sequence: Synthetic oligonucleotide
<220>
   <223> RMRPO3SP516Y02
<400> 216
   atgtaccaga acaacccggg gt 22
<210> 217
   <211> 23
   <212> DNA
   <213> Artificial Sequence
<220>
   <223> Description of Artificial Sequence: Synthetic oligonucleotide
<220>
   <223> RMRPO3SP516V07
<400> 217
   tggtccagaa taacccgctg cgg 23
<210> 218
   <211> 23
   <212> DNA
   <213> Artificial Sequence
<220>
   <223> Description of Artificial Sequence: Synthetic oligonucleotide
<220>
   <223> RMRPO3SP516V08
<400> 218
   tggtccaaaa caacccgctg cgg 23
<210> 219
   <211> 19
   <212> DNA
   <213> Artificial Sequence
<220>
   <223> Description of Artificial Sequence: Synthetic oligonucleotide
<220>
   <223> RMRPO3SP516V09
<400> 219
   ggtccagaac aacccgctg 19
<210> 220
   <211> 20
   <212> DNA
   <213> Artificial Sequence
<220>
   <223> Description of Artificial Sequence: Synthetic oligonucleotide
<220>
   <223> RMRPO3SP516V10
<400> 220
   atggtccaga acaacccgct 20
<210> 221
   <211> 19
   <212> DNA
   <213> Artificial Sequence
<220>
   <223> Description of Artificial Sequence: Synthetic oligonucleotide
<220>
   <223> RMRPO3SP516V11
<400> 221
   catggtccag aacaacccg 19
<210> 222
   <211> 21
   <212> DNA
   <213> Artificial Sequence
<220>
   <223> Description of Artificial Sequence: Synthetic oligonucleotide
<220>
   <223> RMRPO3SP516V12
<400> 222
   atggtccaga acaaccggtt g 21
<210> 223
   <211> 19
   <212> DNA
   <213> Artificial Sequence
<220>
   <223> Description of Artificial Sequence: Synthetic oligonucleotide
<220>
   <223> RMRPO3S516V11B
<400> 223
   catggtccag aacaacccg 19
<210> 224
   <211> 19
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S516V11C
<400> 224
   caugguccag aacaacccg 19
<210> 225
   <211> 19
   <212> DNA
   <213> Artificial Sequence
<220>
   <223> Description of Artificial Sequence: Synthetic oligonucleotide
<220>
   <223> RMRPO3S516V11D
<400> 225
   catggtccag aacaacccg 19
<210> 226
   <211> 18
   <212> DNA
   <213> Artificial Sequence
<220>
   <223> Description of Artificial Sequence: Synthetic oligonucleotide
<220>
   <223> RMRPO3S516V11E
<400> 226
   atggtccaga acaacccg 18
<210> 227
   <211> 19
   <212> DNA
   <213> Artificial Sequence
<220>
   <223> Description of Artificial Sequence: Synthetic oligonucleotide
<220>
   <223> RMRPO3S516V11F
<400> 227
   catggtccag aacaacccg 19
<210> 228
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3S516V11G
<400> 228
   caugguccag aacaac 16
<210> 229
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3516V11C2
<400> 229
   caugguccag aacaac 16
<210> 230
   <211> 16
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RM516V11G2
<400> 230
   ccugguccag aacaac 16
<210> 231
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3516V11G2
<400> 231
   caugguccag aaca 14
<210> 232
   <211> 14
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3516V11G3
<400> 232
   cauggtccag aaca 14
<210> 233
   <211> 22
   <212> DNA
   <213> Artificial Sequence
<220>
   <223> Description of Artificial Sequence: Synthetic oligonucleotide
<220>
   <223> RMRPO3S516V12B
<400> 233
   catggtccag aacaaccggt tg 22
<210> 234
   <211> 17
   <212> DNA
   <213> Artificial Sequence
<220>
   <223> Description of Artificial Sequence: Synthetic oligonucleotide
<220>
   <223> Description of Combined DNA/RNA Molecule: Synthetic oligonucleotide
<220>
   <223> RMRPO3516V11C4
<400> 234
   caugguccag aacaacc 17
<210> 235
   <211> 20
   <212> DNA
   <213> Artificial Sequence
<220>
   <223> Description of Artificial Sequence: Synthetic oligonucleotide
<220>
   <223> RMRPO3516V11E2
<400> 235
   tggtccagaa caacccgctg 20
<210> 236
   <211> 20
   <212> DNA
   <213> Artificial Sequence
<220>
   <223> Description of Artificial Sequence: Synthetic oligonucleotide
<220>
   <223> RMRPO3516V11E3
<400> 236
   atggtccaga acaacagttg 20
<210> 237
   <211> 19
   <212> DNA
   <213> Artificial Sequence
<220>
   <223> Description of Artificial Sequence: Synthetic oligonucleotide
<220>
   <223> RMRPO3516V11E5
<400> 237
   tggtccagaa caacccgct 19
<210> 238
   <211> 19
   <212> DNA
   <213> Artificial Sequence
<220>
   <223> Description of Artificial Sequence: Synthetic oligonucleotide
<220>
   <223> RMRPO3516V11E6
<400> 238
   tggtccagaa caacccgct 19
<210> 239
   <211> 19
   <212> DNA
   <213> Artificial Sequence
<220>
   <223> Description of Artificial Sequence: Synthetic oligonucleotide
<220>
   <223> RMRPO3SP516Y03
<400> 239
   atgtaccaga acaacccgc 19
<210> 240
   <211> 21
   <212> DNA
   <213> Artificial Sequence
<220>
   <223> Description of Artificial Sequence: Synthetic oligonucleotide
<220>
   <223> RMRPO3S516YB2
<400> 240
   atgtaccaga acaacccgct g 21
<210> 241
   <211> 21
   <212> DNA
   <213> Artificial Sequence
<220>
   <223> Description of Artificial Sequence: Synthetic oligonucleotide
<220>
   <223> RMRPO3SP516Y
<400> 241
   atgtaccaga acaacccgct g 21
<210> 242
   <211> 19
   <212> DNA
   <213> Artificial Sequence
<220>
   <223> Description of Artificial Sequence: Synthetic oligonucleotide
<220>
   <223> RMRPO3AP516Y
<400> 242
   ctggtacatg aattggctc 19
<210> 243
   <211> 22
   <212> DNA
   <213> Artificial Sequence
<220>
   <223> Description of Artificial Sequence: Synthetic oligonucleotide
<220>
   <223> RMRPO3A2P516Y
<400> 243
   ctggtacatg aattggctca gc 22
<210> 244
   <211> 22
   <212> DNA
   <213> Artificial Sequence
<220>
   <223> Description of Artificial Sequence: Synthetic oligonucleotide
<220>
   <223> RMRPO3SP516Y02
<400> 244
   atgtaccaga acaacccggg gt 22
<210> 245
   <211> 22
   <212> DNA
   <213> Artificial Sequence
<220>
   <223> Description of Artificial Sequence: Synthetic oligonucleotide
<220>
   <223> RMRPO3SP516Y4B
<400> 245
   atgtaccaga acaacccgct gt 22
<210> 246
   <211> 21
   <212> DNA
   <213> Artificial Sequence
<220>
   <223> Description of Artificial Sequence: Synthetic oligonucleotide
<220>
   <223> RMRPO3S516YB
<400> 246
   atgtaccaga acaacccgct g 21
<210> 247
   <211> 21
   <212> DNA
   <213> Artificial Sequence
<220>
   <223> Description of Artificial Sequence: Synthetic oligonucleotide
<220>
   <223> RMRPO3S516YC
<400> 247
   atgtaccaga acaacccgct g 21
<210> 248
   <211> 14
   <212> DNA
   <213> Artificial Sequence
<220>
   <223> Description of Artificial Sequence: Synthetic oligonucleotide
<220>
   <223> RMRPO3H533P10B
<400> 248
   cgccggggcc cggc 14
<210> 249
   <211> 16
   <212> DNA
   <213> Artificial Sequence
<220>
   <223> Description of Artificial Sequence: Synthetic oligonucleotide
<220>
   <223> RMRPO3H533P10D
<400> 249
   cgccggggcc cggcgg 16
<210> 250
   <211> 19
   <212> DNA
   <213> Artificial Sequence
<220>
   <223> Description of Artificial Sequence: Synthetic oligonucleotide
<220>
   <223> RMRPO3H533
<400> 250
   ccggcgccga cagtcggcg 19
<210> 251
   <211> 17
   <212> DNA
   <213> Artificial Sequence
<220>
   <223> Description of Artificial Sequence: Synthetic oligonucleotide
<220>
   <223> RMRPO3H533P02
<400> 251
   ccggcgccga cagtcgg 17
<210> 252
   <211> 19
   <212> DNA
   <213> Artificial Sequence
<220>
   <223> Description of Artificial Sequence: Synthetic oligonucleotide
<220>
   <223> RMRPO3H533P03
<400> 252
   ccggcgccta cagtcggcg 19
<210> 253
   <211> 19
   <212> DNA
   <213> Artificial Sequence
<220>
   <223> Description of Artificial Sequence: Synthetic oligonucleotide
<220>
   <223> RMRPO3H533P04
<400> 253
   ccggcgccaa cagtcggcg 19
<210> 254
   <211> 19
   <212> DNA
   <213> Artificial Sequence
<220>
   <223> Description of Artificial Sequence: Synthetic oligonucleotide
<220>
   <223> RMRPO3H533P05
<400> 254
   ccggcgccca cagtcggcg 19
<210> 255
   <211> 17
   <212> DNA
   <213> Artificial Sequence
<220>
   <223> Description of Artificial Sequence: Synthetic oligonucleotide
<220>
   <223> RMRPO3H533P06
<400> 255
   ccggcaccga cagtcgg 17
<210> 256
   <211> 17
   <212> DNA
   <213> Artificial Sequence
<220>
   <223> Description of Artificial Sequence: Synthetic oligonucleotide
<220>
   <223> RMRPO3H533P07
<400> 256
   ccggcgtcga cagtcgg 17
<210> 257
   <211> 18
   <212> DNA
   <213> Artificial Sequence
<220>
   <223> Description of Artificial Sequence: Synthetic oligonucleotide
<220>
   <223> RMRPO3H533P08
<400> 257
   ccggcgccga cagtcggc 18
<210> 258
   <211> 18
   <212> DNA
   <213> Artificial Sequence
<220>
   <223> Description of Artificial Sequence: Synthetic oligonucleotide
<220>
   <223> RMRPO3H533P09
<400> 258
   ccggcaccga cagtcggc 18
<210> 259
   <211> 16
   <212> DNA
   <213> Artificial Sequence
<220>
   <223> Description of Artificial Sequence: Synthetic oligonucleotide
<220>
   <223> RMRPO3H533P10
<400> 259
   cgccggggcc cggcgg 16
<210> 260
   <211> 15
   <212> DNA
   <213> Artificial Sequence
<220>
   <223> Description of Artificial Sequence: Synthetic oligonucleotide
<220>
   <223> RMRPO3H533P11
<400> 260
   cgccggggcc cggcg 15
<210> 261
   <211> 14
   <212> DNA
   <213> Artificial Sequence
<220>
   <223> Description of Artificial Sequence: Synthetic oligonucleotide
<220>
   <223> RMRPO3H533P12
<400> 261
   cgccggggcc cggc 14
<210> 262
   <211> 17
   <212> DNA
   <213> Artificial Sequence
<220>
   <223> Description of Artificial Sequence: Synthetic oligonucleotide
<220>
   <223> RMRPO3H533P8B
<400> 262
   ccggcgccga cagtcgg 17
<210> 263
   <211> 16
   <212> DNA
   <213> Artificial Sequence
<220>
   <223> Description of Artificial Sequence: Synthetic oligonucleotide
<220>
   <223> RMRPO3H533P8C
<400> 263
   ccggcgccga cagtcg 16
<210> 264
   <211> 14
   <212> DNA
   <213> Artificial Sequence
<220>
   <223> Description of Artificial Sequence: Synthetic oligonucleotide
<220>
   <223> RMRPO3H533P13
<400> 264
   cgccggggcc ggcc 14
<210> 265
   <211> 16
   <212> DNA
   <213> Artificial Sequence
<220>
   <223> Description of Artificial Sequence: Synthetic oligonucleotide
<220>
   <223> RMRPO3H533P10C
<400> 265
   cgccggggcc cggcgg 16
<210> 266
   <211> 16
   <212> DNA
   <213> Artificial Sequence
<220>
   <223> Description of Artificial Sequence: Synthetic oligonucleotide
<220>
   <223> RMRPO3H533P10E
<400> 266
   cgccggggcc cggcgg 16
<210> 267
   <211> 14
   <212> DNA
   <213> Artificial Sequence
<220>
   <223> Description of Artificial Sequence: Synthetic oligonucleotide
<220>
   <223> RMRPO3533P10C2
<400> 267
   cgccggggcc cggc 14
<210> 268
   <211> 14
   <212> DNA
   <213> Artificial Sequence
<220>
   <223> Description of Artificial Sequence: Synthetic oligonucleotide
<220>
   <223> RMRPO3533P12B
<400> 268
   cgccggggcc cggc 14
<210> 269
   <211> 14
   <212> DNA
   <213> Artificial Sequence
<220>
   <223> Description of Artificial Sequence: Synthetic oligonucleotide
<220>
   <223> RMRPO3533P12C
<400> 269
   cgccggggcc cggc 14
<210> 270
   <211> 16
   <212> DNA
   <213> Artificial Sequence
<220>
   <223> Description of Artificial Sequence: Synthetic oligonucleotide
<220>
   <223> RMRPO3533P12B2
<400> 270
   cgccggggcc cggcgc 16
<210> 271
   <211> 16
   <212> DNA
   <213> Artificial Sequence
<220>
   <223> Description of Artificial Sequence: Synthetic oligonucleotide
<220>
   <223> RMRPO3533P12C2
<400> 271
   cgccggggcc cggcgg 16
<210> 272
   <211> 16
   <212> DNA
   <213> Artificial Sequence
<220>
   <223> Description of Artificial Sequence: Synthetic oligonucleotide
<220>
   <223> RMRPO3533P12C3
<400> 272
   cgccgaggcc cggcgg 16
<210> 273
   <211> 16
   <212> DNA
   <213> Artificial Sequence
<220>
   <223> Description of Artificial Sequence: Synthetic oligonucleotide
<220>
   <223> RMRPO3533P12C4
<400> 273
   agccggggcc cggcgg 16
<210> 274
   <211> 14
   <212> DNA
   <213> Artificial Sequence
<220>
   <223> Description of Artificial Sequence: Synthetic oligonucleotide
<220>
   <223> RMRPO3533P13
<400> 274
   ccggggcccg gcgg 14
<210> 275
   <211> 14
   <212> DNA
   <213> Artificial Sequence
<220>
   <223> Description of Artificial Sequence: Synthetic oligonucleotide
<220>
   <223> RMRPO3533P14
<400> 275
   ccggggcccg gcgg 14
<210> 276
   <211> 15
   <212> DNA
   <213> Artificial Sequence
<220>
   <223> Description of Artificial Sequence: Synthetic oligonucleotide
<220>
   <223> RMRPO3H533P02B
<400> 276
   ccggcgccga cagtc 15
<210> 277
   <211> 18
   <212> DNA
   <213> Artificial Sequence
<220>
   <223> Description of Artificial Sequence: Synthetic oligonucleotide
<220>
   <223> AYRPO3511PFM01
<400> 277
   cagccgagcc aattcatg 18
<210> 278
   <211> 18
   <212> DNA
   <213> Artificial Sequence
<220>
   <223> Description of Artificial Sequence: Synthetic oligonucleotide
<220>
   <223> AYRPO3511PFM02
<400> 278
   cagccgagcc aattcatg 18
<210> 279
   <211> 18
   <212> DNA
   <213> Artificial Sequence
<220>
   <223> Description of Artificial Sequence: Synthetic oligonucleotide
<220>
   <223> AYRPO3511PFM03
<400> 279
   cagccgagcc aattcatg 18
<210> 280
   <211> 18
   <212> DNA
   <213> Artificial Sequence
<220>
   <223> Description of Artificial Sequence: Synthetic oligonucleotide
<220>
   <223> AYRPO3511PFM04
<400> 280
   cagccgagcc aattcatg 18
<210> 281
   <211> 18
   <212> DNA
   <213> Artificial Sequence
<220>
   <223> Description of Artificial Sequence: Synthetic oligonucleotide
<220>
   <223> AYRPO3511PFM05
<400> 281
   cagccgagcc aattcatg 18
<210> 282
   <211> 18
   <212> DNA
   <213> Artificial Sequence
<220>
   <223> Description of Artificial Sequence: Synthetic oligonucleotide
<220>
   <223> AYRPO3526RFM01
<400> 282
   cttgcgggtc aaccccga 18
<210> 283
   <211> 16
   <212> DNA
   <213> Artificial Sequence
<220>
   <223> Description of Artificial Sequence: Synthetic oligonucleotide
<220>
   <223> AYRPO3526RFM02
<400> 283
   tgcgggtcaa ccccga 16
<210> 284
   <211> 16
   <212> DNA
   <213> Artificial Sequence
<220>
   <223> Description of Artificial Sequence: Synthetic oligonucleotide
<220>
   <223> AYRPO3526RFM03
<400> 284
   tgcgggtcaa ccccga 16
<210> 285
   <211> 18
   <212> DNA
   <213> Artificial Sequence
<220>
   <223> Description of Artificial Sequence: Synthetic oligonucleotide
<220>
   <223> AYRPO3526RFM04
<400> 285
   cttgcgggtc aaccccga 18
<210> 286
   <211> 18
   <212> DNA
   <213> Artificial Sequence
<220>
   <223> Description of Artificial Sequence: Synthetic oligonucleotide
<220>
   <223> AYRPO3526RFM05
<400> 286
   cttgcgggtc aaccccga 18
<210> 287
   <211> 16
   <212> DNA
   <213> Artificial Sequence
<220>
   <223> Description of Artificial Sequence: Synthetic oligonucleotide
<220>
   <223> AYRPO3526RFM06
<400> 287
   tgcgggtcaa ccccga 16
<210> 288
   <211> 16
   <212> DNA
   <213> Artificial Sequence
<220>
   <223> Description of Artificial Sequence: Synthetic oligonucleotide
<220>
   <223> AYRPO3526RFM07
<400> 288
   tgcgggtcaa ccccga 16
<210> 289
   <211> 18
   <212> DNA
   <213> Artificial Sequence
<220>
   <223> Description of Artificial Sequence: Synthetic oligonucleotide
<220>
   <223> AYRPO3526RFM08
<400> 289
   cttgcgggtc aaccccga 18
<210> 290
   <211> 18
   <212> DNA
   <213> Artificial Sequence
<220>
   <223> Description of Artificial Sequence: Synthetic probe
<220>
   <223> AYKAT315ICM01
<400> 290
   gatcaccatc ggcatcga 18
<210> 291
   <211> 19
   <212> DNA
   <213> Artificial Sequence
<220>
   <223> Description of Artificial Sequence: Synthetic probe
<220>
   <223> AYKAT315ICM02
<400> 291
   caccatcggc atcgaggtc 19
<210> 292
   <211> 19
   <212> DNA
   <213> Artificial Sequence
<220>
   <223> Description of Artificial Sequence: Synthetic probe
<220>
   <223> AYKAT315ICM03
<400> 292
   catcggcatc gaggtcgta 19
<210> 293
   <211> 22
   <212> DNA
   <213> Artificial Sequence
<220>
   <223> Description of Artificial Sequence: Synthetic probe
<220>
   <223> AYKAT315ICM04
<400> 293
   caccatcggc atcgaggtcg ta 22
<210> 294
   <211> 18
   <212> DNA
   <213> Artificial Sequence
<220>
   <223> Description of Artificial Sequence: Synthetic probe
<220>
   <223> AYKAT315ICM05
<400> 294
   atcggcatcg aggtcgta 18
<210> 295
   <211> 18
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYKAT315ICM06
<400> 295
   aucggcaucg aggucgua 18
<210> 296
   <211> 18
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYKAT315ICM07
<400> 296
   aucggcaucg aggucgua 18
<210> 297
   <211> 19
   <212> DNA
   <213> Artificial Sequence
<220>
   <223> Description of Artificial Sequence: Synthetic probe
<220>
   <223> AYKAT315ICM03a
<400> 297
   catcggcatc gaggtcgta 19
<210> 298
   <211> 19
   <212> DNA
   <213> Artificial Sequence
<220>
   <223> Description of Artificial Sequence: Synthetic probe
<220>
   <223> AYKAT315ICM03b
<400> 298
   catcggcatc gaggtcgta 19
<210> 299
   <211> 19
   <212> DNA
   <213> Artificial Sequence
<220>
   <223> Description of Artificial Sequence: Synthetic probe
<220>
   <223> AYKAT315ICM03c
<400> 299
   catcggcatc gaggtcgta 19
<210> 300
   <211> 19
   <212> DNA
   <213> Artificial Sequence
<220>
   <223> Description of Artificial Sequence: Synthetic probe
<220>
   <223> AYKAT315ICM03d
<400> 300
   catcggcatc gaggtcgta 19
<210> 301
   <211> 19
   <212> DNA
   <213> Artificial Sequence
<220>
   <223> Description of Artificial Sequence: Synthetic probe
<220>
   <223> AYKAT315ICY03a1
<400> 301
   catcggcatc gaggtcgta 19
<210> 302
   <211> 19
   <212> DNA
   <213> Artificial Sequence
<220>
   <223> Description of Artificial Sequence: Synthetic probe
<220>
   <223> AYKAT315ICY03a2
<400> 302
   catcggcatc gaggtcgta 19
<210> 303
   <211> 19
   <212> DNA
   <213> Artificial Sequence
<220>
   <223> Description of Artificial Sequence: Synthetic probe
<220>
   <223> AYKAT315ICY03a3
<400> 303
   catcggcatc gaggtcgta 19
<210> 304
   <211> 19
   <212> DNA
   <213> Artificial Sequence
<220>
   <223> Description of Artificial Sequence: Synthetic probe
<220>
   <223> AYKAT315ICY03a4
<400> 304
   catcggcatc gaggtcgta 19
<210> 305
   <211> 19
   <212> DNA
   <213> Artificial Sequence
<220>
   <223> Description of Artificial Sequence: Synthetic probe
<220>
   <223> AYKAT315NCM01
<400> 305
   caacggcatc gaggtcgta 19
<210> 306
   <211> 19
   <212> DNA
   <213> Artificial Sequence
<220>
   <223> Description of Artificial Sequence: Synthetic probe
<220>
   <223> AYKAT315NCM02
<400> 306
   caacggcatc gaggtcgta 19
<210> 307
   <211> 19
   <212> DNA
   <213> Artificial Sequence
<220>
   <223> Description of Artificial Sequence: Synthetic probe
<220>
   <223> AYKAT315NCM03
<400> 307
   caacggcatc gaggtcgta 19
<210> 308
   <211> 19
   <212> DNA
   <213> Artificial Sequence
<220>
   <223> Description of Artificial Sequence: Synthetic probe
<220>
   <223> AYKAT315NCM04
<400> 308
   caacggcatc gaggtcgta 19
<210> 309
   <211> 19
   <212> DNA
   <213> Artificial Sequence
<220>
   <223> Description of Artificial Sequence: Synthetic probe
<220>
   <223> AYKAT315NCM05
<400> 309
   caacggcatc gaggtcgta 19
<210> 310
   <211> 19
   <212> DNA
   <213> Artificial Sequence
<220>
   <223> Description of Artificial Sequence: Synthetic probe
<220>
   <223> AYKAT315NCM04a
<400> 310
   caacggcatc gaggtcgta 19
<210> 311
   <211> 19
   <212> DNA
   <213> Artificial Sequence
<220>
   <223> Description of Artificial Sequence: Synthetic probe
<220>
   <223> AYKAT315NCM05a
<400> 311
   caacggcatc gaggtcgta 19
<210> 312
   <211> 19
   <212> DNA
   <213> Artificial Sequence
<220>
   <223> Description of Artificial Sequence: Synthetic probe
<220>
   <223> AYKAT315NCM07
<400> 312
   caacggcatc gaggtcgta 19
<210> 313
   <211> 18
   <212> DNA
   <213> Artificial Sequence
<220>
   <223> Description of Artificial Sequence: Synthetic probe
<220>
   <223> AYKAT315NCM08
<400> 313
   aacggcatcg aggtcgta 18
<210> 314
   <211> 19
   <212> DNA
   <213> Artificial Sequence
<220>
   <223> Description of Artificial Sequence: Synthetic probe
<220>
   <223> AYKAT315NCM09
<400> 314
   caccaacggc atcgaggtc 19
<210> 315
   <211> 22
   <212> DNA
   <213> Artificial Sequence
<220>
   <223> Description of Artificial Sequence: Synthetic probe
<220>
   <223> AYKAT315NCM10
<400> 315
   caccaacggc atcgaggtcg ta 22
<210> 316
   <211> 19
   <212> DNA
   <213> Artificial Sequence
<220>
   <223> Description of Artificial Sequence: Synthetic probe
<220>
   <223> AYKAT315NCM11
<400> 316
   caacggcatc gaggtcgta 19
<210> 317
   <211> 18
   <212> DNA
   <213> Artificial Sequence
<220>
   <223> Description of Artificial Sequence: Synthetic probe
<220>
   <223> AYKAT315NCM12
<400> 317
   aacggcatcg aggtcgta 18
<210> 318
   <211> 19
   <212> DNA
   <213> Artificial Sequence
<220>
   <223> Description of Artificial Sequence: Synthetic probe
<220>
   <223> AYKAT315NCM13
<400> 318
   caccaacggc atcgaggtc 19
<210> 319
   <211> 22
   <212> DNA
   <213> Artificial Sequence
<220>
   <223> Description of Artificial Sequence: Synthetic probe
<220>
   <223> AYKAT315NCM14
<400> 319
   caccaacggc atcgaggtcg ta 22
<210> 320
   <211> 19
   <212> DNA
   <213> Artificial Sequence
<220>
   <223> Description of Artificial Sequence: Synthetic probe
<220>
   <223> AYKAT315NCM15
<400> 320
   caacggcatc gaggtcgta 19
<210> 321
   <211> 18
   <212> DNA
   <213> Artificial Sequence
<220>
   <223> Description of Artificial Sequence: Synthetic probe
<220>
   <223> AYKAT315NCM16
<400> 321
   aacggcatcg aggtcgta 18
<210> 322
   <211> 19
   <212> DNA
   <213> Artificial Sequence
<220>
   <223> Description of Artificial Sequence: Synthetic probe
<220>
   <223> AYKAT315NCM17
<400> 322
   caccaacggc atcgaggtc 19
<210> 323
   <211> 22
   <212> DNA
   <213> Artificial Sequence
<220>
   <223> Description of Artificial Sequence: Synthetic probe
<220>
   <223> AYKAT315NCM18
<400> 323
   caccaacggc atcgaggtcg ta 22
<210> 324
   <211> 19
   <212> DNA
   <213> Artificial Sequence
<220>
   <223> Description of Artificial Sequence: Synthetic probe
<220>
   <223> AYKAT315NCM19
<400> 324
   caacggcatc gaggtcgta 19
<210> 325
   <211> 20
   <212> DNA
   <213> Artificial Sequence
<220>
   <223> Description of Artificial Sequence: Synthetic probe
<220>
   <223> AYKAT315NCM20
<400> 325
   ccaacggcat cgaggtcgta 20
<210> 326
   <211> 20
   <212> DNA
   <213> Artificial Sequence
<220>
   <223> Description of Artificial Sequence: Synthetic probe
<220>
   <223> AYKAT315NCM21
<400> 326
   ccaacggcat cgaggtcgta 20
<210> 327
   <211> 21
   <212> DNA
   <213> Artificial Sequence
<220>
   <223> Description of Artificial Sequence: Synthetic probe
<220>
   <223> AYKAT315NCM22
<400> 327
   accaacggca tcgaggtcgt a 21
<210> 328
   <211> 21
   <212> DNA
   <213> Artificial Sequence
<220>
   <223> Description of Artificial Sequence: Synthetic probe
<220>
   <223> AYKAT315NCM23
<400> 328
   accaacggca tcgaggtcgt a 21
<210> 329
   <211> 19
   <212> DNA
   <213> Artificial Sequence
<220>
   <223> Description of Artificial Sequence: Synthetic probe
<220>
   <223> AYKAT315TCM01
<400> 329
   caccaccggc atcgaggtc 19
<210> 330
   <211> 19
   <212> DNA
   <213> Artificial Sequence
<220>
   <223> Description of Artificial Sequence: Synthetic probe
<220>
   <223> AYKAT315TCM02
<400> 330
   caccaccggc atcgaggtc 19
<210> 331
   <211> 19
   <212> DNA
   <213> Artificial Sequence
<220>
   <223> Description of Artificial Sequence: Synthetic probe
<220>
   <223> AYKAT315TCM03
<400> 331
   caccggcatc gaggtcgta 19
<210> 332
   <211> 16
   <212> DNA
   <213> Artificial Sequence
<220>
   <223> Description of Artificial Sequence: Synthetic probe
<220>
   <223> AYKAT315TCM04
<400> 332
   caccggcatc gaggtc 16
<210> 333
   <211> 15
   <212> DNA
   <213> Artificial Sequence
<220>
   <223> Description of Artificial Sequence: Synthetic probe
<220>
   <223> AYKAT315TCM05
<400> 333
   caccaccggc atcga 15
<210> 334
   <211> 15
   <212> DNA
   <213> Artificial Sequence
<220>
   <223> Description of Artificial Sequence: Synthetic probe
<220>
   <223> AYKAT315TCM05a
<400> 334
   caccaccggc atcga 15
<210> 335
   <211> 15
   <212> DNA
   <213> Artificial Sequence
<220>
   <223> Description of Artificial Sequence: Synthetic probe
<220>
   <223> AYKAT315TCM05b
<400> 335
   caccaccggc atcga 15
<210> 336
   <211> 15
   <212> DNA
   <213> Artificial Sequence
<220>
   <223> Description of Artificial Sequence: Synthetic probe
<220>
   <223> AYKAT315TCM05c
<400> 336
   caccaccggc atcga 15
<210> 337
   <211> 15
   <212> DNA
   <213> Artificial Sequence
<220>
   <223> Description of Artificial Sequence: Synthetic probe
<220>
   <223> AYKAT315TCY05b1
<400> 337
   caccaccggc atcga 15
<210> 338
   <211> 15
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYKAT315TCY05b2
<400> 338
   caccaccggc atcga 15
<210> 339
   <211> 15
   <212> DNA
   <213> Artificial Sequence
<220>
   <223> Description of Artificial Sequence: Synthetic probe
<220>
   <223> AYKAT315TCY05b3
<400> 339
   caccaccggc atcga 15
<210> 340
   <211> 15
   <212> DNA
   <213> Artificial Sequence
<220>
   <223> Description of Artificial Sequence: Synthetic probe
<220>
   <223> AYKAT315TCY05b4
<400> 340
   caccaccggc atcga 15
<210> 341
   <211> 15
   <212> DNA
   <213> Artificial Sequence
<220>
   <223> Description of Artificial Sequence: Synthetic probe
<220>
   <223> AYKAT315T2CM01
<400> 341
   caccacaggc atcga 15
<210> 342
   <211> 15
   <212> DNA
   <213> Artificial Sequence
<220>
   <223> Description of Artificial Sequence: Synthetic probe
<220>
   <223> AYKAT315T2CM02
<400> 342
   caccacaggc atcga 15
<210> 343
   <211> 15
   <212> DNA
   <213> Artificial Sequence
<220>
   <223> Description of Artificial Sequence: Synthetic probe
<220>
   <223> AYKAT315T2CM03
<400> 343
   caccacaggc atcga 15
<210> 344
   <211> 20
   <212> DNA
   <213> Artificial Sequence
<220>
   <223> Description of Artificial Sequence: Synthetic probe
<220>
   <223> AYINHA15TCM01
<400> 344
   gcgagatgat aggttgtcgg 20
<210> 345
   <211> 23
   <212> DNA
   <213> Artificial Sequence
<220>
   <223> Description of Artificial Sequence: Synthetic probe
<220>
   <223> AYINHA15TCM02
<400> 345
   gagatgatag gttgtcgggg tga 23
<210> 346
   <211> 20
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM03
<400> 346
   gcgagaugau agguugucgg 20
<210> 347
   <211> 22
   <212> DNA
   <213> Artificial Sequence
<220>
   <223> Description of Artificial Sequence: Synthetic probe
<220>
   <223> AYINHA15TCM04
<400> 347
   agatgatagg ttgtcggggt ga 22
<210> 348
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM05
<400> 348
   agaugauagg uugucggggu ga 22
<210> 349
   <211> 21
   <212> DNA
   <213> Artificial Sequence
<220>
   <223> Description of Artificial Sequence: Synthetic probe
<220>
   <223> AYINHA15TCM06
<400> 349
   gatgataggt tgtcggggtg a 21
<210> 350
   <211> 22
   <212> DNA
   <213> Artificial Sequence
<220>
   <223> Description of Artificial Sequence: Synthetic probe
<220>
   <223> AYINHA15TCM04a
<400> 350
   agatgatagg ttgtcggggt ga 22
<210> 351
   <211> 22
   <212> DNA
   <213> Artificial Sequence
<220>
   <223> Description of Artificial Sequence: Synthetic probe
<220>
   <223> AYINHA15TCM04b
<400> 351
   agatgatagg ttgtcggggt ga 22
<210> 352
   <211> 22
   <212> DNA
   <213> Artificial Sequence
<220>
   <223> Description of Artificial Sequence: Synthetic probe
<220>
   <223> AYINHA15TCM04c
<400> 352
   agatgatagg ttgtcggggt ga 22
<210> 353
   <211> 22
   <212> DNA
   <213> Artificial Sequence
<220>
   <223> Description of Artificial Sequence: Synthetic probe
<220>
   <223> AYINHA15TCM04d
<400> 353
   agatgatagg ttgtcggggt ga 22
<210> 354
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM07
<400> 354
   agaugauagg uugucggggu ga 22
<210> 355
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM08
<400> 355
   gaugauaggu ugucggggug a 21
<210> 356
   <211> 20
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM09
<400> 356
   augauagguu gucgggguga 20
<210> 357
   <211> 20
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM09a
<400> 357
   augauagguu gucgggguga 20
<210> 358
   <211> 20
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM09b
<400> 358
   atgauagguu gucgggguga 20
<210> 359
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM06a
<400> 359
   gaugauaggu ugucggggug a 21
<210> 360
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA15TCM06b
<400> 360
   gatgauaggu ugucggggug a 21
<210> 361
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA15TCM06A_1
<400> 361
   gaugauaggu ugucggggug a 21
<210> 362
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA15TCM06A_2
<400> 362
   gaugauaggu ugucggggug a 21
<210> 363
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA15TCM06B_1
<400> 363
   gatgauaggu ugucggggug a 21
<210> 364
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA15TCM06B_2
<400> 364
   gatgauaggu ugucggggug a 21
<210> 365
   <211> 20
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFYINHA15TCM09A_1
<400> 365
   augauagguu gucgggguga 20
<210> 366
   <211> 20
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA15TCM09A_1
<400> 366
   augauagguu gucgggguga 20
<210> 367
   <211> 20
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA15TCM09B_1
<400> 367
   atgauagguu gucgggguga 20
<210> 368
   <211> 20
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA15TCM09B_2
<400> 368
   atgauagguu gucgggguga 20
<210> 369
   <211> 18
   <212> DNA
   <213> Artificial Sequence
<220>
   <223> Description of Artificial Sequence: Synthetic probe
<220>
   <223> AYINHAR15TCM01
<400> 369
   ctatcatctc gccgcggc 18
<210> 370
   <211> 19
   <212> DNA
   <213> Artificial Sequence
<220>
   <223> Description of Artificial Sequence: Synthetic probe
<220>
   <223> AYINHAR15TCM02
<400> 370
   ctatcatctc gccgcggcc 19
<210> 371
   <211> 20
   <212> DNA
   <213> Artificial Sequence
<220>
   <223> Description of Artificial Sequence: Synthetic probe
<220>
   <223> AYINHAR15TCM03
<400> 371
   ctatcatctc gccgcggccg 20
<210> 372
   <211> 18
   <212> DNA
   <213> Artificial Sequence
<220>
   <223> Description of Artificial Sequence: Synthetic probe
<220>
   <223> AYINHAR15TCM04
<400> 372
   tatcatctcg ccgcggcc 18
<210> 373
   <211> 22
   <212> DNA
   <213> Artificial Sequence
<220>
   <223> Description of Artificial Sequence: Synthetic probe
<220>
   <223> AYINHA8ACM01
<400> 373
   taggatgtcg gggtgactgc ca 22
<210> 374
   <211> 20
   <212> DNA
   <213> Artificial Sequence
<220>
   <223> Description of Artificial Sequence: Synthetic probe
<220>
   <223> AYINHA8ACM02
<400> 374
   taggatgtcg gggtgactgc 20
<210> 375
   <211> 22
   <212> DNA
   <213> Artificial Sequence
<220>
   <223> Description of Artificial Sequence: Synthetic probe
<220>
   <223> AYINHA8ACM03
<400> 375
   gataggatgt cggggtgact gc 22
<210> 376
   <211> 18
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA8ACM04
<400> 376
   uaggaugucg gggugacu 18
<210> 377
   <211> 22
   <212> DNA
   <213> Artificial Sequence
<220>
   <223> Description of Artificial Sequence: Synthetic probe
<220>
   <223> AYINHA8ACM05
<400> 377
   taggatgtcg gggtgactgc ca 22
<210> 378
   <211> 22
   <212> DNA
   <213> Artificial Sequence
<220>
   <223> Description of Artificial Sequence: Synthetic probe
<220>
   <223> AYINHA8ACM06
<400> 378
   taggatgtcg gggtgactgc ca 22
<210> 379
   <211> 22
   <212> DNA
   <213> Artificial Sequence
<220>
   <223> Description of Artificial Sequence: Synthetic probe
<220>
   <223> AYINHA8ACM07
<400> 379
   taggatgtcg gggtgactgc ca 22
<210> 380
   <211> 22
   <212> DNA
   <213> Artificial Sequence
<220>
   <223> Description of Artificial Sequence: Synthetic probe
<220>
   <223> AYINHA8ACM08
<400> 380
   taggatgtcg gggtgactgc ca 22
<210> 381
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA8ACM08a
<400> 381
   uaggaugucg gggugacugc ca 22
<210> 382
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHA8ACM08b
<400> 382
   uaggaugucg gggugacugc ca 22
<210> 383
   <211> 22
   <212> DNA
   <213> Artificial Sequence
<220>
   <223> Description of Artificial Sequence: Synthetic probe
<220>
   <223> AYINHAR8ACM01
<400> 383
   cgacatccta tcgtctcgcc gc 22
<210> 384
   <211> 21
   <212> DNA
   <213> Artificial Sequence
<220>
   <223> Description of Artificial Sequence: Synthetic probe
<220>
   <223> AYINHAR8ACM02
<400> 384
   gacatcctat cgtctcgccg c 21
<210> 385
   <211> 20
   <212> DNA
   <213> Artificial Sequence
<220>
   <223> Description of Artificial Sequence: Synthetic probe
<220>
   <223> AYINHAR8ACM03
<400> 385
   acatcctatc gtctcgccgc 20
<210> 386
   <211> 19
   <212> DNA
   <213> Artificial Sequence
<220>
   <223> Description of Artificial Sequence: Synthetic probe
<220>
   <223> AYINHAR8ACM04
<400> 386
   catcctatcg tctcgccgc 19
<210> 387
   <211> 20
   <212> DNA
   <213> Artificial Sequence
<220>
   <223> Description of Artificial Sequence: Synthetic probe
<220>
   <223> AYINHAR8ACM05
<400> 387
   cgacatccta tcgtctcgcc 20
<210> 388
   <211> 21
   <212> DNA
   <213> Artificial Sequence
<220>
   <223> Description of Artificial Sequence: Synthetic probe
<220>
   <223> AYINHAR8ACM02a
<400> 388
   gacatcctat cgtctcgccg c 21
<210> 389
   <211> 21
   <212> DNA
   <213> Artificial Sequence
<220>
   <223> Description of Artificial Sequence: Synthetic probe
<220>
   <223> AYINHAR8ACM02b
<400> 389
   gacatcctat cgtctcgccg c 21
<210> 390
   <211> 21
   <212> DNA
   <213> Artificial Sequence
<220>
   <223> Description of Artificial Sequence: Synthetic probe
<220>
   <223> AYINHAR8ACM02c
<400> 390
   gacatcctat cgtctcgccg c 21
<210> 391
   <211> 21
   <212> DNA
   <213> Artificial Sequence
<220>
   <223> Description of Artificial Sequence: Synthetic probe
<220>
   <223> AYINHAR8ACM02d
<400> 391
   gacatcctat cgtctcgccg c 21
<210> 392
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHAR8ACM02e
<400> 392
   gacatccuau cgucucgccg c 21
<210> 393
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHAR8ACM02f
<400> 393
   gacatccuau cgucucgccg c 21
<210> 394
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHAR8ACM02g
<400> 394
   gacatccuau cgucucgccg c 21
<210> 395
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYINHAR8ACM02h
<400> 395
   gacatccuau cgucucgccg c 21
<210> 396
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA8ACM08A_1
<400> 396
   uaggaugucg gggugacugc ca 22
<210> 397
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA8ACM08A_2
<400> 397
   uaggaugucg gggugacugc ca 22
<210> 398
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA8ACM08B_1
<400> 398
   uaggaugucg gggugacugc ca 22
<210> 399
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> JFINHA8ACM08B_2
<400> 399
   uaggaugucg gggugacugc ca 22
<210> 400
   <211> 20
   <212> DNA
   <213> Artificial Sequence
<220>
   <223> Description of Artificial Sequence: Synthetic probe
<220>
   <223> AYINHA8ACM01
<400> 400
   taggctgtcg gggtgactgc 20
<210> 401
   <211> 20
   <212> DNA
   <213> Artificial Sequence
<220>
   <223> Description of Artificial Sequence: Synthetic probe
<220>
   <223> AYINHA8ACM02
<400> 401
   taggctgtcg gggtgactgc 20
<210> 402
   <211> 22
   <212> DNA
   <213> Artificial Sequence
<220>
   <223> Description of Artificial Sequence: Synthetic probe
<220>
   <223> AYINHA8ACM03
<400> 402
   gataggctgt cggggtgact gc 22
<210> 403
   <211> 21
   <212> DNA
   <213> Artificial Sequence
<220>
   <223> Description of Artificial Sequence: Synthetic probe
<220>
   <223> AYINHA8ACM04
<400> 403
   ataggctgtc ggggtgactg c 21
<210> 404
   <211> 19
   <212> DNA
   <213> Artificial Sequence
<220>
   <223> Description of Artificial Sequence: Synthetic probe
<220>
   <223> AYINHA8ACM05
<400> 404
   aggctgtcgg ggtgactgc 19
<210> 405
   <211> 22
   <212> DNA
   <213> Artificial Sequence
<220>
   <223> Description of Artificial Sequence: Synthetic probe
<220>
   <223> AYINHAR8CCM01
<400> 405
   cgacagccta tcgtctcgcc gc 22
<210> 406
   <211> 21
   <212> DNA
   <213> Artificial Sequence
<220>
   <223> Description of Artificial Sequence: Synthetic probe
<220>
   <223> AYINHAR8CCM02
<400> 406
   gacagcctat cgtctcgccg c 21
<210> 407
   <211> 20
   <212> DNA
   <213> Artificial Sequence
<220>
   <223> Description of Artificial Sequence: Synthetic probe
<220>
   <223> AYINHAR8CCM03
<400> 407
   acagcctatc gtctcgccgc 20
<210> 408
   <211> 19
   <212> DNA
   <213> Artificial Sequence
<220>
   <223> Description of Artificial Sequence: Synthetic probe
<220>
   <223> AYINHAR8CCM04
<400> 408
   cagcctatcg tctcgccgc 19
<210> 409
   <211> 20
   <212> DNA
   <213> Artificial Sequence
<220>
   <223> Description of Artificial Sequence: Synthetic probe
<220>
   <223> AYINHAR8CCM05
<400> 409
   cgacagccta tcgtctcgcc 20
<210> 410
   <211> 18
   <212> DNA
   <213> Artificial Sequence
<220>
   <223> Description of Artificial Sequence: Synthetic forward primer
<220>
   <223> AYKATG003
<400> 410
   gcggtcacac tttcggta 18
<210> 411
   <211> 16
   <212> DNA
   <213> Artificial Sequence
<220>
   <223> Description of Artificial Sequence: Synthetic reverse primer
<220>
   <223> AYKATG002
<400> 411
   cttggcggtg tattgc 16
<210> 412
   <211> 20
   <212> DNA
   <213> Artificial Sequence
<220>
   <223> Description of Artificial Sequence: Synthetic forward primer
<220>
   <223> AYINHA001
<400> 412
   gaagtgtgct gagtcacacc 20
<210> 413
   <211> 18
   <212> DNA
   <213> Artificial Sequence
<220>
   <223> Description of Artificial Sequence: Synthetic reverse primer
<220>
   <223> AYINHA002
<400> 413
   ggactgaacg ggatacga 18
<210> 414
   <211> 266
   <212> DNA
   <213> Mycobacterium tuberculosis
<220>
   <223> katG WT amplicon
<400> 414
   gcggtcacac tttcggtaag acccatggcg ccggcccggc cgatctggtc ggccccgaac 60
   ccgaggctgc tccgctggag cagatgggct tgggctggaa gagctcgtat ggcaccggaa 120
   ccggtaagga cgcgatcacc agcggcatcg aggtcgtatg gacgaacacc ccgacgaaat 180
   gggacaacag tttcctcgag atcctgtacg gctacgagtg ggagctgacg aagagccctg 240
   ctggcgcttg gcaatacacc gccaag 266
<210> 415
   <211> 191
   <212> DNA
   <213> Mycobacterium tuberculosis
<220>
   <223> inhA WT amplicon
<400> 415
   gaagtgtgct gagtcacacc gacaaacgtc acgagcgtaa ccccagtgcg aaagttcccg 60
   ccggaaatcg cagccacgtt acgctcgtgg acataccgat ttcggcccgg ccgcggcgag 120
   acgataggtt gtcggggtga ctgccacagc cactgaaggg gccaaacccc cattcgtatc 180
   ccgttcagtc c 191
<210> 416
   <211> 184
   <212> DNA
   <213> Mycobacterium tuberculosis
<220>
   <223> RIF WT amplicon
<400> 416
   ggacgtggag gcgatcacac cgcagacgtt gatcaacatc cggccggtgg tcgccgcgat 60
   caaggagttc ttcggcacca gccagctgag ccaattcatg gaccagaaca acccgctgtc 120
   ggggttgacc cacaagcgcc gactgtcggc gctggggccc ggcggtctgt cacgtgagcg 180
   tgcc 184
<210> 417
   <211> 184
   <212> DNA
   <213> Artificial Sequence
<220>
   <223> Description of Artificial Sequence: Synthetic polynucleotide
<220>
   <223> RIF MT03 amplicon
<400> 417
   ggacgtggag gcgatcacac cgcagacgtt gatcaacatc cggccggtgg tcgccgcgat      60
   caaggagttc ttcggcacca gccagccgag ccaattcatg gaccagaaca acccgctgtc     120
   ggggttgacc gacaagcgcc gactgtcggc gcctgggccc ggcggtctgt cacgtgagcg     180
   tgcc                                                                  184
<210> 418
   <211> 184
   <212> DNA
   <213> Artificial Sequence
<220>
   <223> Description of Artificial Sequence: Synthetic polynucleotide
<220>
   <223> RIF MT04 amplicon
<400> 418
   ggacgtggag gcgatcacac cgcagacgtt gatcaacatc cggccggtgg tcgccgcgat      60
   caaggagttc ttcggcagca gccagctgag ccaattcatg gtccagaaca acccgctgtc     120
   ggggttgacc aacaagcgcc gactgtcggc gctggggtcc ggcggtctgt cacgtgagcg     180
   tgcc                                                                  184
<210> 419
   <211> 184
   <212> DNA
   <213> Artificial Sequence
<220>
   <223> Description of Artificial Sequence: Synthetic polynucleotide
<220>
   <223> RIF MT05 amplicon
<400> 419
   ggacgtggag gcgatcacac cgcagacgtt gatcaacatc cggccggtgg tcgccgcgat      60
   caaggagttc ttcggcgcca gccagctgag ccaattcatg taccagaaca acccgctgtc     120
   ggggttgacc cgcaagcgcc gactgtcggc gctggggcac ggcggtctgt cacgtgagcg     180
   tgcc                                                                  184
<210> 420
   <211> 184
   <212> DNA
   <213> Artificial Sequence
<220>
   <223> Description of Artificial Sequence: Synthetic polynucleotide
<220>
   <223> RIF MT06 amplicon
<400> 420
   ggacgtggag gcgatcacac cgcagacgtt gatcaacatc cggccggtgg tcgccgcgat 60
   caaggagttc ttcggcccca gccagctgag ccaattcatg gaccagaaca acccgctgtc 120
   ggggttgacc tacaagcgcc gactgtcggc gccggggccc ggcggtctgt cacgtgagcg 180
   tgcc 184
<210> 421
   <211> 184
   <212> DNA
   <213> Artificial Sequence
<220>
   <223> Description of Artificial Sequence: Synthetic polynucleotide
<220>
   <223> RIF MT07 amplicon
<400> 421
   ggacgtggag gcgatcacac cgcagacgtt gatcaacatc cggccggtgg tcgccgcgat 60
   caaggagttc ttcggcacca gccagcggag ccaattcatg gaccagaaca acccgctgtc 120
   ggggttgacc ctcaagcgcc gactgtcggc gctggggccc ggcggtccgt cacgtgagcg 180
   tgcc 184
<210> 422
   <211> 184
   <212> DNA
   <213> Artificial Sequence
<220>
   <223> Description of Artificial Sequence: Synthetic polynucleotide
<220>
   <223> RIF MT08 amplicon
<400> 422
   ggacgtggag gcgatcacac cgcagacgtt gatcaacatc cggccggtgg tcgccgcgat 60
   caaggagttc ttcggcacca gccagctgag cctattcatg gaccagaaca acccgctgca 120
   ggggttgacc cacaagcgcc gactgttggc gctggggccc ggcggtctgt cacgtgagcg 180
   tgcc 184
<210> 423
   <211> 184
   <212> DNA
   <213> Artificial Sequence
<220>
   <223> Description of Artificial Sequence: Synthetic polynucleotide
<220>
   <223> RIF MT09 amplicon
<400> 423
   ggacgtggag gcgatcacac cgcatacgtt gatcaacatc cggccggtgg tcgccgcgat 60
   caaggagtcc ttcggcacca gccagctgag caaattcatg gaccagaaca acccgctgtt 120
   ggggttgacc cacaagcgcc gactgtgggc gctggggccc ggcggtctgt cacgtgagcg 180
   tgcc 184
<210> 424
   <211> 184
   <212> DNA
   <213> Artificial Sequence
<220>
   <223> Description of Artificial Sequence: Synthetic polynucleotide
<220>
   <223> RIF MT10 amplicon
<400> 424
   ggacgtggag gcgatcacac cgcagacgtt gatcaacatc cggccggtgg tcgccgcgat 60
   caaggagtta ttcggcacca gccagctgag cccattcatg gaccagaaca acccgctgtg 120
   ggggttgacc cacaagcgcc gactgtttgc gctggggccc ggcggtctgt cacgtgagcg 180
   tgcc 184
<210> 425
   <211> 191
   <212> DNA
   <213> Mycobacterium tuberculosis
<220>
   <223> WT_INHA amplicon
<400> 425
   gaagtgtgct gagtcacacc gacaaacgtc acgagcgtaa ccccagtgcg aaagttcccg 60
   ccggaaatcg cagccacgtt acgctcgtgg acataccgat ttcggcccgg ccgcggcgag 120
   acgataggtt gtcggggtga ctgccacagc cactgaaggg gccaaacccc cattcgtatc 180
   ccgttcagtc c 191
<210> 426
   <211> 191
   <212> DNA
   <213> Artificial Sequence
<220>
   <223> Description of Artificial Sequence: Synthetic polynucleotide
<220>
   <223> MT_INHA02 amplicon
<400> 426
   gaagtgtgct gagtcacacc gacaaacgtc acgagcgtaa ccccagtgcg aaagttcccg 60
   ccggaaatcg cagccacgtt acgctcgtgg acataccgat ttcggcccgg ccgcggcgag 120
   atgataggtt gtcggggtga ctgccacagc cactgaaggg gccaaacccc cattcgtatc 180
   ccgttcagtc c 191
<210> 427
   <211> 191
   <212> DNA
   <213> Artificial Sequence
<220>
   <223> Description of Artificial Sequence: Synthetic polynucleotide
<220>
   <223> MT_INHA03 amplicon
<400> 427
   gaagtgtgct gagtcacacc gacaaacgtc acgagcgtaa ccccagtgcg aaagttcccg 60
   ccggaaatcg cagccacgtt acgctcgtgg acataccgat ttcggcccgg ccgcggcgag 120
   acgataggta gtcggggtga ctgccacagc cactgaaggg gccaaacccc cattcgtatc 180
   ccgttcagtc c 191
<210> 428
   <211> 191
   <212> DNA
   <213> Artificial Sequence
<220>
   <223> Description of Artificial Sequence: Synthetic polynucleotide
<220>
   <223> MT_INHA04 amplicon
<400> 428
   gaagtgtgct gagtcacacc gacaaacgtc acgagcgtaa ccccagtgcg aaagttcccg 60
   ccggaaatcg cagccacgtt acgctcgtgg acataccgat ttcggcccgg ccgcggcgag 120
   acgataggtc gtcggggtga ctgccacagc cactgaaggg gccaaacccc cattcgtatc 180
   ccgttcagtc c 191
<210> 429
   <211> 266
   <212> DNA
   <213> Mycobacterium tuberculosis
<220>
   <223> WT_KATG01 amplicon
<400> 429
   gcggtcacac tttcggtaag acccatggcg ccggcccggc cgatctggtc ggccccgaac 60
   ccgaggctgc tccgctggag cagatgggct tgggctggaa gagctcgtat ggcaccggaa 120
   ccggtaagga cgcgatcacc agcggcatcg aggtcgtatg gacgaacacc ccgacgaaat 180
   gggacaacag tttcctcgag atcctgtacg gctacgagtg ggagctgacg aagagccctg 240
   ctggcgcttg gcaatacacc gccaag 266
<210> 430
   <211> 266
   <212> DNA
   <213> Artificial Sequence
<220>
   <223> Description of Artificial Sequence: Synthetic polynucleotide
<220>
   <223> MT_KATG02 amplicon
<400> 430
   gcggtcacac tttcggtaag acccatggcg ccggcccggc cgatctggtc ggccccgaac 60
   ccgaggctgc tccgctggag cagatgggct tgggctggaa gagctcgtat ggcaccggaa 120
   ccggtaagga cgcgatcacc atcggcatcg aggtcgtatg gacgaacacc ccgacgaaat 180
   gggacaacag tttcctcgag atcctgtacg gctacgagtg ggagctgacg aagagccctg 240
   ctggcgcttg gcaatacacc gccaag 266
<210> 431
   <211> 266
   <212> DNA
   <213> Artificial Sequence
<220>
   <223> Description of Artificial Sequence: Synthetic polynucleotide
<220>
   <223> MT_KATG03 amplicon
<400> 431
   gcggtcacac tttcggtaag acccatggcg ccggcccggc cgatctggtc ggccccgaac 60
   ccgaggctgc tccgctggag cagatgggct tgggctggaa gagctcgtat ggcaccggaa 120
   ccggtaagga cgcgatcacc aacggcatcg aggtcgtatg gacgaacacc ccgacgaaat 180
   gggacaacag tttcctcgag atcctgtacg gctacgagtg ggagctgacg aagagccctg 240
   ctggcgcttg gcaatacacc gccaag 266
<210> 432
   <211> 266
   <212> DNA
   <213> Artificial Sequence
<220>
   <223> Description of Artificial Sequence: Synthetic polynucleotide
<220>
   <223> MT_KATG04 amplicon
<400> 432
   gcggtcacac tttcggtaag acccatggcg ccggcccggc cgatctggtc ggccccgaac 60
   ccgaggctgc tccgctggag cagatgggct tgggctggaa gagctcgtat ggcaccggaa 120
   ccggtaagga cgcgatcacc accggcatcg aggtcgtatg gacgaacacc ccgacgaaat 180
   gggacaacag tttcctcgag atcctgtacg gctacgagtg ggagctgacg aagagccctg 240
   ctggcgcttg gcaatacacc gccaag 266
<210> 433
   <211> 266
   <212> DNA
   <213> Artificial Sequence
<220>
   <223> Description of Artificial Sequence: Synthetic polynucleotide
<220>
   <223> MT_KATG05 amplicon
<400> 433
   gcggtcacac tttcggtaag acccatggcg ccggcccggc cgatctggtc ggccccgaac      60
   ccgaggctgc tccgctggag cagatgggct tgggctggaa gagctcgtat ggcaccggaa     120
   ccggtaagga cgcgatcacc acaggcatcg aggtcgtatg gacgaacacc ccgacgaaat     180
   gggacaacag tttcctcgag atcctgtacg gctacgagtg ggagctgacg aagagccctg     240
   ctggcgcttg gcaatacacc gccaag                                          266
<210> 434
   <211> 18
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> RM315N2B
<400> 434
   caacgcaucg aggucgua 18
<210> 435
   <211> 21
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> RM315T2
<400> 435
   accacaggca tcgaggtcgt a 21
<210> 436
   <211> 17
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> RMINHA8C4BB
<400> 436
   acagccuauc gucucgc 17
<210> 437
   <211> 18
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> AYRPO3526RFM11
<400> 437
   cttgcgggtc aaccccga 18
<210> 438
   <211> 22
   <212> DNA
   <213> Artificial Sequence
<220>
   <223> Description of Artificial Sequence: Synthetic probe
<220>
   <223> Description of Combined DNA/RNA Molecule: Synthetic probe
<220>
   <223> RM_NEW526N11B3
<400> 438
   ttgttggtca accccgacgg gg 22